# DEMANDE DE BREVET EUROPEEN

(11) **EP 0 428 434 A2**
(43) Date de publication de la demande: **22.05.1991**
(21) Numéro de dépôt: 90403125.9
(22) Date de dépôt: 06.11.1990
(51) Int. Cl.: C07D 211/14, C07D 409/06, C07D 401/06, C07D 401/12, C07D 409/12, C07D 413/12, C07D 405/12, C07D 211/32, C07D 211/22, C07D 211/70, C07D 295/13

(54) **Composés aromatiques aminés et leurs énantiomères, procédé pour leur préparation et compositions pharmaceutiques les contenant**

(30) Priorité: 06.11.1989 FR 8914517; 15.06.1990 FR 9007534
(71) Demandeur: SANOFI, F-75008 Paris (FR)
(72) Inventeur: Emonds-Alt, Xavier, F-34980 Combaillaux (FR); Goulaouic, Pierre, F-34000 Montpellier (FR); Proietto, Vincenzo, F-34680 St. Georges d'Orques (FR); Van Broeck, Didier, F-34570 Murviel Les Montpellier (FR)
(74) Mandataire: Gillard, Marie-Louise

(57) **Abrégé**

L'invention concerne des composés de formule 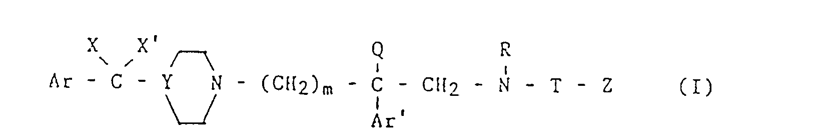 dans laquelle :
- m est un nombre entier de 1 à 3 ;
- Ar et Ar représentent, indépendamment, un groupe thiényle, un groupe phényle substitué ou non substitué; un groupe imidazolyle; Ar pouvant également être un groupe benzothiényle non substitué ou substitué par un halogène; un groupe naphtyle non substitué ou substitué par un halogène; un groupe biphényle; un indolyle non substitué ou substitué;
- X représente l'hydrogène;
- X représente l'hydrogène, un groupe hydroxyle, ou est réuni à X ci-dessous pour former une liaison carbone-carbone;
. ou bien X et X, ensemble, forment un groupe oxo ou dialkylaminoalkyloxyimino de formule = N-O-(CH₂)p-Am, où p est 2 ou 3 et Am est un groupe dialkylamino;
- Y représente un atome d'azote ou un groupe C(X") où X" est l'hydrogène ou, avec X', forme une liaison carbone-carbone;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino;
- R représente l'hydrogène, un groupe méthyle ou un groupe (CH₂)ₙ-L, où n est un nombre entier de 2 à 6 et L est l'hydrogène ou un groupe amino,
- T représente un groupe choisi parmi 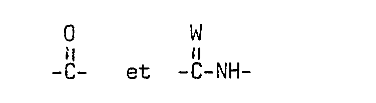 W étant un atome d'oxygène ou de soufre, et
- Z représente soit l'hydrogène, soit M ou OM lorsque T représente le groupe 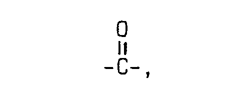 soit M lorsque T représente le groupe 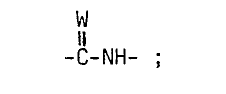
M représente l'hydrogène ou un alkyle droit ou ramifié; un phénylalkyle non substitué ou substitué; un pyridylalkyle; un naphtylalkyle; un pyridylthioalkyle; un styryle; un (méthyl-1) imidazolyl-2 thioalkyle ; un oxo-1 phénylindan-3 yle-2; un groupe aromatique ou hétéroaromatique non substitué ou substitué; ou un de leurs sels avec des acides minéraux ou organiques.

Application : antagonistes des récepteurs des neurokinines.

## Description

La présente invention a pour objet de nouveaux dérivés aromatiques substitués par un groupement amino et par diverses fonctions esters, amines ou amides, ainsi que leurs énantiomères.

La présente invention concerne également le procédé d'obtention des composés, qui peut être énantiosélectif et l'utilisation des composés selon l'invention dans des compositions à usage thérapeutique et plus particulièrement dans les phénomènes pathologiques qui impliquent le système des neurokinines comme : la douleur (D. REGOLI et al., Life Sciences, 1987, 40, 109-117), l'allergie et l'inflammation (J.E. MORLAY et al., Life Sciences, 1987, 41, 527-544), l'insuffisance circulatoire (J. LOSAY et al., 1977, Substance P, Von Euler, U.S. and Pernow ed., 287-293, Raven Press, New York), les troubles gastro- intestinaux (D. REGOLI et al., Trends Pharmacol. Sci., 1985, 6, 481-484), les troubles respiratoires (J. MIZRAHI et al., Pharmacology, 1982, 25, 39-50).

Des ligands endogènes aux récepteurs des neurokinines ont été décrits, telles la substance P (SP), la neurokinine A (NKA) (S.J. BAILEY et al., 1983, Substance P, P. Skrabanck ed., 16-17 Boole Press, Dublin) et la neurokinine B (NKB) (S.P. WATSON, Life Sciences, 1983, 25, 797-808).

Les récepteurs aux neurokinines ont été reconnus sur de nombreuses préparations et sont actuellement classés en trois types : NK,, NK₂ et NKs. Alors que la plupart des préparations étudiées jusqu'à maintenant, présentent plusieurs types de récepteurs, tel l'iléon de cobaye (NK,, NK₂ et NK₃), certaines d'entre elles n'en possèderaient qu'un seul, telles l'artère carotide de chien (NK₁), l'artère pulmonaire de lapin dépourvue d'endothélium (NK₂) et la veine porte de rat (NK₃) (D. REGOLI et al., Trends Pharmacol. Sci., 1988, 9, 290-295 et Pharmacology, 1989, 38, 1-15).

Une caractérisation plus précise des différents récepteurs est rendue possible par la synthèse récente d'agonistes sélectifs. Ainsi, la [Sar⁹, Met-(O₂)¹¹] SP, la [Nle¹⁰] NKA₄₋₁₀, et la [Me Phe⁷] -NKB présenteraient une sélectivité respective pour les récepteurs NK₁, NK₂ et NK₃ (cf D. REGOLI, 1988 et 1989 précedemment cité).

On a maintenant trouvé que certains composés aromatiques aminés possèdent des propriétés pharmacologiques intéressantes, en tant qu'anta gonistes des récepteurs des neurokinines et sont notamment utiles pour le traitement de toute pathologie substance P et neurokinine dépendante.

Ainsi selon un de ses aspects, la présente invention concerne des dérivés aromatiques aminés de formule : 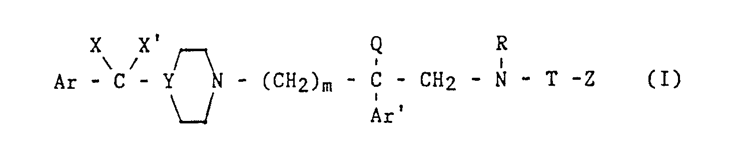 dans laquelle :
- m est un nombre entier de 1 à 3;
- Ar et Ar représentent, indépendamment, un groupe thiényle ; un groupe phényle non substitué, mono ou di-substitué par un atome d'halogène, de préférence un atome de chlore ou de fluor,par un alkyle en C₁-C₃, par un trifluorométhyle, par un alcoxy dans lequel l'alkyle est en C₁-C₃, par un hydroxyle,par un méthylènedioxy ; un groupe imidazolyle; Ar pouvant également être un groupe benzothiényle non substitué ou substitué par un halogène de préférence par un atome de chlore ou de fluor ; un groupe naphtyle non substitué ou substitué par un halogène de préférence par un atome de fluor; un groupe biphényle; un indolyle non substitué ou substitué sur l'azote par un groupe benzyle;
- X représente l'hydrogène;
- X' représente l'hydrogène, un groupe hydroxyle, ou est réuni à X" ci-dessous pour former une liaison carbone-carbone ;
. ou bien X et X , ensemble, forment un groupe oxo ou dialkylaminoalkyloxyimino de formule =N-0-(CH2)p-Am, où p est 2 ou 3 et Am est un groupe dialkylamino, chaque alkyle pouvant contenir de 1 à 4 atomes de carbone;
- Y représente un atome d'azote ou un groupe C(X") où X" est l'hydrogène ou, avec X , forme une liaison carbone-carbone ;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino, chaque alkyle pouvant contenir de 1 à 4 atomes de carbone;
- R représente l'hydrogène, un groupe méthyle ou un groupe (CH₂)ₙ-L, où n est un nombre entier de 2 à 6 et L est l'hydrogène ou un groupe amino, - T représente un groupe choisi parmi 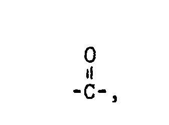 et 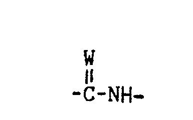 W étant un atome d'oxygène ou de soufre, et
- Z représente soit l'hydrogène, soit M ou OM lorsque T représente le groupe 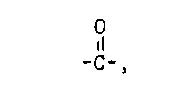 soit M lorsque T représente le groupe 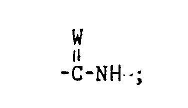
M représente l'hydrogène ou un alkyle droit ou ramifié en Ci-Ce ; un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1 ) imidazolyl-2 thioalkyle dans lequel le groupe alkyle comprend de 1 à 3 atomes de carbone ; un oxo-1 phénylindan-3 yle-2 ; un groupe aromatique ou hétéroaromatique non substitué, mono ou polysubstitué ;
ou un de leurs sels avec des acides minéraux ou organiques.

Les sels des composés de formule (I) selon la présente invention comprennent aussi bien ceux avec des acides minéraux ou organiques qui permettent une séparation ou une cristallisation convenable des composés de formule (1), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate, le glycolate, le gluconate, le citrate, l'iséthionate.

De façon particulière, dans la formule (1), Z représente un groupe aromatique ou hétéroaromatique mono-, di- ou tricyclique, pouvant porter un ou plusieurs substituants, dont un atome de carbone du carbocycle aromatique ou de l'hétérocycle aromatique est directement lié au groupe T.

Plus particulièrement, le radical Z peut être un groupe phényle, qui peut être non substitué ou éventuellement contenir un ou plusieurs substituants.

Lorsque Z est un groupe phényle, celui-ci peut être de préférence mono-ou disubstitué, notamment en position 2,4, mais aussi par exemple en position 2,3, 4,5, 3,4 ou 3,5; il peut aussi être trisubstitué, notamment en position 2,4,6, mais aussi par exemple en 2,3,4, 2,3,5 ou 2,4,5, 3,4,5; tétrasubstitué, par exemple en 2,3,4,5; ou pentasubstitué. Les substituants du groupe phényle peuvent être: F; CI; Br; 1; CN; OH; NH₂; NH-CO-NH₂; NOz; CONH₂; CF₃; alkyle en Ci-Cio, de préférence en C,-C₄, méthyle ou éthyle étant préférés, ainsi que par exemple n-propyle, isopropyle, n-butyle, isobutyle, sec-butyle, tert.-butyle, pentyle ou n-pentyle, hexyle ou n-hexyle, heptyle ou n-heptyle, octyle ou n-octyle, nonyle ou n-nonyle ainsi que décyle ou n-décyle; alcényle contenant 2 à 10, de préférence 2-4 atomes de carbone, par exemple vinyle, allyle, 1-propényle, isopropényle, butényle ou 1-butén-1-, -2-, -3- ou -4-yle, 2-butén-1-yle, 2-butén-2- yle, pentényle, hexényle ou décényle; alcynyle contenant 2 à 10, de préférence 2-4 atomes de carbone, par exemple éthynyle, 1-propyn-1-yle, propargyle, butynyle ou 2-butyn-1-yle, pentynyle, décynyle; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexy- le, cycloheptyle ou cyclooctyle; bicycloalkyle contenant 4 à 11, de préférence 7 atomes de carbone, exo ou endo 2-norbornyle étant préférés, ainsi que par exemple 2-isobornyle ou 5-camphyle; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle; alcoxy contenant 1 à 10, de préférence 1-4 atomes de carbone, méthoxy ou éthoxy étant préférés, ainsi que par exemple n-propoxy, isopropoxy, n-butoxy, isobutoxy, sec-butoxy, tert-butoxy, pentyloxy, hexyloxy, heptyloxy, octyloxy, nonyloxy ou décyloxy; alcoxyalkyle contenant 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle; alcoxyalcoxyalkyle contenant jusqu'à 10, de préférence de 4 à 7 atomes de carbone, par exemple alcoxyalcoxyméthyle, par exemple 2-méthoxyéthoxyméthyle, 2-éthoxyéthoxyméthyle ou 2-isopro- poxyéthoxyméthyle, alcoxyalcoxyéthyle par exemple 2-(2-méthoxyéthoxy)éthyle ou 2-(2-éthoxyéthoxy)-éthyle; alcoxyalcoxy contenant de 2 à 10, de préférence de 3 à 6 atomes de carbone, par exemple 2-méthoxyéthoxy, 2-éthoxyéthoxy ou 2-n-butoxyéthoxy; alcényloxy contenant 2 à 10, de préférence 2 à 4 atomes de carbone, allyloxy étant préféré, ainsi que par exemple vinyloxy, propényloxy, isopropényloxy, butényloxy tel que 1-butén-1-, -2-, -3- ou -4-yloxy, 2-butén-1-yloxy, 2-butén-2-yloxy, pentényloxy, hexény- loxy ou décényloxy; alcényloxyalkyle avec jusqu'à 10, de préférence 3-6 atomes de carbone, par exemple allyloxyméthyle; alcynyloxy contenant de 2 à 10, de préférence 2 à 4 atomes de carbone, propargyloxy étant préféré, ainsi que par exemple éthynyloxy, 1-propyn-1-yloxy, butynyloxy ou 2-butyn-1-yloxy, pentyny- loxy ou décynyloxy; alcynyloxyalkyle contenant de 3 à 10, de préférence 3 à 6 atomes de carbone, par exemple éthynyloxyméthyle, propargyloxyméthyle ou 2-(2-butyn-1-yloxy)éthyle; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexy- loxy, cycloheptyloxy ou cyclooctyloxy; alkylthio contenant de 1 à 10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio; alkylthioalkyle contenant de 2 à 10, de préférence 2 à 6 atomes de carbone, par exemple méthylthiométhy- le, 2-méthylthioéthyle ou 2-n-butylthioéthyle; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi que aroylamino ou ben- zoylamino; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoé- thyle, formylaminopropyle, acétylaminopropyle, propionylaminopropyle, formylaminobutyle, acétylaminobu- tyle, ainsi que propionylaminobutyle, butyrylaminobutyle; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy; alcoxycarbonyle contenant de 2 à 5, de préférence 2 et 3 atomes de carbone, méthoxycarbonyle et éthoxycar- bonyle étant préférés, ainsi que par exemple n-propoxycarbonyle, isopro- poxycarbonyle, n-butoxycarbonyle, isobutoxycarbonyle, sec-butoxycarbonyle ou tert-butoxycarbonyle; cy- cloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle; alkylaminocarbonylamino contenant de 2 à 4 atomes de carbone, tel que méthylaminocarbonylamino, éthylaminocarbonylamino, propylaminocarbonylamino; dialkylaminocarbonyla- mino contenant de 3 à 7, de préférence 3 à 5 atomes de carbone, de préférence diméthylaminocarbonyla- mino, ainsi que di-n-propylaminocarbonylamino, diisopropylaminocarbonylamino; (1-pyrrolidino)-carbonyla- mino; (1-pipéridino)carbonylamino; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cycloheptylaminocarbonylamino, alky- laminocarbonylaminoalkyle contenant de 3 à 9, de préférence 4 à 7 atomes de carbone, méthylaminocarbo- nylaminoéthyle, éthylaminocarbonylaminoéthyle, éthylaminocarbonylaminopropyle, éthylaminocarbonylami- nobutyle étant préférés, ainsi que par exemple méthylaminocarbonylaminométhyle, n-propylaminocarbony- laminobutyle, n-butylaminocarbonylaminobutyle; dialkylaminocarbonylaminoalkyle contenant de 4 à 11 atomes de carbone, par exemple diméthylaminocarbonylaminométhyle, diéthylaminocarbonylaminoéthyle, diéthylaminocarbonylaminopropyle, diéthylaminocarbonylaminobutyle;, (1-pyrrolidino)carbonylaminoéthyle; (1-pipéridino)- carbonylaminoéthyle; cycloalkylaminocarbonylaminoalkyle contenant de 5 à 12, de préférence 8 à 11 atomes de carbone, cyclopentylamino- carbonylaminoéthyle, cyclopentylaminocarbonylaminopro- pyle, cyclopentylaminocarbonylaminobutyle, cyclohexylaminocarbonylaminoéthyle, cyclohexylaminocarbo- nylaminopropyle et cyclohexylaminocarbonylaminobutyle étant préférés, ainsi que par exemple cyclopropy- laminocarbonylaminométhyle , cycloheptylaminocarbonylaminoéthyle; alcoxycarbonylaminoalkyle contenant de 3 à 12, de préférence 4 à 9 atomes de carbone, méthoxycarbonylaminoéthyle, éthoxycarbonylaminoé- thyle, n-propoxycarbonylaminoéthyle, isopropoxycarbonylaminoéthyle, n-butoxycarbonylaminoéthyle, isobu- toxycarbonylaminoéthyle, sec-butoxycarbonylaminoéthyle, tert-butoxycarbonylaminoéthyle, éthoxycarbony- laminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopropoxycarbonylaminobutyle; cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopenty- loxycarbonylaminobutyle, cyclohexyloxycarbonylaminoéthyle, cyclohexyloxycarbonylaminopropyle, cyclo- hexyloxycarbonylaminobutyle étant préférés, ainsi que par exemple cyclopropyloxycarbonylaminométhyle, cycloheptyloxycarbonylaminoéthyle, carbamoylalkyle contenant de 2 à 5, de préférence 2 atomes de carbone, de préférence carbamoylméthyle, ainsi que carbamoyléthyle, carbamoylpropyle, carbamoylbutyle; alkylaminocarbonylalkyle contenant de 3 à 9, de préférence 3 à 6 atomes de carbone, méthylaminocarbo- nyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminocarbonylméthyle, tert-butylaminocarbonylméthyle étant préférés, ainsi que par exemple éthylaminocarbonyléthyle, éthylaminocarbonylpro- pyle, éthylaminocarbonylbutyle, n-propylaminocarbonylbutyle,n-butylaminocarbonylbutyle; dialkylaminocar- bonylalkyle contenant de 4 à 11, de préférence 4 à 8 atomes de carbone, diméthylaminocarbonylméth- yle,diéthylaminocarbonylméthyle, di-n-propylaminocarbonylméthyle; ainsi que par exemple diéthylaminocar- bonyléthyle, diéthylaminocarbonylpropyle, diéthylaminocarbonylbutyle; (1-pyrrolidino) carbonylméthyle ; (1-pipéridino) carbonylméthyle; (1-pipéridino) carbonyléthyle; cycloalkylaminocarbonylalkyle contenant de 5 à 12, de préférence 7 ou 8 atomes de carbone, cyclopentylaminocarbonylméthyle et cyclohexylaminocarbo- nylméthyle étant préférés, ainsi que par exemple cyclopropylaminocarbonylméthyle, cyclobutylaminocarbo- nylméthyle, cycloheptylaminocarbonylméthyle, cyclohexylaminocarbonyléthyle, cyclohexylaminocarbonyl- propyle, cyclohexylaminocarbonylbutyle; alkylaminocarbonylalcoxy contenant de 3 à 10, de préférence 3 à 5 atomes de carbone, méthylaminocarbonylméthoxy étant préféré, ainsi que par exemple méthylaminocar- bonyléthoxy, méthylaminocarbonylpropoxy; dialkylaminocarbonylalkoxy contenant de 4 à 10, de préférence 4 à 7 atomes de carbone, tel que diméthylaminocarbonylméthoxy, diéthylaminocarbonyléthoxy, (pipéridinyl-1)carbonylméthoxy; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le groupe Z est avantageusement un groupe phényle ; un groupe benzyle ; un groupe benzoyle ; un groupe phénylthioalkyle dans lequel l'alkyle est en C,-C₃.

Le groupe phényle Z est de préférence mono ou disubstitué par un halogène, le groupe 2,4-dichlorophényle étant particulièrement préféré.

Le radical Z peut également répresenter un groupe aromatique bicyclique tel que le 1- ou 2-naphtyle; 1-, 2-, 3-, 4-, 5-, 6-, 7-indényle; dont une ou plusieurs liaisons peuvent être hydrogénées, lesdits groupes pouvant être non substitués ou contenir éventuellement un ou plusieurs substituants tel que : un halogène et plus particulièrement un atome de fluor, le groupe alkyle, phényle, cyano, hydroxyalkyle, hydroxy, oxo, alkylcarbonylamino et alcoxycarbonyle, thioalkyle dans lesquels les alkyles sont en C₁-C₄.

Le radical Z peut être aussi un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinoly- le, isoquinolyle, benzoxazolyle, benzisoxazolyle, benzoxazinyle, benzodioxinyle, isoxazolyle, benzopyranny- le, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en Cₗ-C₄.

Selon un autre de ses aspects, la présente invention concerne un procédé pour la préparation de composés aromatiques aminés différemment substitués de formule (I) et de leurs sels, caractérisé en ce que
a) on traite une amine libre de formule : 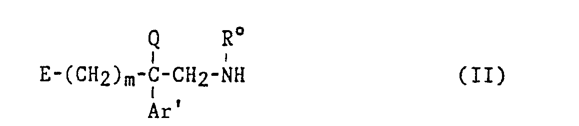 dans laquelle m, Ar et Q sont tels que définis précédemment; R représente l'hydrogène, un groupe méthyle ou un groupe (CH₂)ₙ-L°, où n, est tel que défini précédemment et L° est l'hydrogène ou un groupe amino protégé par un groupe N-protecteur ; et E représente un groupe hydroxy, un groupe O-protégé tel que tétrahydropyranyl-2 oxy, ou un groupe 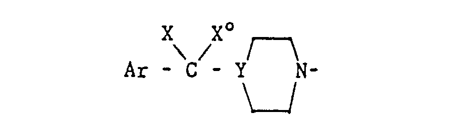 dans lequel Ar, X et Y sont tels que définis précédemment et X° représente le groupe X , tel que défini précédemment, dans lequel le groupe hydroxy est protégé par un groupe 0-protecteur; - soit avec un dérivé fonctionnel d'un acide de formule : 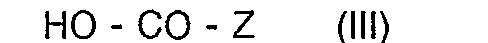 dans laquelle Z est tel que défini précédemment, lorsqu'on doit préparer un composé de formule (I) où T est -CO-, - soit avec un iso(thio)cyanate de formule : 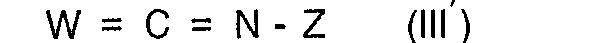 dans laquelle W et Z sont tels que définis précédemment, lorsqu'on doit préparer un composé de formule (I) où Test 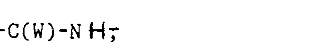 pour former le composé de formule : 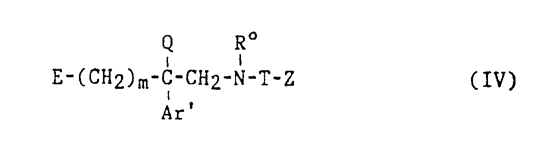
b) puis, lorsque E représente le tétrahydropyranyloxy, on élimine le groupe tétrahydropyranyle par hydrolyse acide, l'hydrolyse pouvant alternativement avoir lieu à l'étape (a) sur l'amine de départ de formule (II),
c) on traite l'alcanolamine N-substituée ainsi obtenue de formule : 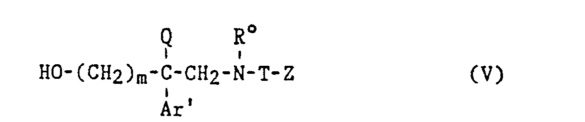 avec le chlorure de méthanesulfonyle et,
d) on fait réagir le mésylate ainsi obtenu de formule : 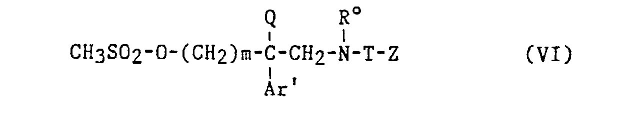 avec une amine secondaire de formule : 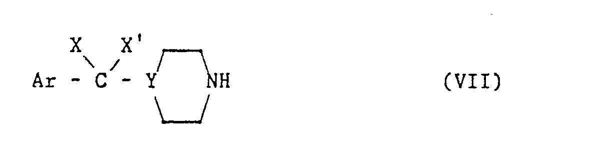 dans laquelle Ar, Y, X et X' sont tels que définis précédemment
e) on élimine les groupes O-protecteur et N-protecteur éventuels et on transforme éventuellement le produit ainsi obtenu en un de ses sels.

Comme dérivé fonctionnel de l'acide (III), on utilise l'acide lui-même, convenablement activé par exemple par le cyclohexylcarbodiimide ou par l'hexafluorophosphate de benzotriazolyl N-oxytrisdiméthylaminophosphonium (BOP), ou bien un des dérivés fonctionnels qui réagissent avec les amines, par exemple un anhydride, un anhydride mixte, le chlorure ou un ester activé. Lorsque Z est un groupe OM, l'acide concerné est l'acide carbonique et, comme dérivé fonctionnel, on utilise le monochlorure, à savoir un chloroformiate CI-CO-OM.

Les groupes N-protecteurs éventuellement présents dans le groupe R° du composé de la formule (II) sont les groupes N-protecteurs classiques bien connus de l'homme de l'art et de préférence ceux qui sont éliminables par hydrolyse acide, tels que le groupe trityle et méthoxytrityle.

Les groupes 0-protecteurs éventuellement présents dans le groupe X° sont également les groupes O-protecteurs classiques bien connus de l'homme de l'art et de préférence ceux qui sont éliminables par hydrolyse acide douce, tels que les groupes tétrahydropyranyle-2, t-butyldiméthylsilyle et méthoxyméthyle.

Lorsque comme produit de départ on utilise un composé de formule (II) où E représente un groupe 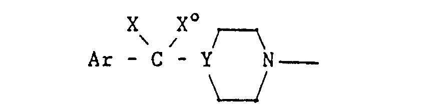 le procédé de la présente invention peut être représenté et illustré en détail par le Schéma 1 ci-après :

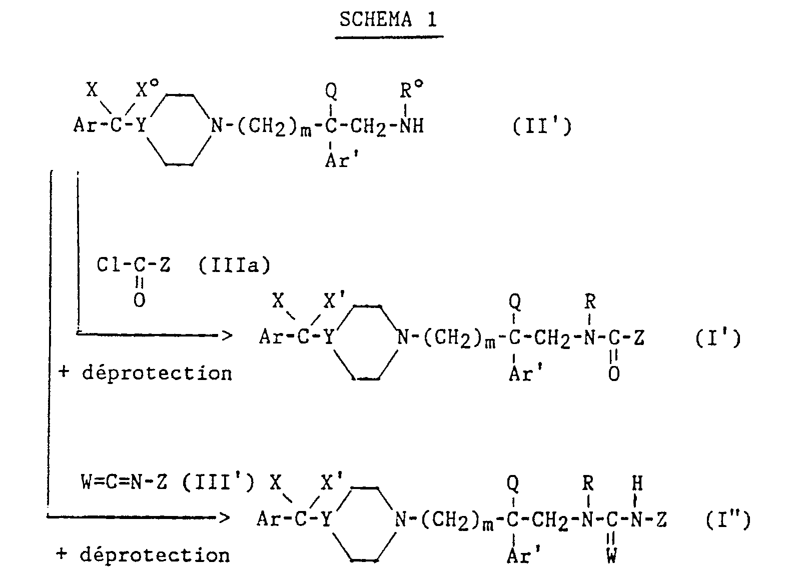

Dans la formule (IIIa) ci-dessus, on considère le chlorure d'acide comme dérivé fonctionnel réactif de l'acide (III). Le chlorure d'acide est utilisé lorsque l'on désire préparer un composé (I') où Z est OM. La réaction avec le chlorure d'acide est effectuée dans un solvant inerte, tel que le dichlorométhane ou le benzène en présence d'une base telle que par exemple la triéthylamine, à température ambiante.

Dans le cas particulier de Z = OM, la réaction du composé (II') avec le chloroformiate de formule : 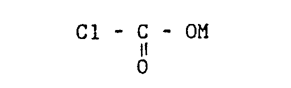 est effectuée selon les méthodes habituelles.

Lorsque Z est autre que OM, on peut utiliser un autre dérivé fonctionnel ou on peut partir de l'acide libre (III) en réalisant un couplage de (II') avec le BOP (hexafluorophosphate de benzotriazolyl N-oxytrisdiméthylaminophosphonium), puis en additionnant l'acide (III) en présence d'une base organique comme par exemple la triéthylamine, dans un solvant comme le dichlorométhane ou le diméthylformamide, à température ambiante, les composés (I') obtenus étant isolés et purifiés selon les méthodes habituelles, comme par exemple la chromatographie ou la recristallisation.

On peut faire aussi réagir (II') avec un iso(thio)cyanate W = C = N-Z (III') dans un solvant inerte anhydre tel que par exemple le benzène, pendant une nuit à température ambiante puis traiter le mélange réactionnel selon les méthodes habituelles pour obtenir les composés (I").

Lorsque comme produit de départ on utilise un composé de formule (II) où E représente un groupe tétrahydropyranyloxy, le procédé de la présente invention peut être représenté et illustré à partir du Schéma 2.

Les réactions du composé (II") avec les réactifs (Illa) et (III') se déroulent comme décrit ci-dessus pour le Schéma 1, le chlorure d'acide (Illa) pouvant être remplaçé par un autre dérivé fonctionnel ou par l'acide libre activé par exemple par le BOP.

L'intermédiaire (IV') ainsi obtenu est déprotégé par hydrolyse acide pour conduire au composé hydroxylé libre (V). La déprotection par hydrolyse du groupe tétrahydropyranyloxy peut être effectuée directement sur le composé (II"). On obtient ainsi le composé hydroxylé (II") qui est mis en réaction directement avec les réactifs (Illa) ou (III') comme décrit dans le Schéma 2 ci-dessous pour fournir le composé (V). On prépare ensuite le mésylate (VI) pour le substituer par une amine secondaire de formule (VII) pour obtenir finalement, après déprotection éventuelle de l'amine L°, les composés (I) selon l'invention.

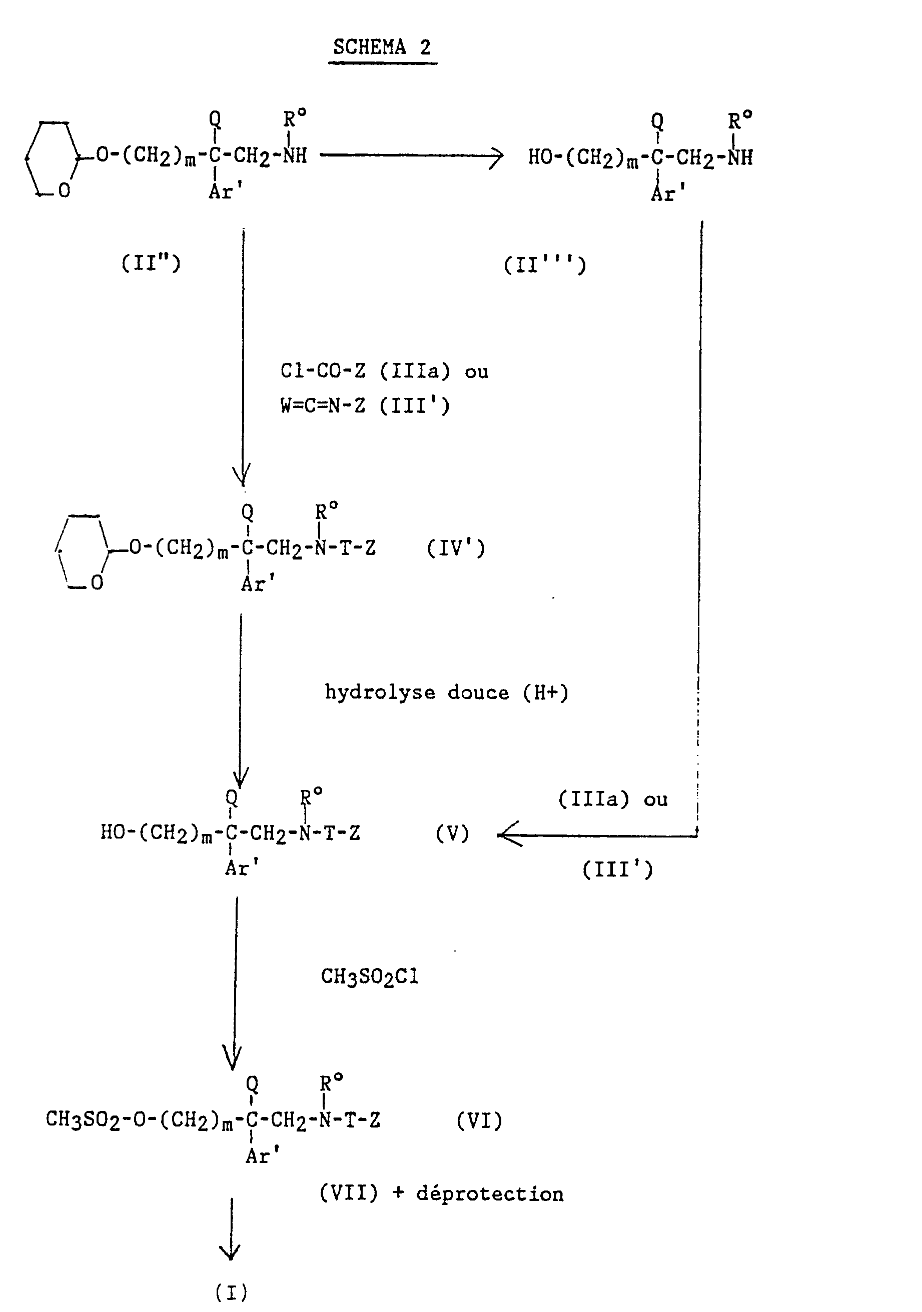

Lorsque le produit obtenu à la fin de la réaction entre le composé de formule (II) et le composé (III) (comme dérivé fonctionnel) ou (III'), a la formule IV où E représente un groupe 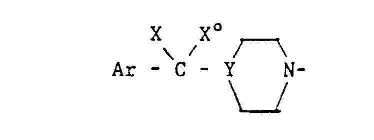 où Ar, X, X et Y sont tels que définis ci-dessus, le produit peut soit représenter le produit final soit présenter un groupe hydroxy O-protégé (dans X°) ou un groupe amino protégé (L°) ou les deux. Dans ce dernier cas, il est souhaitable d'utiliser, dans le produit de départ (II), des groupes O-protecteurs et N-protecteurs pouvant être hydrolysés en même temps.

La déprotection est effectuée selon les méthodes connues; notamment, si l'on utilise, comme groupe O-protecteur, un groupe tétrahydropyranyle, l'hydrolyse peut être effectuée en conditions douces avec de l'acide p-toluènesulfonique dilué. Si la molécule du produit (IV) contient à la fois un groupe tétrahydropyranyloxy et un groupe tritylamino, l'hydrolyse du premier peut être ainsi effectuée en respectant le groupe N-protecteur, alors que l'acide formique libère en même temps les deux groupes protecteurs.

Les produits de formule (I) ainsi obtenus sont isolés, sous forme de base libre ou de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, les composés de formule (I) peuvent être isolés sous forme d'un de leurs sels, par exemple le chlorhydrate ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

La résolution des mélanges racémiques (I) permet d'isoler les énantiomères qui font partie de l'invention.

On peut aussi effectuer le dédoublement de mélanges racémiques des produits de formule (II), notamment des produits de formule (II ) et (II") ou de leurs précurseurs, afin de préparer les énantiomères des produits de formule (1).

Les composés de départ de formule (II) sont préparés à partir de nitriles de formule : 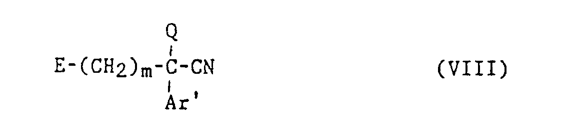 dans laquelle m, E, Q et Ar sont tels que définis ci-dessus, par réduction et alkylation éventuelle de l'amine obtenue.

Pour la préparation des composés de formule (II) où R° est l'hydrogène, les nitriles de départ de formule (VIII) sont soumis à une hydrogénation dans un alcanol tel que l'éthanol, en présence d'un catalyseur tel que par exemple le nickel de Raney et l'amine libre primaire peut être isolée selon les méthodes classiques.

Lorsqu'on souhaite préparer les composés de formule (II) où R est méthyle, on traite l'amine libre, obtenue par hydrogénation du nitrile (VIII) comme décrit ci-dessus, avec un chloroformiate, par exemple avec le chloroformiate de formule CI-CO-OAlk, où Alk est un alkyle en Ci-C₄, de préférence éthyle, pour obtenir les carbamates de formule : 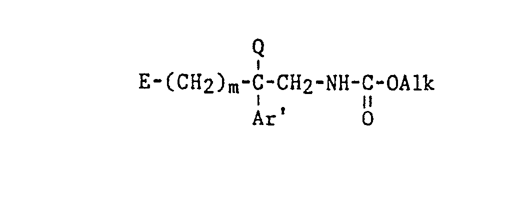 qui sont ensuite réduits par des moyens connus, tels que l'action d'un agent réducteur comme par exemple un hydrure métallique, tel que l'hydrure de sodium et d'aluminium, l'hydrure de lithium et d'aluminium ou par un hydrure du bore, tel que le diméthylsulfure de borane. La réduction est réalisée dans un solvant, tel que l'éther ou le toluène à une température comprise entre la température ambiante et 60⁰ C. La méthylamine ainsi obtenue de formule : 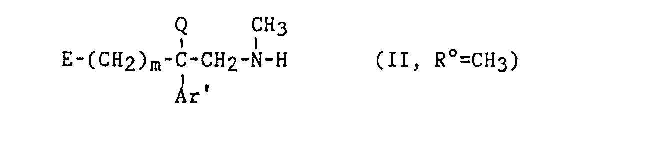 est isolée selon les méthodes habituelles.

Pour préparer les composés de formule (II) où R est un groupe -(CH₂)ₙ-L°, où n et L° sont tels que définis ci-dessus, on traite l'amine libre, obtenue par hydrogénation du nitrile (VIII) comme décrit ci-dessus, avec un dérivé fonctionnel réactif de l'acide de formule : 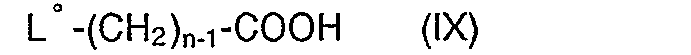 pour obtenir un amide de formule : 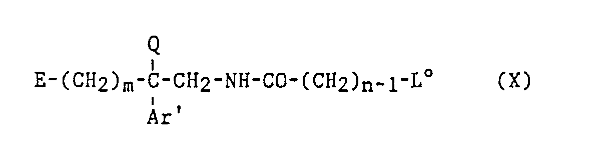 dans laquelle m, n, E, Ar', Q et L sont tels que définis ci-dessus.

L'amide (X), par réduction dans les mêmes conditions que celles décrites ci-dessus pour le nitrile (VIII), donne le composé désiré de formule :

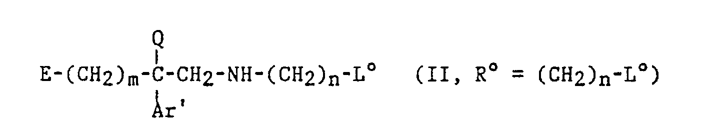

Les nitriles de formule (VIII), sont préparés à partir de nitriles connus, commerciaux ou préparés selon des méthodes connues, de formule : 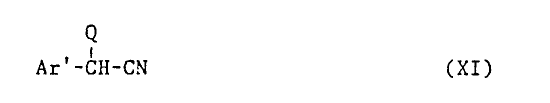 qui par alkylation avec un composé de formule : 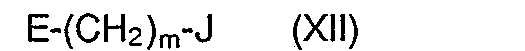 dans laquelle m et E sont tels que définis ci-dessus et J est un atome d'halogène, par exemple de brome ou un groupe hydroxy, donnent les composés (VIII) désirés.

La synthèse des nitriles de formule (VIII) où E est un groupe tétrahydropyranyloxy est réalisée à partir d'un dérivé tétrahydropyranyloxy (THP-O-) obtenu par réaction entre un alcanol de formule Br-(CH₂)ₘ₋OH avec m tel que défini précédemment et le dihydropyrane pour conduire au composé 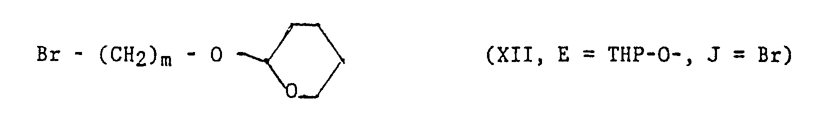 qui est ensuite additionné, en présence d'hydrure alcalin sur le dérivé acétonitrile (XI) pour préparer l'intermédiaire, 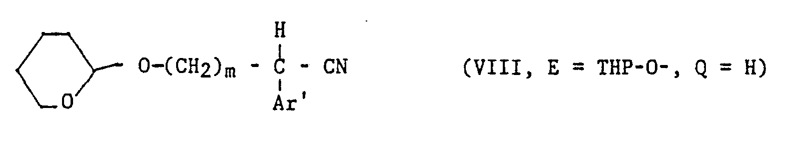 correspondant aux composés de formule (VIII) qui sont des précurseurs intermédiaires des composés (II') du schéma 1 ci-dessus, où Q est l'hydrogène, lequel peut ensuite être alkylé.

La synthèse des nitriles de formule (VIII) où E représente un groupe 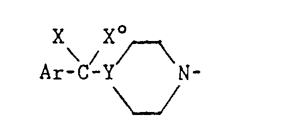 où Ar, X, X° et Y sont tels que définis précédemment, est effectuée selon des méthodes connues par addition sur des dérivés chlorés de formule : 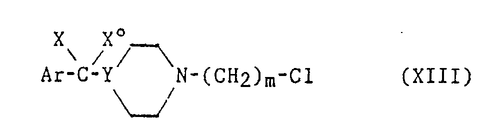 d'un dérivé nitrile de formule : 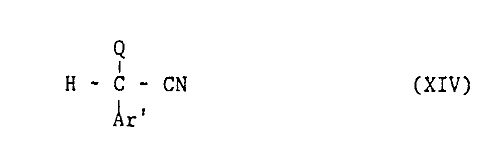 en présence d'amidure de sodium dans un solvant tel que le toluène à des températures comprises entre 30 et 80 °C.

Le dérivé chloré (XIII) est préparé par action d'un réactif chlorant tel que par exemple le chlorure de thionyle sur le dérivé hydroxylé de formule : 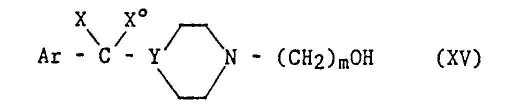 lui même préparé à partir de l'amine de formule : 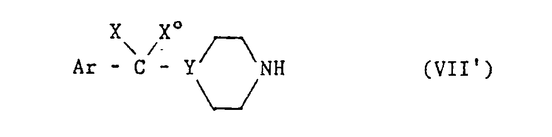 sur laquelle on fait réagir, si m = 2, l'oxyde d'éthylène et si m = 3, un halogéno-3 propanol.

Les amines (II) sont des produits nouveaux qui constituent les intermédiaires clés pour la préparation des composés de formule (I)ci-dessus.De plus, on a trouvé de façon surprenante que les amines de formule (II') sont douées, comme les composés de formule (1), bien qu'à un degré inférieur, d'une bonne activité antagoniste des récepteurs des neurokinines. L'activité de ces composés s'étend aux dérivés déprotégés selon les méthodes classiques rappelées plus haut (X° = X et R = R).

Ainsi, selon un autre de ses aspects, la présente invention concerne des composés de formule : 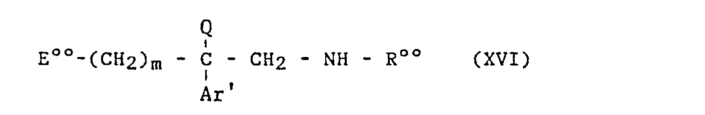 dans laquelle : - E° ° représente un groupe tétrahydropyranyloxy, un groupe hydroxy, ou un groupe 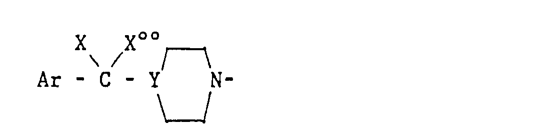
- m est un nombre entier de 1 à 3 ;
- Ar et Ar' représentent, indépendamment, un groupe thiényle ; un groupe phényle non substitué, mono ou di-substitué par un atome d'halogène, de préférence un atome de chlore ou de fluor, par un alkyle en C₁-Cₛ, par un trifluorométhyle par un alcoxy dans lequel l'alkyle est en C₁-C₃, par un hydroxyle,par un méthylènedioxy ; un groupe imidazolyle; Ar' pouvant être un groupe benzothiényle non substitué ou substitué par un halogène de préférence par un atome de chlore ou de fluor ; un groupe naphtyle non substitué ou substitué par un halogène de préférence par un atome de fluor ; un groupe biphényle ; un indolyle non substitué ou substitué sur l'azote par un groupe benzyle ;
- X représente l'hydrogène;
- X° ° représente l'hydrogène, un groupe hydroxyle libre ou protégé par un groupe 0-protecteur, ou est réuni à X ci-dessous pour former une liaison carbone-carbone,
. ou bien X et X° °, ensemble, forment un groupe oxo ou dialkylaminoalkyloxyimino de formule =N-O-(CH₂)p-Am, où p est 2 ou 3 et Am est un groupe dialkylamino, chaque alkyle pouvant contenir de 1 à 4 atomes de carbone;
- Y représente un atome d'azote ou un groupe C(X") où X" est l'hydrogène ou,avec X° °, forme une liaison carbone-carbone ;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino, chaque alkyle pouvant contenir de 1 à 4 atomes de carbone;
- R° représente l'hydrogène, un groupe méthyle ou un groupe (CH₂)ₙ-L° °, où n est un nombre de 2 à 6 et L° ° est l'hydrogène ou un groupe amino libre ou protégé par un groupe N-protecteur ;

ou un de leurs sels.

Les composés de formule XVI comprennent les composés de formule II qui sont des composés protégés, ainsi que les composés déprotégés correspondants.

Ils sont obtenus selon le procédé indiqué précédemment pour les composés de formule (II), chaque étape étant suivie de l'élimination éventuelle des groupes N- et 0-protecteurs. Ils peuvent ensuite être transformés en des sels avec des acides optiquement actifs, ce qui permet d'obtenir des composés optiquement purs.

Les composés de formule (XVI) particulièrement préférés sont ceux dans lesquels E ° ° représente un groupe hydroxy et R°° représente l'hydrogène ; ces composés ainsi que leurs énantiomères correspondent aux composés de formule (II").

Les intermédiaires de formule (IV), où E représente tétrahydropyranyloxy, ainsi que les intermédiaires de formule (V) et de formule (VI) sont des produits nouveaux particulièrement intéressants et représentent un aspect ultérieur de la présente invention. Ces produits, ainsi que les composés correspondants dont les groupes 0- et N-protecteurs ont été éliminés par des méthodes classiques, peuvent être regroupés dans la formule (V ) suivante : 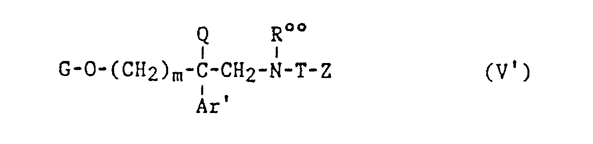 dans laquelle :
- m est un nombre entier de 1 à 3 ;
- G est l'hydrogène, un groupe tétrahydropyranyle, ou un groupe méthanesulfonyle ;
- Ar représente un groupe thiényle; un groupe phényle non substitué,i mono ou di-substitué par un atome d'halogène, de préférence un atome de chlore ou de fluor ou par un groupe trifluorométhyle; par un alcoxy dans lequel l'alkyle est en C₁-C₃, par un hydroxyle ; un groupe benzothiényle non substitué ou substitué par un halogène ; un groupe naphtyle non substitué ou substitué par un halogène, de préférence un atome de fluor ; ou un groupe biphényle; un groupe indolyle non substitué ou substitué sur l'azote par un benzyle
- Q représente l'hydrogène ou un groupe aminoalkyle de formule -(CH₂)q-Am', où q est 2 ou 3 et Am est un groupe pipéridino, benzyl-4 pipéridino ou dialkylamino, chaque alkyle pouvant contenir de 1 à 4 atomes de carbone;
- R° ° représente l'hydrogène, un groupe méthyle ou un groupe (CH₂)ₙ-L° °, où n est un nombre entier de 2 à 6 et L°° est l'hydrogène ou un groupe amino libre ou protégé par un groupe N-protecteur ;
- T représente un groupe choisi parmi 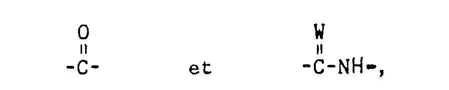 W étant un atome d'oxygène ou de soufre,
- Z représente soit l'hydrogène, soit M ou OM lorsque T représente le groupe 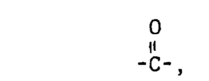 soit M lorsque T représente le groupe 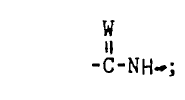
M représente l'hydrogène ou un alkyle droit ou ramifié en Ci-Ce ; un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ; un groupe aromatique ou hétéroaromatique non substitué, mono ou polysubstitué;
et leurs sels avec des acides minéraux ou organiques.

Les composés de formule (V ) sont des produits nouveaux. Ces composés sont préparés selon les étapes a), b) et c) du procédé décrit plus haut pour la préparation des composés de formule (1), suivies de l'élimination éventuelle des groupes N-protecteurs après chaque étape, en utilisant comme produit de départ une amine libre de formule : 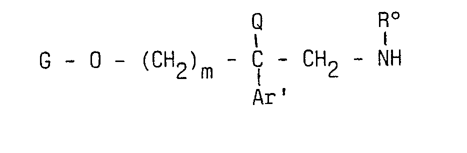 dans laquelle G, m, Q et Ar sont tels que définis ci-dessus, et R° représente l'hydrogène, un groupe méthyle, ou un groupe (CH₂)ₙ-L° où n est un nombre entier de 2 à 6 et L° est l'hydrogène ou un groupe amino protégé par un groupe N-protecteur.

Comme indiqué ci-dessus, les intermédiaires qui sont susceptibles de donner des sels avec des acides optiquement actifs peuvent être résolus afin de permettre la préparation des énantiomères des composés de formule (1).

On peut également prévoir la synthèse stéréospécifique d'intermédiaires qui ne donnent pas de sel permettant la séparation.

Un intermédiaire particulièrement adapté pour une telle synthèse stéréospécifique est l'alcool de formule (V) ci-dessus.

Ainsi, selon un autre de ses aspects, la présente invention concerne les énantiomères et un procédé pour la préparation des énantiomères des composés de formule (I) et de leurs sels ; lesdits énantiomères répondent à la formule (1^{*}), ci-après : 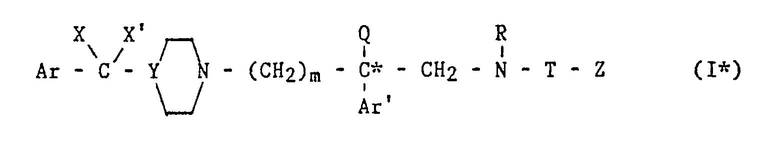 dans laquelle : Ar, Ar', Z, X, X , Y, Q, R, T et m sont tels que définis précédemment et "^{*}" signifie que l'atome de carbone ainsi marqué a une configuration absolue (+) ou (-) déterminée.

Ce procédé est caractérisé en ce que on traite un composé de formule: 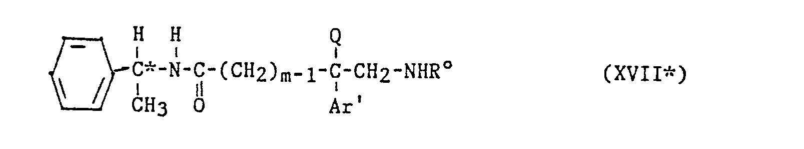 dans un solvant tel que par exemple le dioxanne, en milieu acide, par exemple en présence d'acide chlorhydrique pour fournir l'aminoacide de formule : 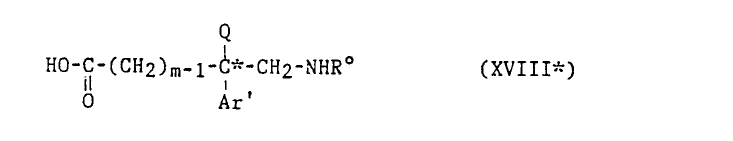 qui est estérifié dans un alcanol AIkOH, où Alk est un alkyle de 1 à 4 atomes de carbone, en milieu acide, puis on traite l'ester correspondant de formule : 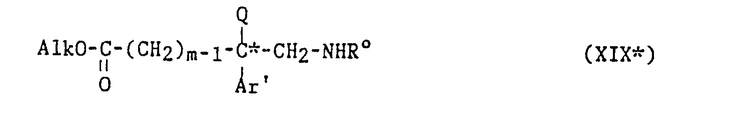 dans lequel Alk, Q, Ar', R et m sont tels que définis ci-dessus,
- soit avec un dérivé fonctionnel d'un acide de formule : 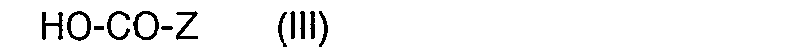
- soit avec un iso(thio)cyanate de formule : 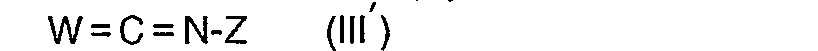 Z et W étant tels que définis ci-dessus,

selon les conditions opératoires identiques à celles utilisées pour la préparation des dérivés (IV) ci-dessus, pour obtenir l'ester de formule : 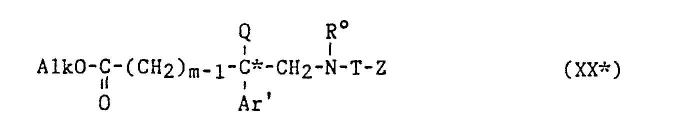 qui est alors réduit en alcool correspondant de formule :

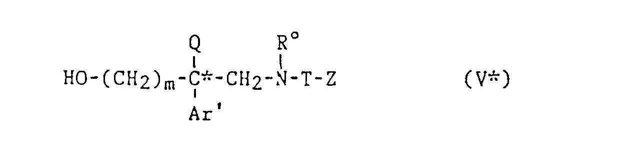

Ces alcools (V^{*}) correspondent aux composés de formule (V ) ci-dessus, dans laquelle m, Q, T, Ar et Z sont tels que définis précédemment, G est l'hydrogène et R° ° est R°, ce dernier ayant la signification précédemment définie, lesdits composés étant sous forme optiquement pure. Ces composés sont nouveaux et font partie de l'invention.

L'alcool (V^{*}) est transformé en dérivé méthanesulfonate de formule: 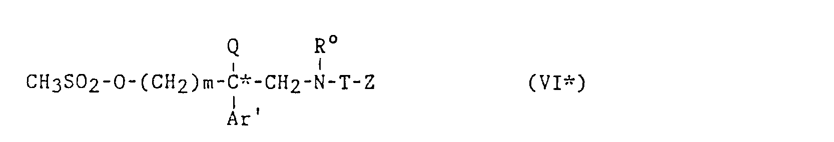 selon les conditions opératoires identiques à celles utilisées pour la préparation des dérivés (VI) ci-dessus.

Les dérivés (VI*) correspondent aux composés de formule (V') ci-dessus dans laquelle m, Q, T, Ar' et Z sont tels que définis précédemment, G est le méthanesulfonyle et R°° est R°, ce dernier ayant la signification précédemment définie, lesdits composés étant sous forme optiquement pure. Ces composés sont nouveaux et font partie de l'invention.

La substitution du mésylate (VI*) par une amine de formule : 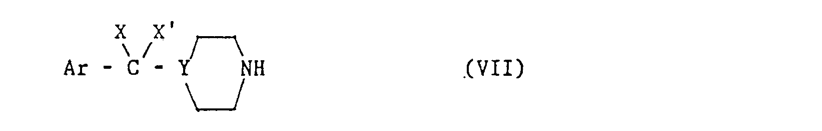 selon les conditions décrites pour l'obtention de (I) ci-dessus permet la préparation des dérivés (1^{*}), après déprotection éventuelle, lesquels sont ensuite éventuellement transformés en l'un de leurs sels selon les méthodes classiques de salification.

Les composés de formule (XVII^{*}) sont connus ou peuvent être aisément préparés selon la méthode décrite par G. HELMCHEN et al. Angew. Chem. Int. Ed. Engl., 1979, 1, 18, 65 ; selon le schéma suivant :

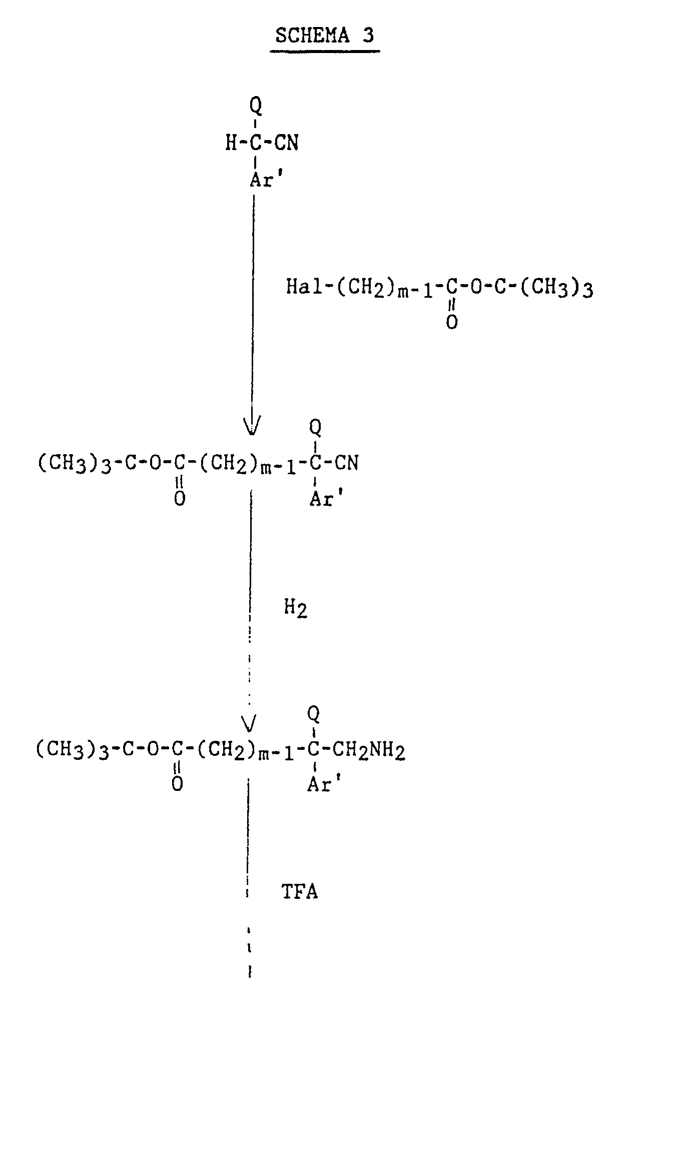

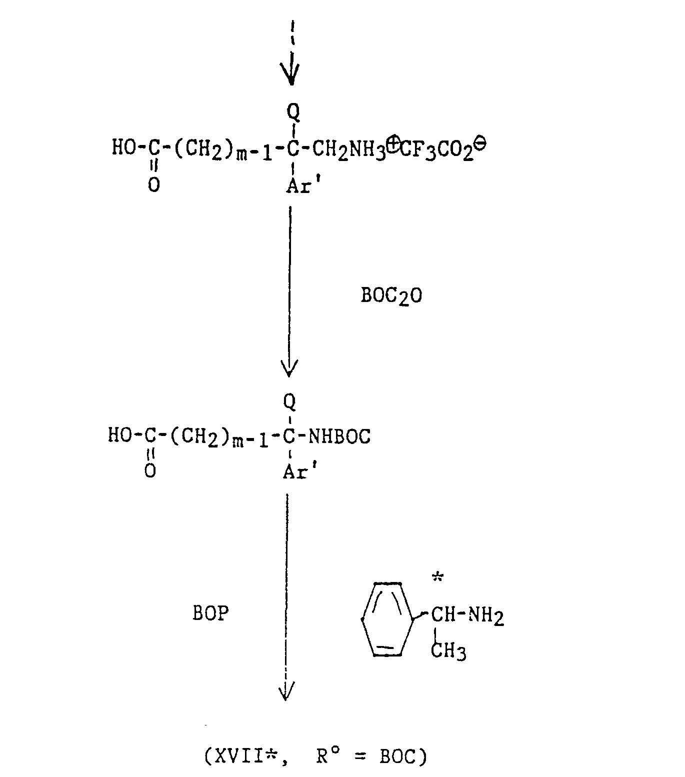

Les produits de formule (1^{*}) ainsi obtenus sont isolés, sous forme de base libre ou de sel, selon les techniques conventionnelles.

Lorsque le composé de formule (1^{*}) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, les composés de formule (1^{*}) peuvent être isolés sous forme d'un de leurs sels, par exemple le chlorhydrate ou l'oxalate ; dans ce cas, s'il est nécessaire la base libre peut être préparée par neutralisation dudit sel avec une base minérale ou organique.

Les composés selon l'invention ont fait l'objet d'essais biochimiques et pharmacologiques.

Les composés (1), (1^{*}) et les composés (XVI), où 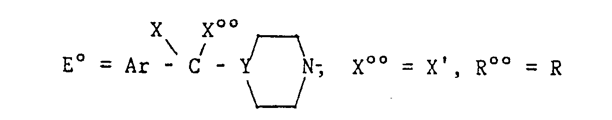 et leurs sels ont montré des propriétés antagonistes de la liaison de la substance P dans des essais réalisés sur des membranes de cortex de rat et de cellules lymphoblastiques IM9, selon M.A. CASCIERI et al., J. Biol. Chem., 1983, 258, 5158-5164 et D.D. PAYA et al., J. lmmunol., 1984, 133, 3260-3265.

Les mêmes composés et leurs sels ont montré des propriétés antagonistes de la liaison de la NKA dans des essais réalisés sur des membranes de duodénum de rat, selon L. BERGSTOM et al., Mol. Pharmacol., 1987, 32, 764-771.

Les mêmes composés et leurs sels ont montré des propriétés antagonistes d'agonistes spécifiques des récepteurs NK, , NK₂ , NK₃ dans des essais réalisés sur différents organes isolés, selon D.REGOLL et al., Trends Pharmacol. Sci., 1988, 9, 290-295.

Les mêmes composés et leurs sels ont montré des propriétés globales antagonistes NKi, NK₂, NK₃ dans des essais réalisés sur différents organes isolés, selon D. REGOLI et al., Trends Pharmacol. Sci., 1988, 9, 290-295 et Pharmacology, 1989, 38, 1-15.

Les mêmes composés et leurs sels ont montré des propriétés antagonistes de l'hypermotilité induite chez le rat par la substance P dans des essais pharmacologiques réalisés selon Elliot et al., Brain Res., 1986,381,68-76.

Les propriétés antagonistes de la salivation induite chez le rat par la substance P ou un agoniste spécifique NK₁ ([Sar⁹ Met (02)¹¹] SP) ont été mises en évidence par des essais pharmacologiques réalisés selon TAKEDA Y. and KRAUSE J.E., Proc. Natl. Acad. Sci. USA, 1989, 86, 392-396.

Les propriétés analgésiques ont été mises en évidence par des essais pharmacologiques réalisés chez le rat arthritique selon V. KAYSER et al.; Proceedings of the Vth World Congress on Pain, DUMMER R, GEBHART G.F. and BOND M.R. ed., Elsevier Biomedical Division, 1988, 72-79.

Les composés de la présente invention sont peu toxiques ; notamment, leur toxicité aigüe est compatible avec leur utilisation comme médicament. Pour une telle utilisation, on administre aux mammifères une quantité efficace d'un composé de formule (1), (1^{*}), (XVI) ou d'un de leurs sels pharmaceutiquement acceptables.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (1), (1^{*}) ou de formule (XVI) ou un de leurs sels pharmaceutiquement acceptables.

Les composés de formule (1), (1^{*}) ou (XVI) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

### EXEMPLE 1

Chlorhydrate de N [(benzyl-4 pipéridinyl-1)-4 (dichloro-3,4 phényl)-2 butyl] -dichloro-2,4 benzamide. SR 45672 A 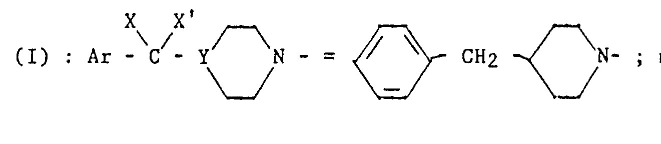 m ₌ 2 ; Q ₌ H ;

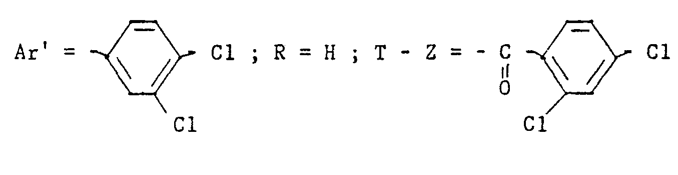

### A) Dichlorhydrate d'amino-1 (benzyl-4 pipéridinyl-1 )-4 (dichloro-3,4 phényl)-2 butane.

14,5 g de chlorhydrate de (benzyl-4 pipéridinyl-1 )-4 (dichloro-3,4 phényl)-2 butyronitrile sont mis en solution dans 400 ml d'éthanol 95°. Une solution de 20 ml d'ammoniaque concentré dans 40 ml d'eau et de nickel de Raney (10 % en poids de la quantité d'amine) sont ajoutés au mélange qui est ensuite placé sous atmosphère d'hydrogène sous forte agitation pendant 4 heures, temps au bout duquel 1,67 I d'hydrogène sont consommés. Après filtration du catalyseur, on concentre le filtrat sous vide, reprend le résidu dans l'acétate d'éthyle, lave à l'eau, sèche et concentre sous vide. Le résidu est repris dans une solution chlorhydrique dans le méthanol, filtré et recristallisé dans un mélange acétone/éther : 3/7. m = 10,2 g F = 210° C.

### B) SR 45672 A

2,3 g du produit obtenu précédemment et 1 g de chlorure de l'acide dichloro-2,4 benzoïque sont mis en solution dans 100 ml de dichlorométhane en présence de 0,03 g de triéthylamine. Le mélange réactionnel est agité pendant 4 heures à température ambiante puis concentré sous vide, repris dans l'eau, extrait à l'éther, séché sur MgS0₄ et concentré sous vide. Le résidu est chromatographié sur gel de silice, éluant : dichlorométhane/méthanol: 97/3. La concentration des fractions pures fournit un résidu qui est repris dans l'éther chlorhydrique. m = 1 g F = 86-87 C.

### EXEMPLE 2

Chlorhydrate de N [(benzyl-4 pipéridinyl-1)-5 (dichloro-3,4 phényl)-2 pentyl]-dichloro-2,4 benzamide . SR 45083 A 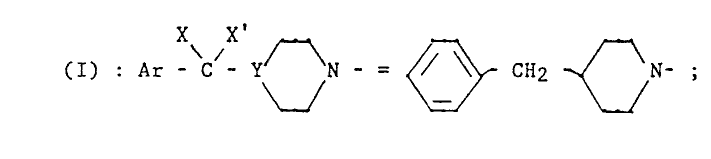 m ₌ 3 ; Q ₌ H ;

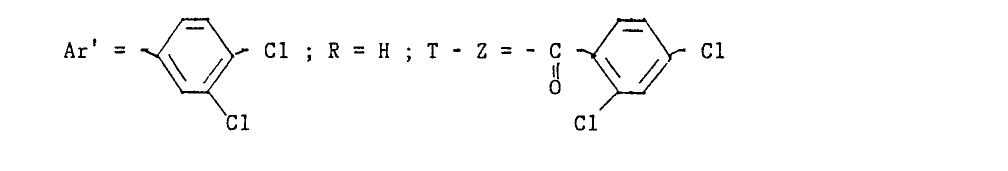

En procédant comme dans l'exemple 1 mais en utilisant comme produit de départ la (benzyl-4 pipéridinyl-1)-5 (dichloro-3,4 phényl)-2 pentylnitrile on obtient le SR 45083 A, recristallisé dans un mélange dichlorométhane, pentane. F = 98-100° C.

### EXEMPLE 3

Chlorhydrate hémihydrate de N [(benzyl-4 pipéridinyl-1)-4 (difluoro-3,4 phényl)-2 butyl]-diméthyl-2,4 benzamide. SR 46316 A 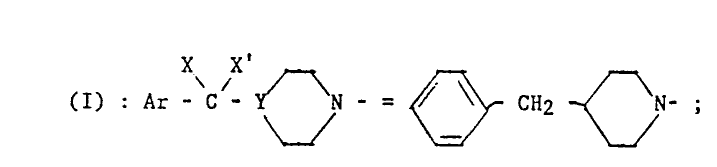 m = 2 ; Q ₌ H ; 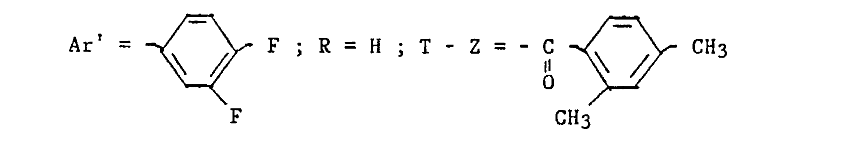 1,2 g de BOP sont ajoutés à une solution de 1 g d'amino-1 (benzyl-4 pipéridinyl-1)-3 (difluoro-3,4 phényl)-2 butane, 0,34 g d'acide diméthyl-2,4 benzoïque et 1 g de triéthylamine dans 50 ml de dichlorométhane. Le mélange réactionnel est agité pendant une heure à température ambiante et concentré sous vide. Le résidu est repris dans l'eau, extrait à l'éther, lavé à l'eau puis avec une solution de bicarbonate de sodium, séché sur MgS04 puis concentré sous vide. Le résidu est repris dans le chlorure de méthylène dans lequel on prépare le chlorhydrate qui est filtré et lavé à l'éther. m = 0,4 g F = 99-103 C.

Les composés décrits dans les tableaux 1 et 2 ont été préparés selon les exemples 1, 2 ou 3.

Dans la formule ci-dessous, le groupe Z indiqué dans la formule I est un groupe phényle non substitué, mono, di- ou trisubstitué par A, A', A".

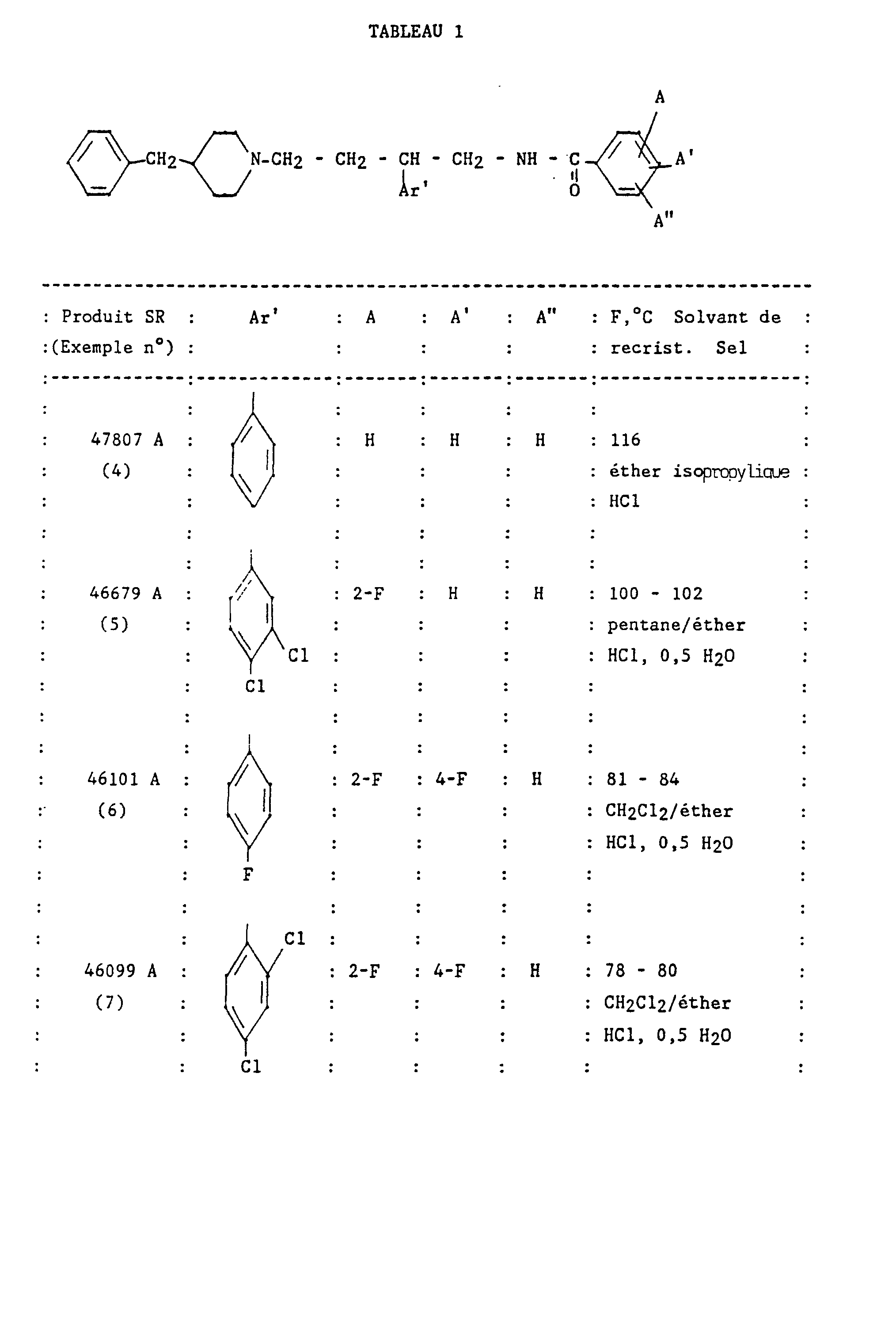

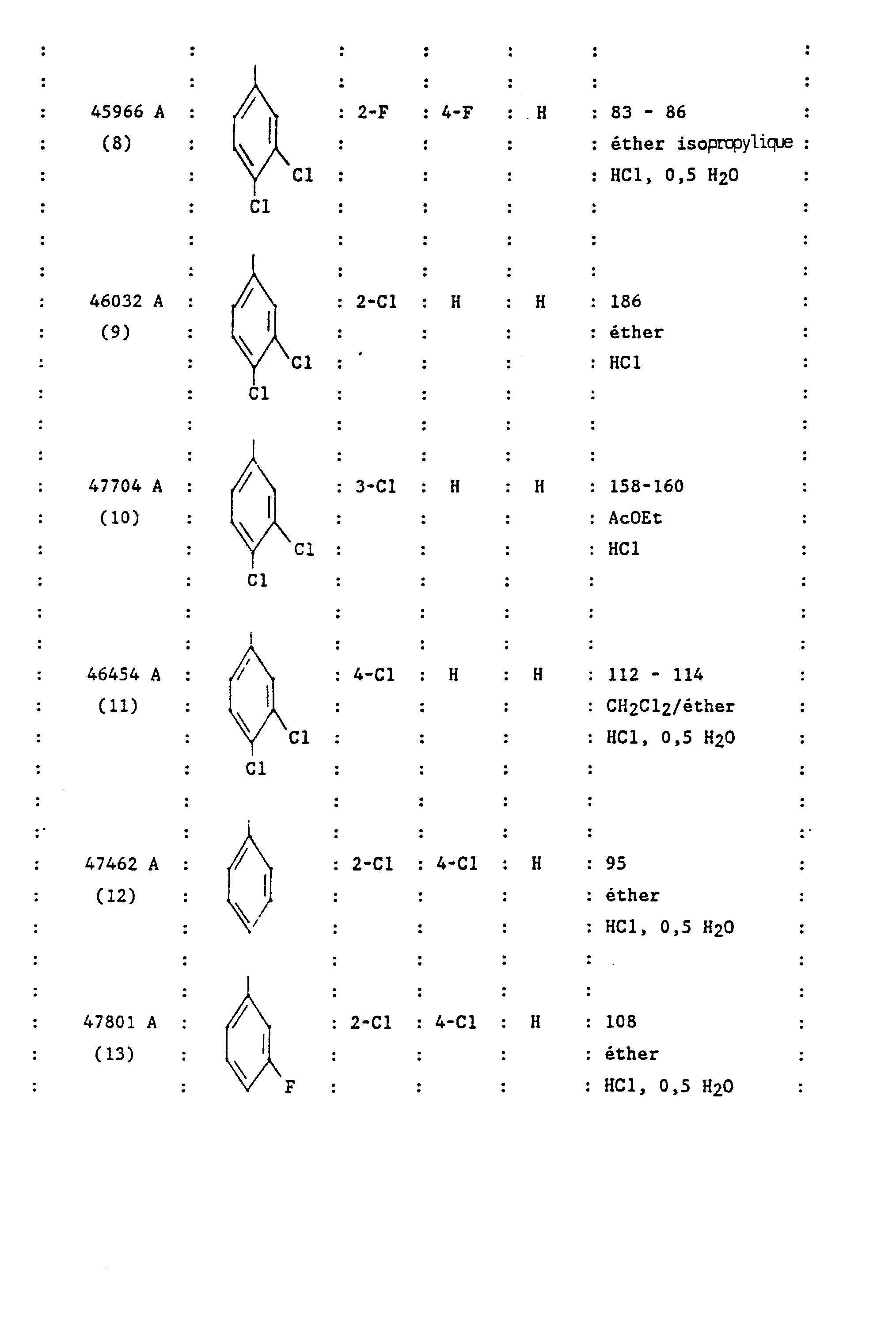

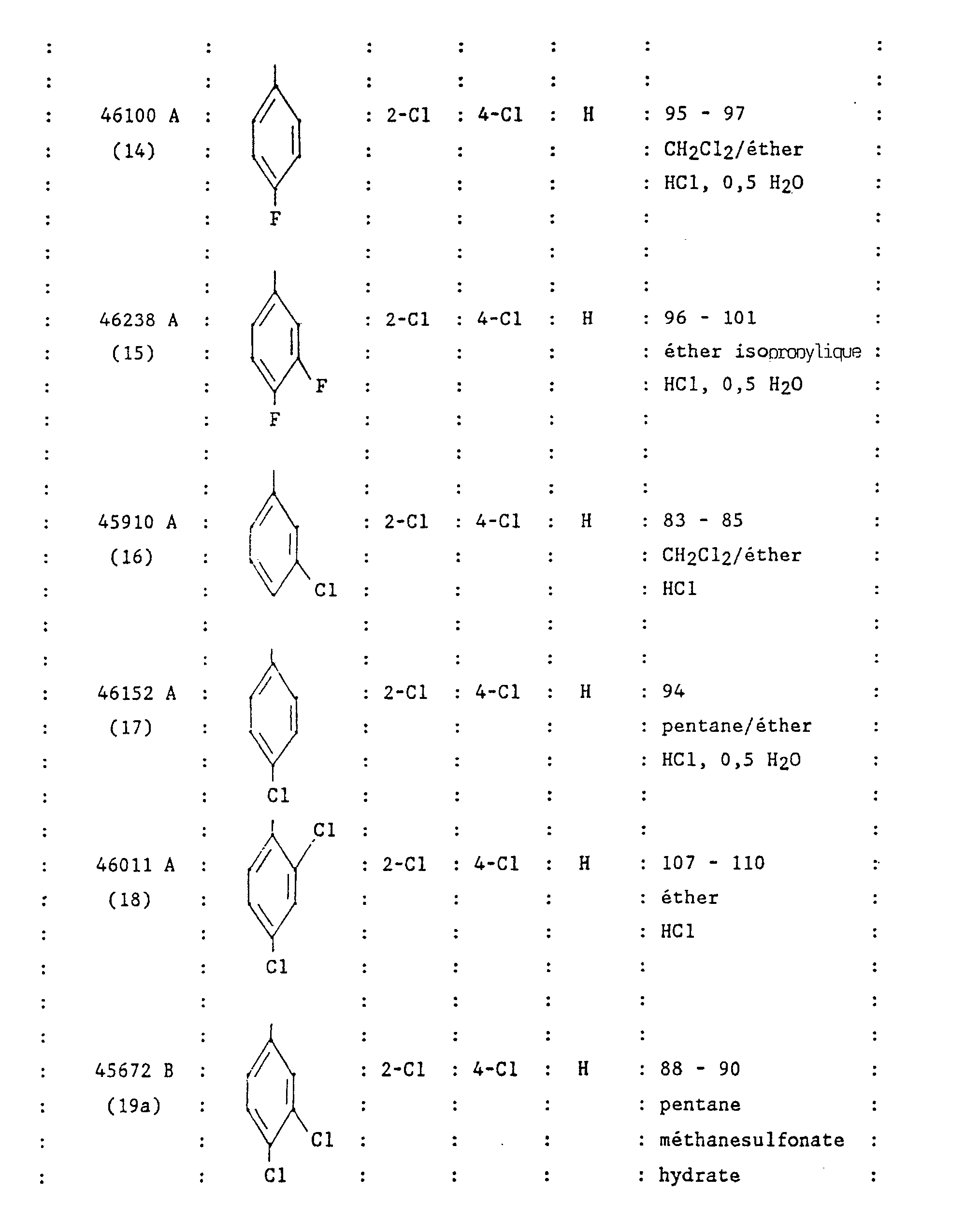

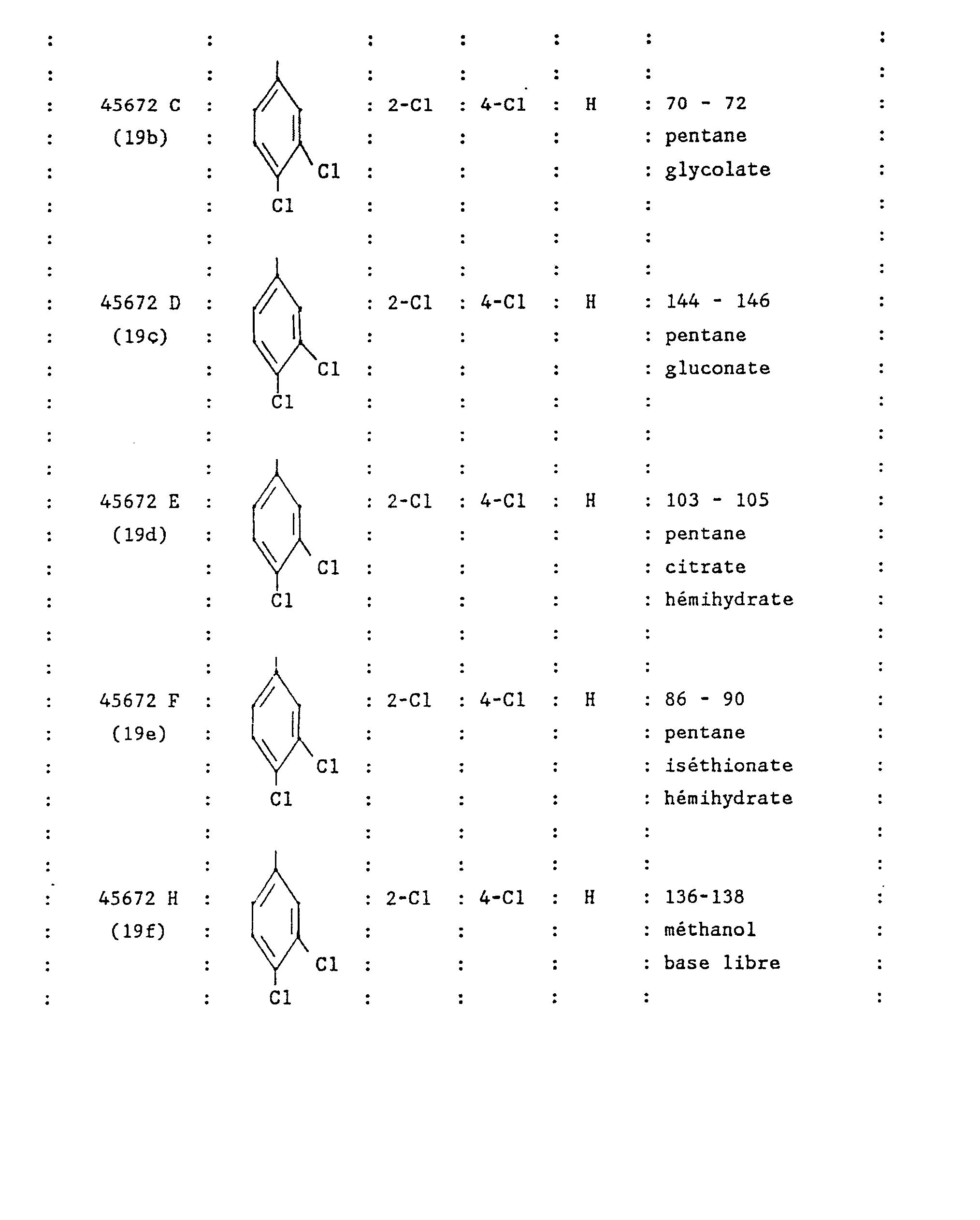

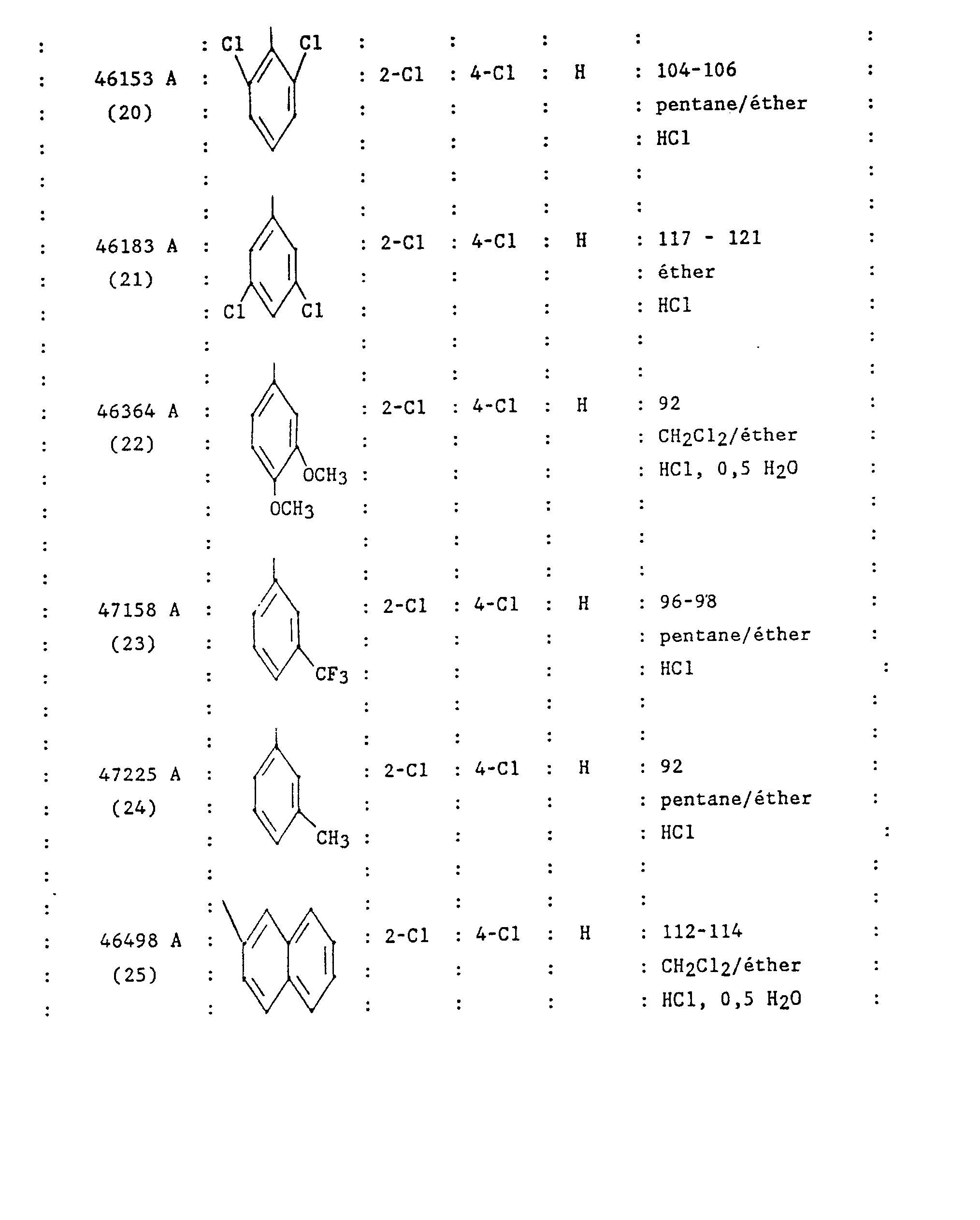

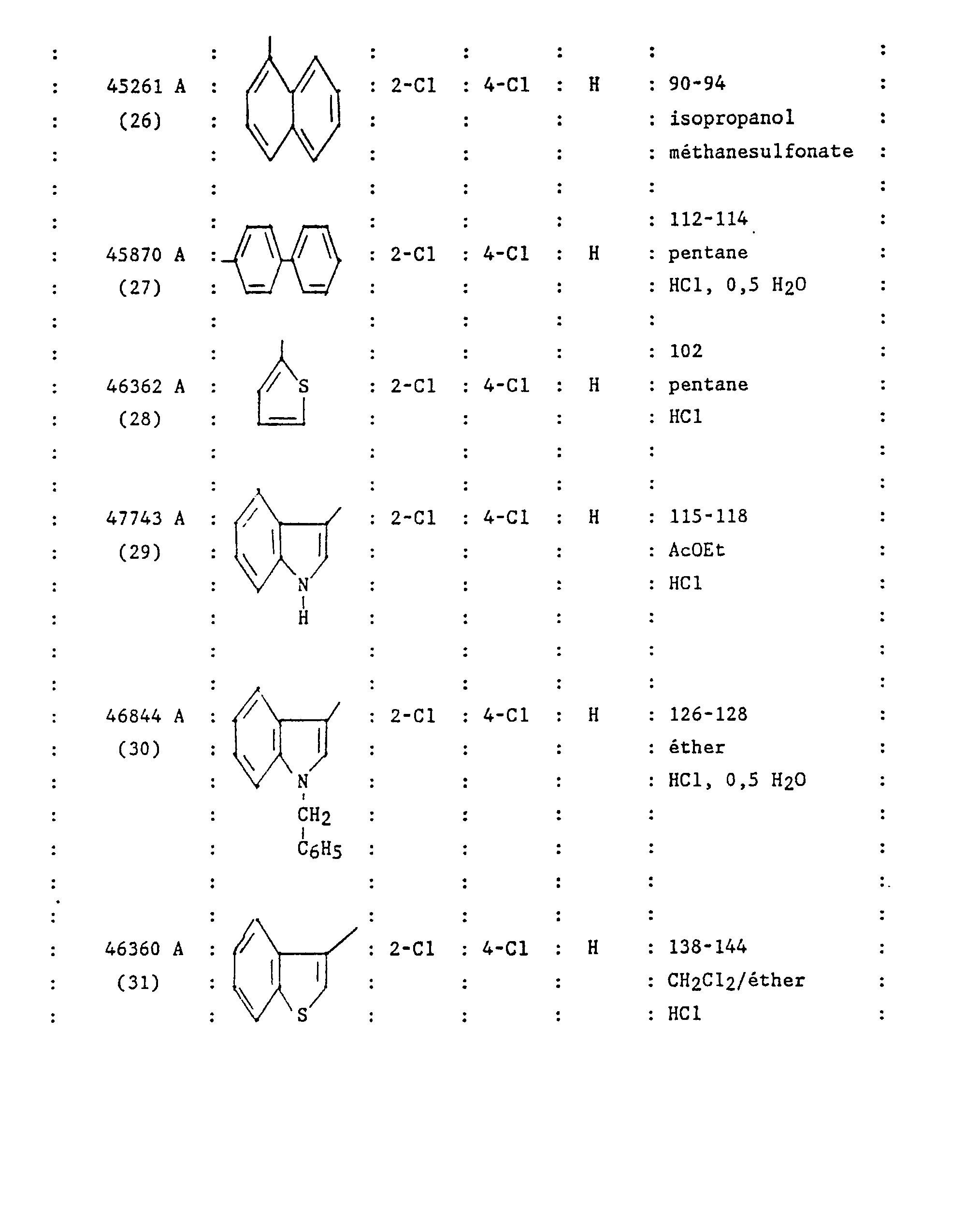

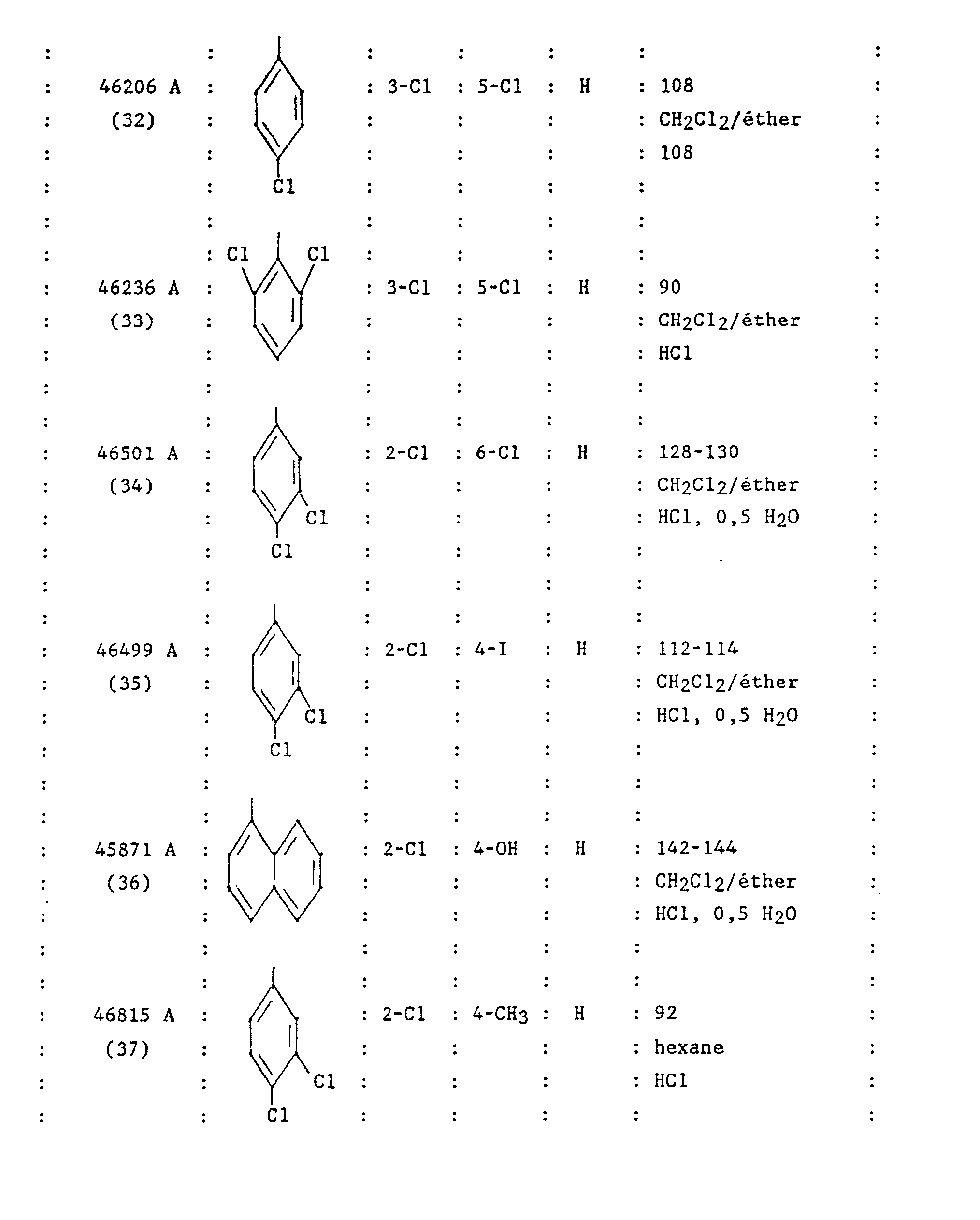

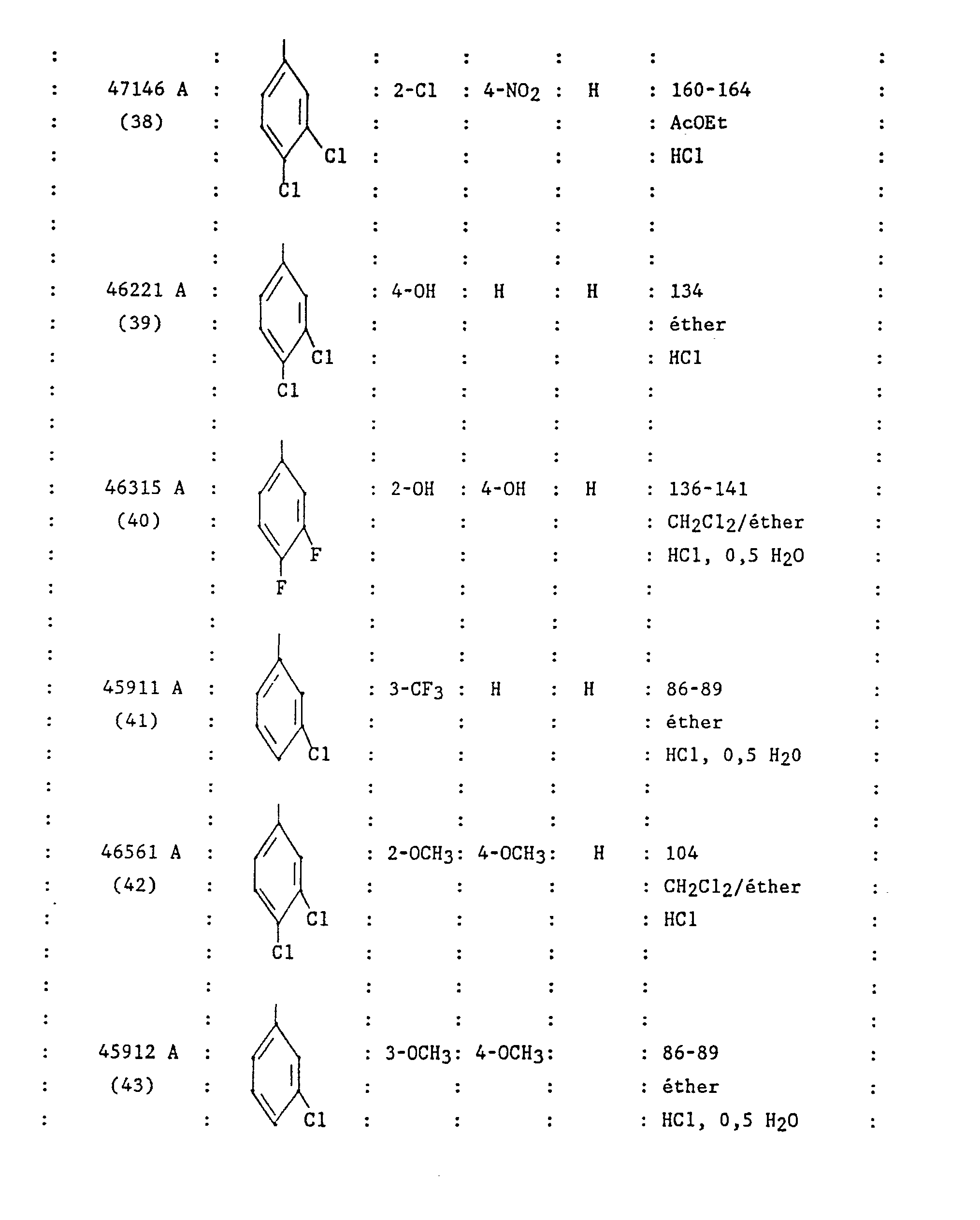

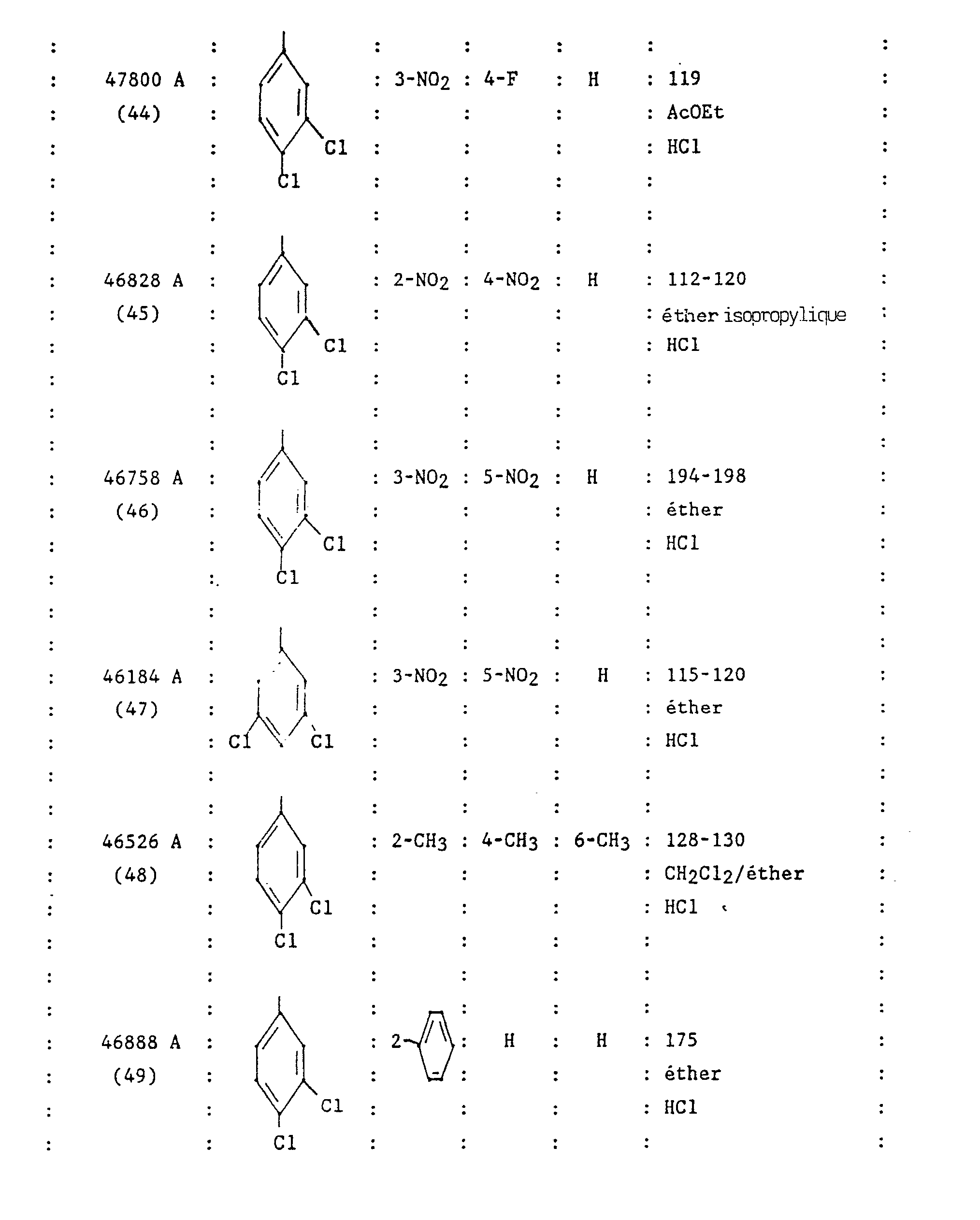

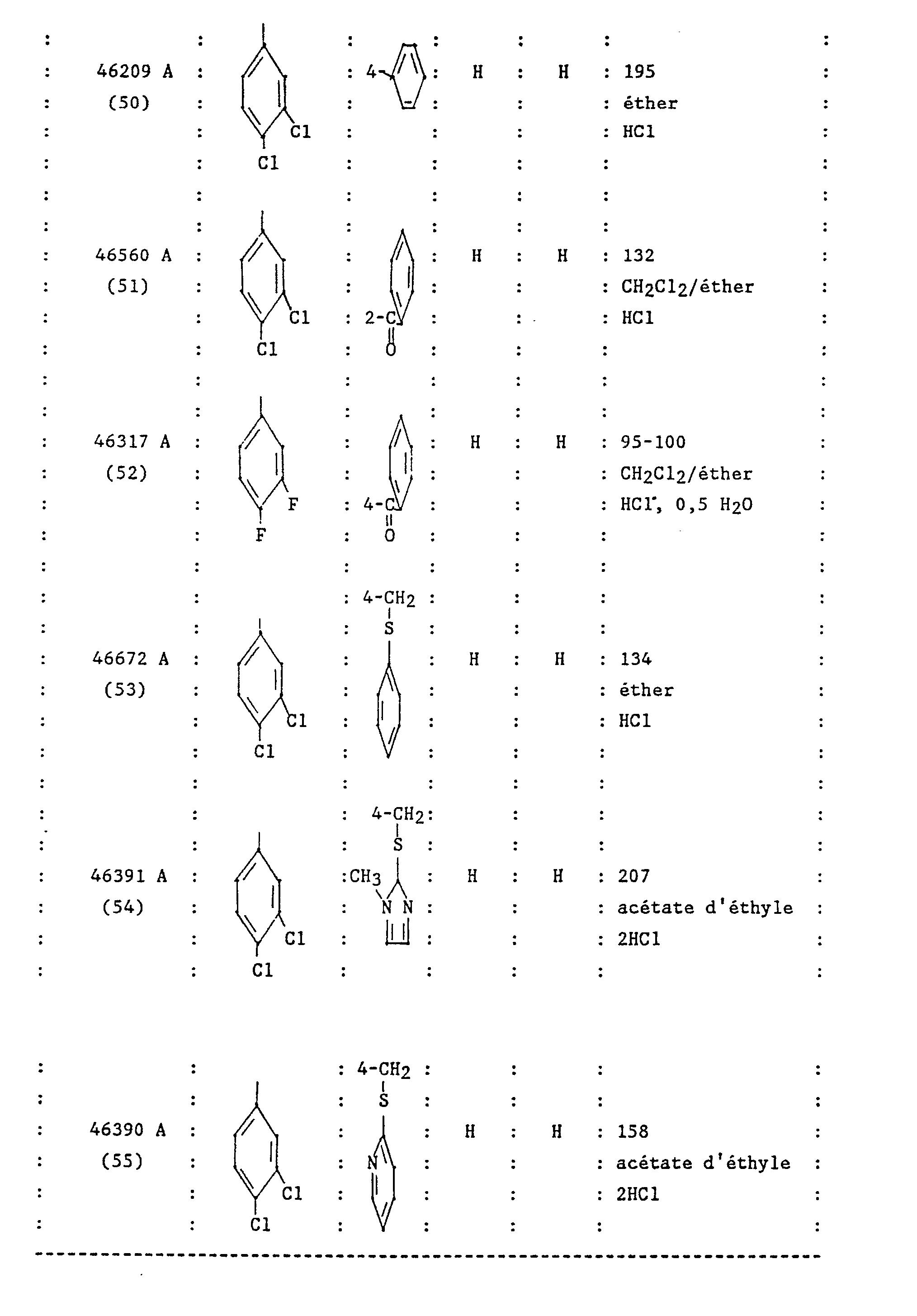

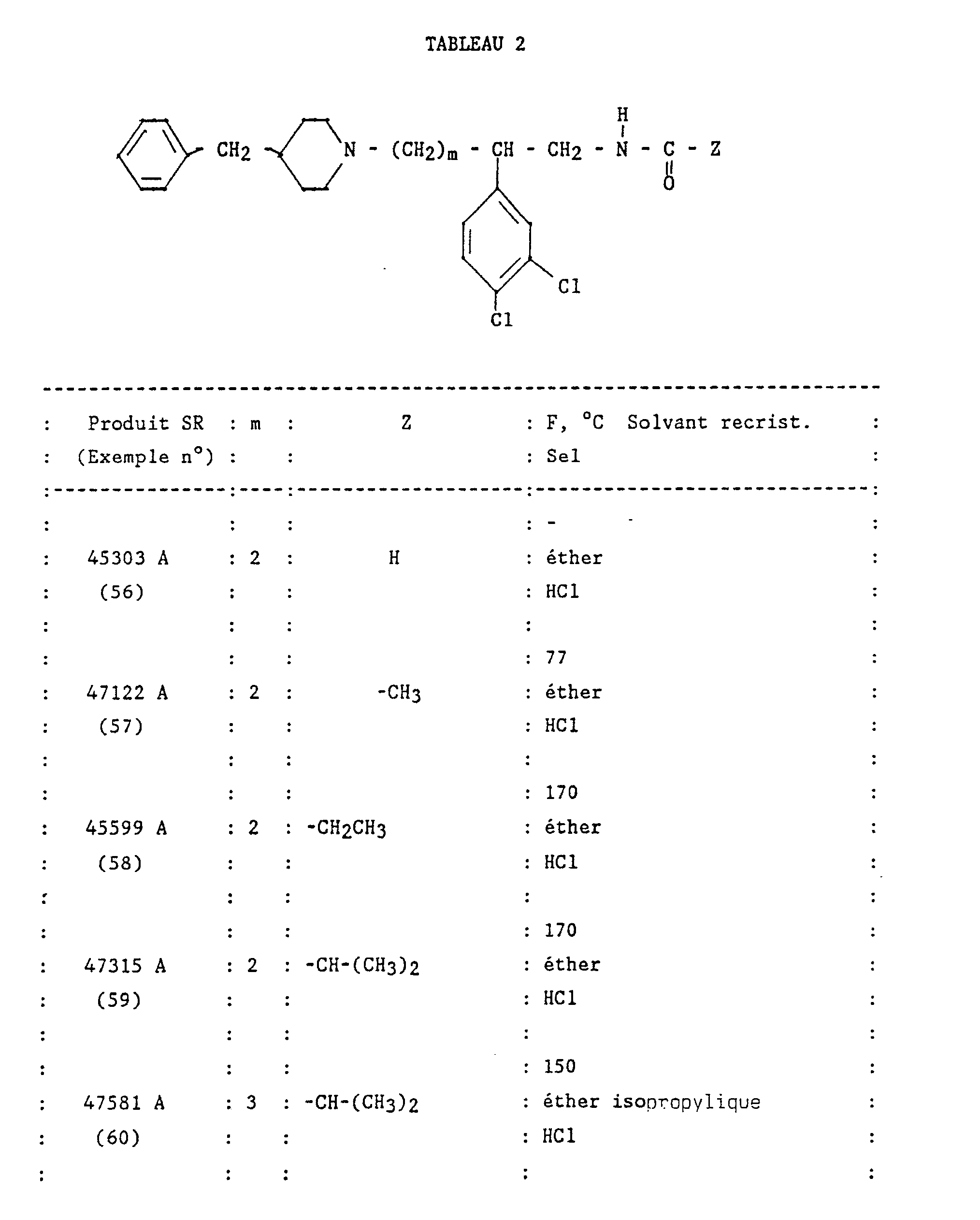

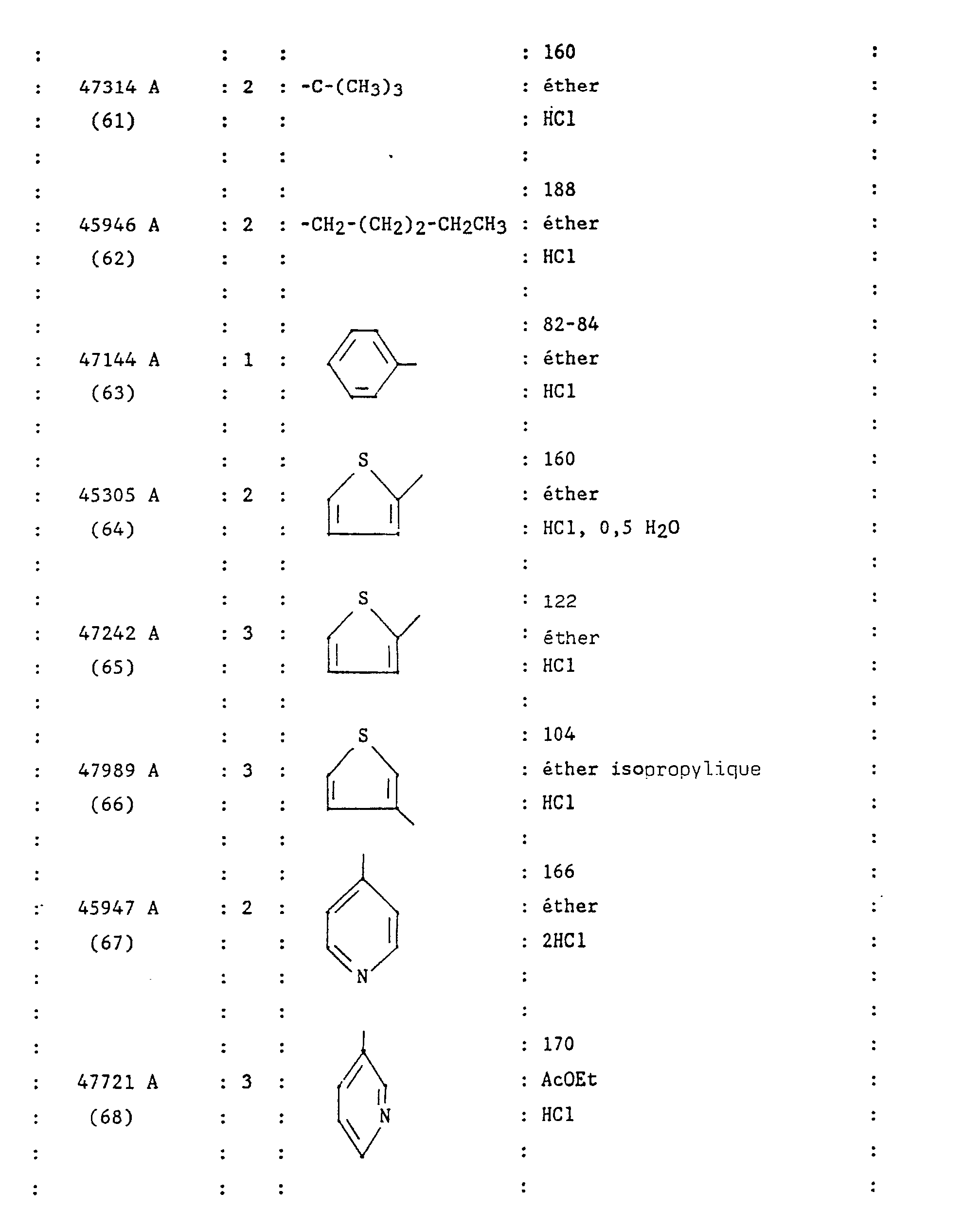

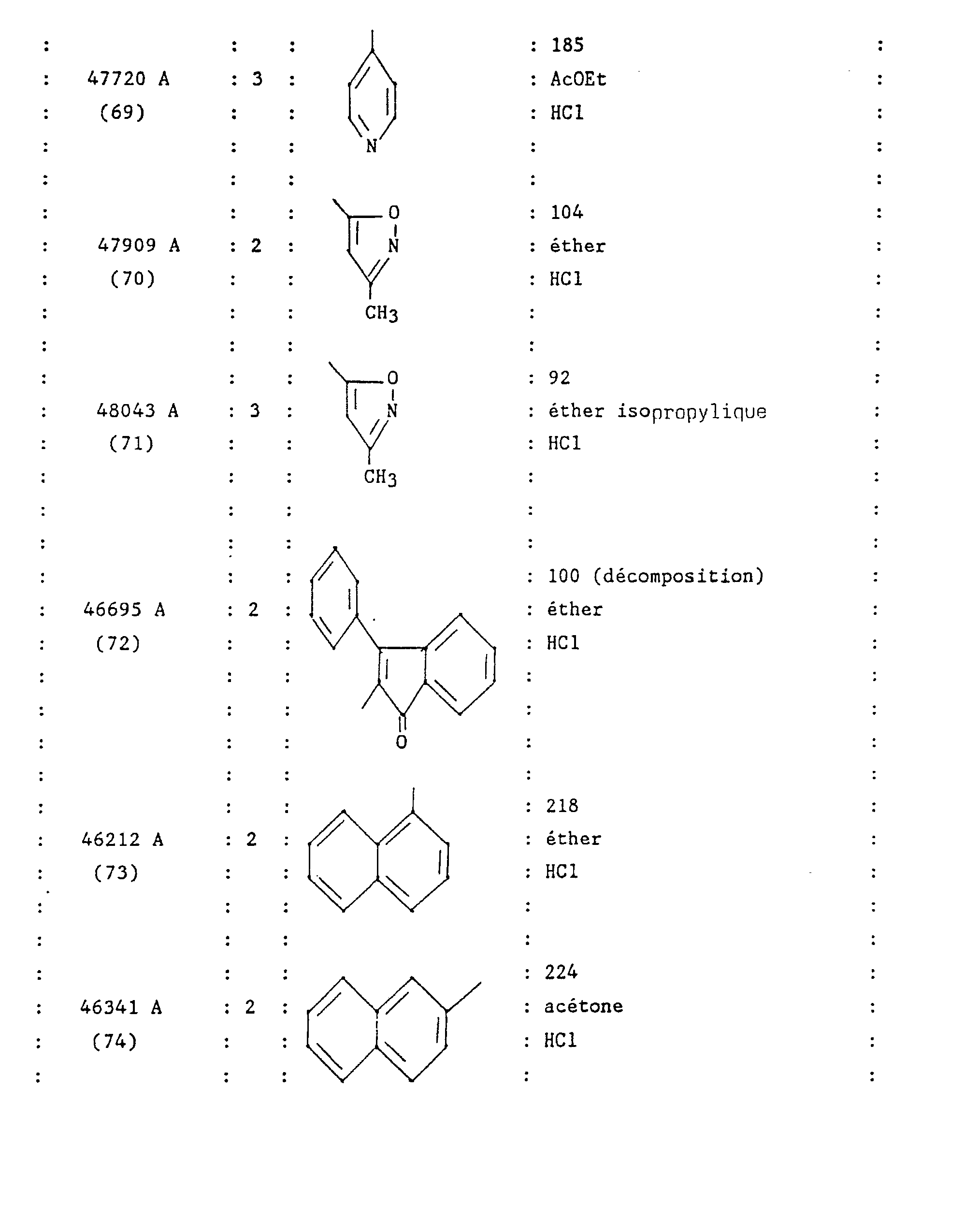

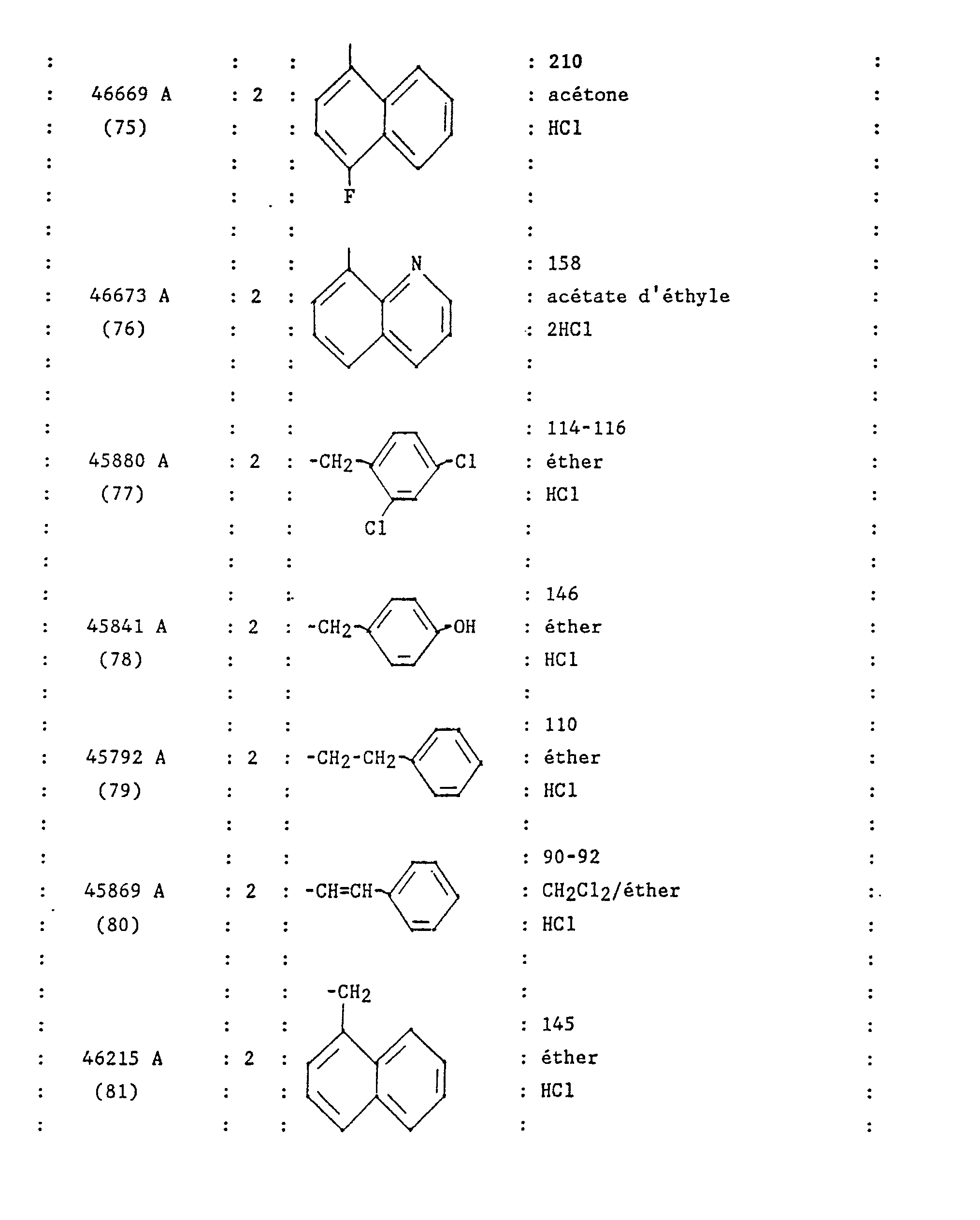

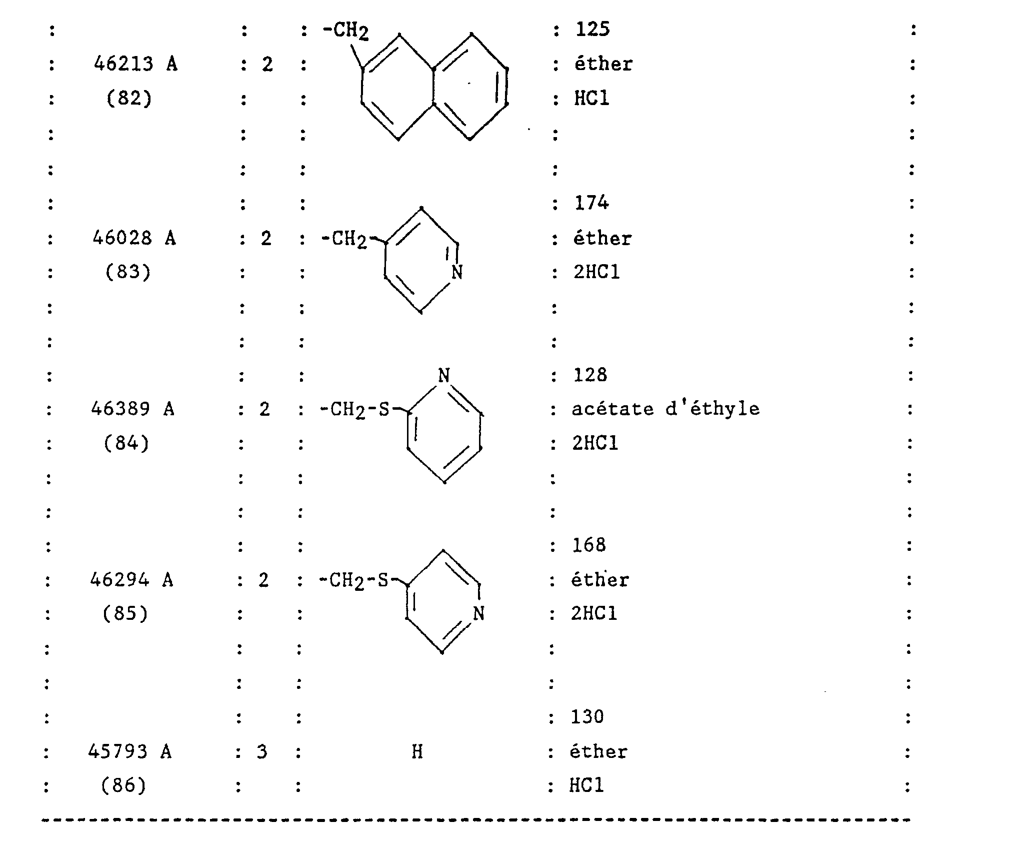

### EXEMPLE 87

Chlorhydrate de N'[(benzyl-4 pipéridinyl-1)-4 (dichloro-3,4 phényl)-2 butyl] N-naphtyl-1 urée. SR 45924 A. 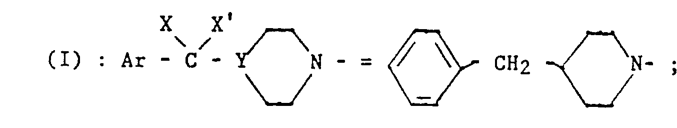 m ₌ 2 ; Q ₌ H ;

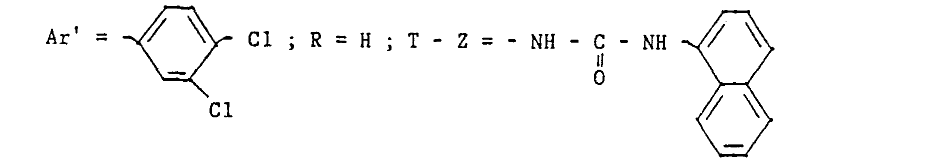

2,52 g d'amino-1 (benzyl-4 pipéridinyl-1 )-4 (dichloro-3,4 phényl)-2 butane sont mis en solution dans 30 ml de benzène anhydre puis on ajoute 1,09 g de naphtyl-1 isocyanate et laisse le mélange réactionnel sous agitation pendant une nuit à température ambiante. L'excès d'isocyanate est décomposé par addition de 10 ml de méthanol et chauffage du mélange à ébullition pendant 30 minutes.

Le mélange est concentré sous vide et le résidu est repris dans un mélange acétate d'éthyle-eau, lavé avec une solution de soude à 10 % puis à l'eau, décanté, séché sur MgSO₄ et concentré sous vide. Le résidu est repris dans l'acétone puis on ajoute de l'éther chlorhydrique, filtre le chlorhydrate et le concrétise dans l'éther. m = 3,3 g Y = 174°C

Les composés décrits dans le tableau 3 ont été préparés selon l'exemple 87.

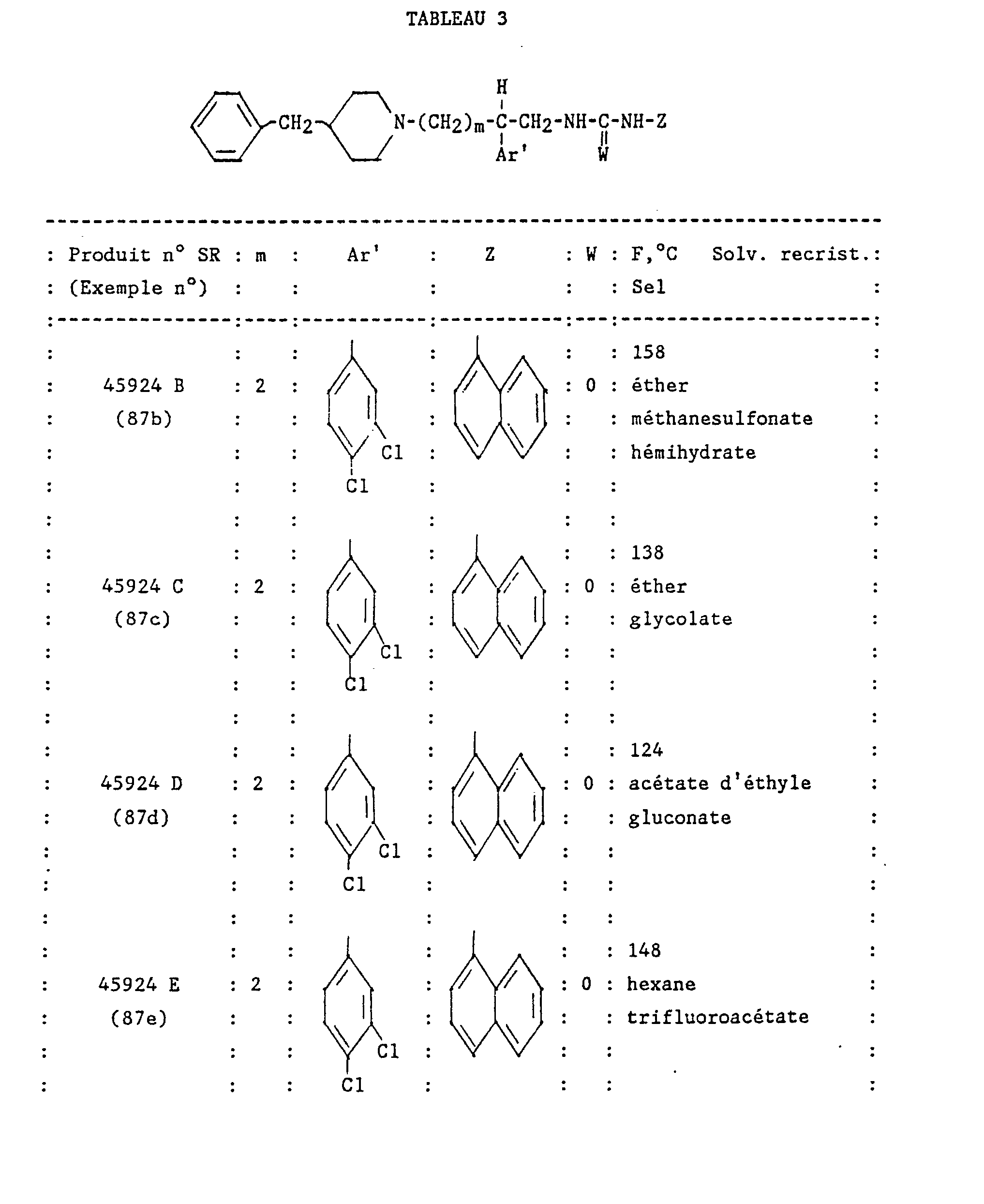

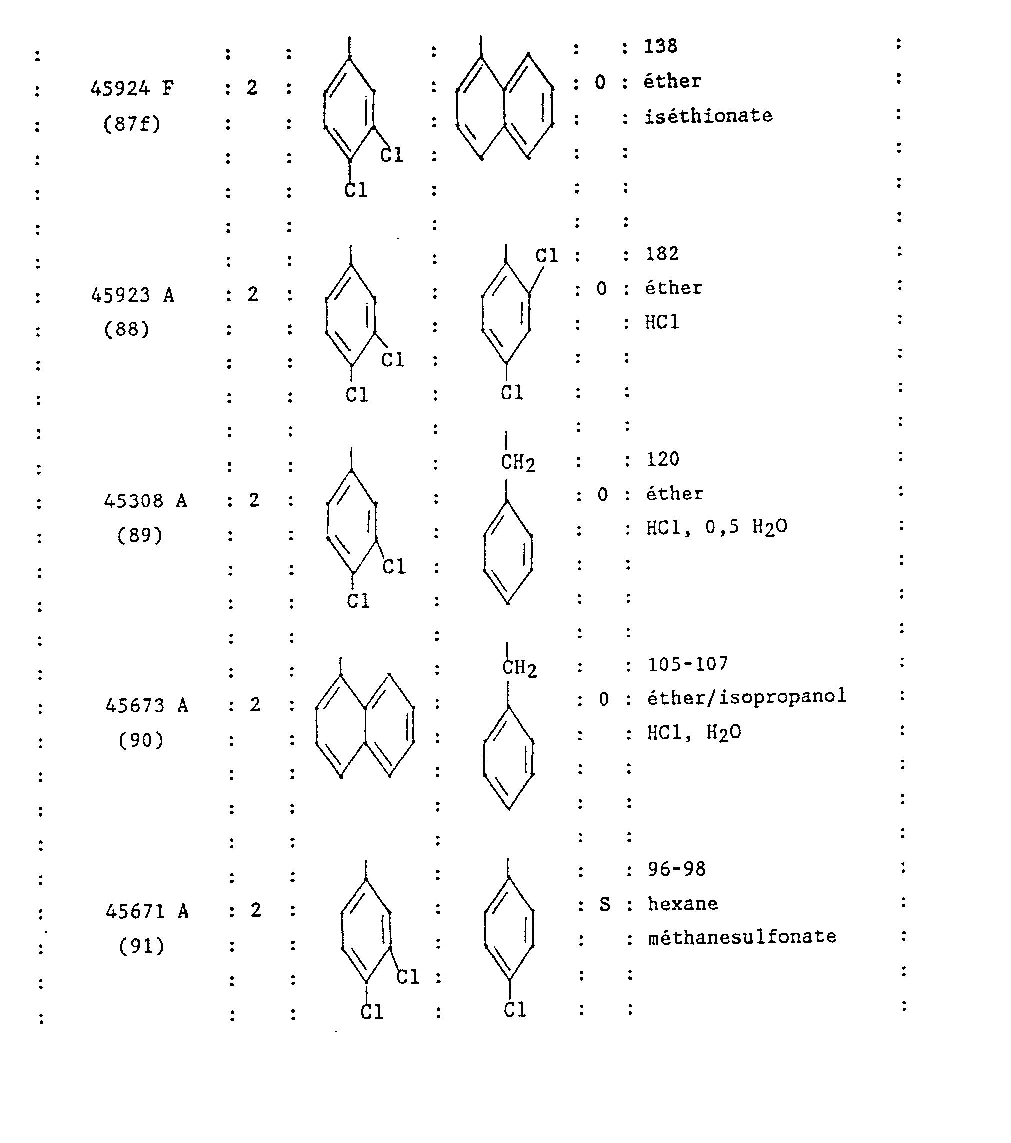

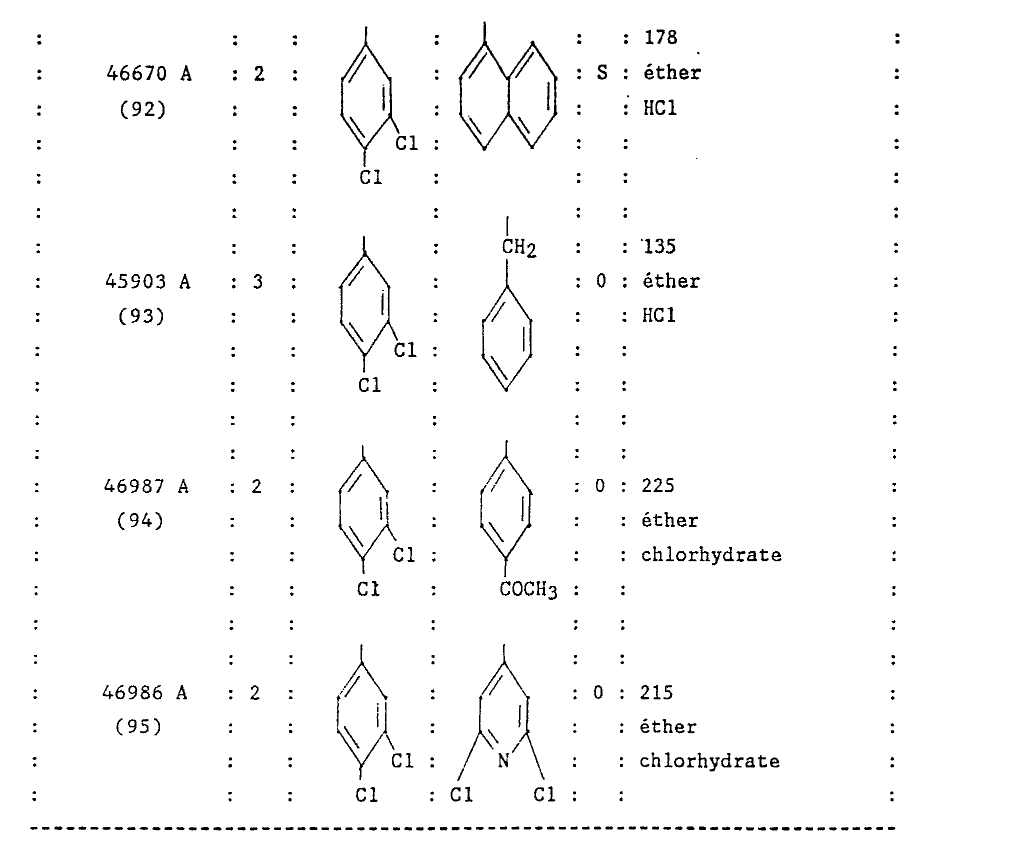

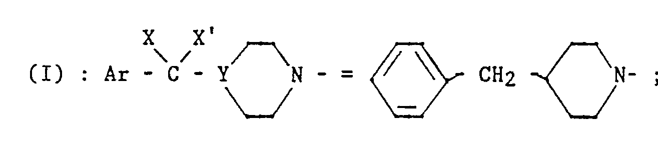

^{m = 2} ; Q ₌ H ;

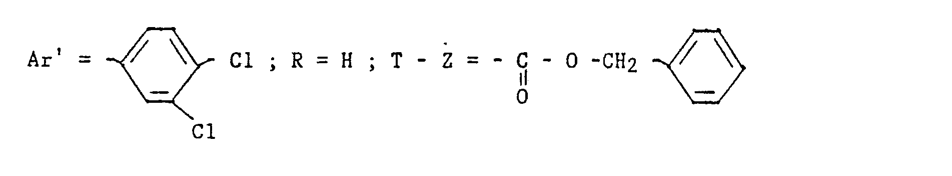

2,26 g de chlorhydrate d'amino-1 (benzyl-4 pipéridinyl-1 )-4 (dichloro-3,4 phényl)-2 butane et 0,89 g de chloroformiate de benzyle sont mis en solution dans 30 ml de dichlorométhane. Le mélange est refroidi à 0°C puis on ajoute 1,52 g de triéthylamine en solution dans 10 ml de dichlorométhane. Le mélange réactionnel est laissé 1 heure à température ambiante puis concentré sous vide. Le résidu est repris dans l'eau, extrait à l'acétate d'éthyle, lavé avec une solution de soude à 10 % puis avec une solution saturée de chlorure de sodium, séché sur MgS0₄ et concentré sous vide. Le résidu est chromatographié sur gel de silice, éluant : dichlorométhane/méthanol : 96/4. Après concentration sous vide des fractions pures, le résidu est répris dans l'éther, on ajoute de l'éther chlorhydrique et filtre le chlorhydrate. m = 1,7 g F = 130°C

### EXEMPLE 97

Chlorhydrate de N [(benzyl-4 pipéridinyl-1)-4 (dichloro-3,4 phényl)-2 butyl] carbamate d'éthyle. 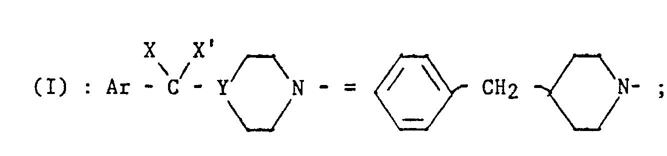 m = 2 ; Q ₌ H ;

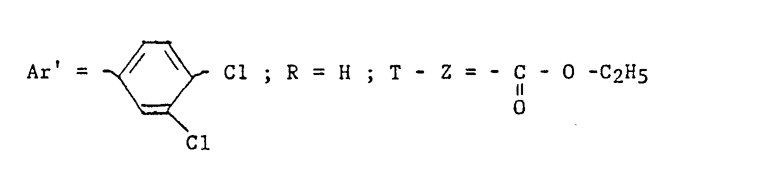

9,8 g de dichlorhydrate d'amino-1 (benzyl-4 pipéridinyl-1 )-4 (dichloro-3,4 phényl)-2 butane et 6,7 g de triéthylamine sont mis en solution dans 200 ml de dichlorométhane. On additionne à cette solution, goutte à goutte et à température ambiante, 2,28 g de chloroformiate d'éthyle et abandonne le mélange réactionnel pendant une heure à température ambiante, sous agitation. Le mélange réactionnel est concentré sous vide, le résidu est repris dans l'acétate d'éthyle/eau et lavé sucessivement avec une solution de soude à 5 %, à l'eau et avec une solution saturée de chlorure de sodium. La phase organique est séchée sur Na₂SO₄ et concentreé sous vide pour fournir un résidu qui est chromatographié sur gel de silice, éluant dichlorométhane/méthanol : 94/6. La concentration des fractions pures donne un résidu qui est repris dans l'acétate d'éthyle. On ajoute de l'éther chlorhydrique à la solution et filtre le chlorhydrate. m = 6,5 g F = 108-110°C

### EXEMPLE 98

Chlorhydrate de N-méthyl-N [(benzyl-4 pipéridinyi-1)-4 (dichloro-3,4 phényl)-2 butyl] dichloro-2,4 benzamide. SR 46650 A. 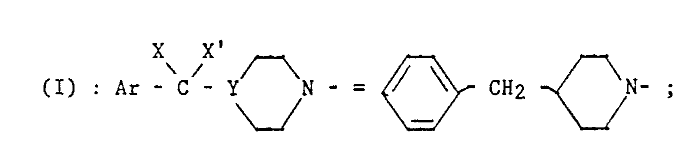 m = 2 ; Q ₌ H ;

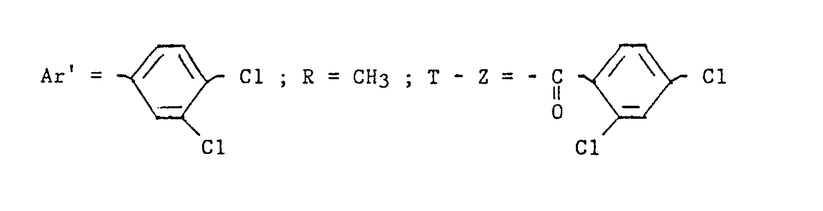

### a) Chlorhydrate de (benzyl-4 pipéridinyl-1)-4 (dichloro-3,4 phényl)-2 N-méthylamino-1 butane.

6,5 g du produit obtenu dans l'exemple 97 et 1,6 g d'hydrure de lithium aluminium sont mis en solution dans 150 ml de tétrahydrofuranne et chauffés à reflux pendant 3 heures. Le mélange réactionnel est hydrolysé par addition d'une solution de soude 2 N puis filtré sur célite. Le filtrat est concentré sous vide, le résidu est repris dans l'acétate d'éthyle et l'addition d'éther chlorhydrique permet l'obtention du chlorhydrate. m = 4,3 g F = 234-236 °C

### b) SR 46650 A.

En faisant réagir le chlorure de l'acide dichloro-2,4 benzoïque sur le produit obtenu précédemment selon le mode opératoire décrit dans l'exemple 1 on obtient le SR 46650 A. F = 140-142°C

### EXEMPLE 99

Dichlorhydrate hémihydrate de N-(amino-1 hexyl) N [(benzyl-4 pipéridinyl-1)-4 (dichloro-3,4 phényl)-2 butyl] dichloro-2,4 benzamide. SR 46510 A.

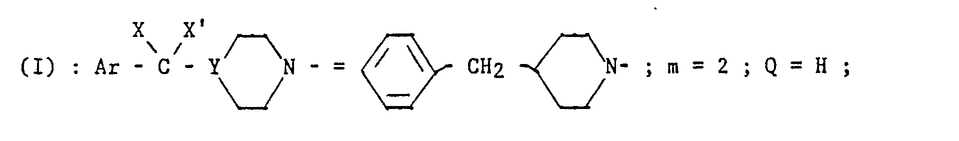

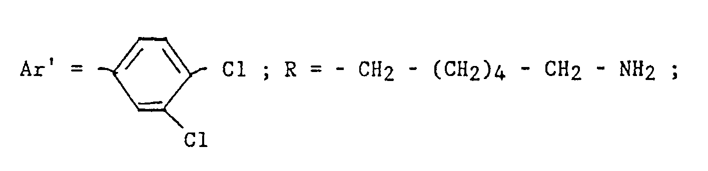

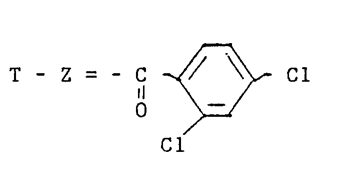

### a) (benzyl-4 pipéridinyl-1 )-4 (dichloro-3,4 phényl)-2 N(tritylaminopentylamido)-1 butane.

3 g de chlorhydrate d'amino-1 (benzyl-4 pipéridinyl-1)-4 (dichloro-3,4 phényl)-2 butane sont mis en suspension dans 60 ml de chlorure de méthylène en présence de 3,2 ml de triéthylamine. Après dissolution de la diamine, on ajoute 2,5 g d'acide tritylaminocaproïque puis 3,2 g de BOP. Le mélange réactionnel est agité à température ambiante pendant 30 minutes, lavé à l'eau, avec une solution de soude diluée puis à l'eau, décanté, séché sur MgS0₄, filtré et concentré sous vide. Le résidu est chromatographié sur gel de silice, éluant : chlorure de méthylène/méthanol : 95/5. La concentration des fractions pures permet l'obtention de 3,6 g du produit attendu.

### b) (benzyl-4 pipéridinyl-1 )-4 (dichloro-3,4 phényl)-2 N(tritylamino-1 hexylamino)-1 butane.

3,6 g du produit obtenu précédemment sont mis en solution dans 40 ml de tétrahydrofuranne et additionnés goutte à goutte à une suspension de 600 mg d'hydrure de lithium aluminium dans 20 ml de tétrahydrofuranne. Le mélange réactionnel est chauffé à reflux pendant 18 heures puis refroidi, hydrolysé, filtré et concentré sous vide. Le résidu est chromatographié sur gel de silice, éluant chlorure de méthylène/méthanol 80/20. La concentration des fractions pures fournit 1,9 g du produit attendu.

### c) N[(benzyl-4 pipéridinyl-l)-4 (dichloro-3,4 phényl)-2 N(tritylamino-1 hexylamino)-butyl]dichloro-2,4 benzamide.

1,9 g du produit obtenu précédemment sont mis en solution dans 30 ml de chlorure de méthylène. La solution est refroidie à -20° C puis on ajoute 0,57 g de chlorure de dichloro-2,4 benzoyle dans 10 ml de chlorure de méthylène. On laisse revenir le mélange à température ambiante, le lave deux fois à l'eau, décante, sèche sur MgSO₄ et concentre sous vide. Le résidu est chromatographié sur gel de silice, éluant : chlorure de méthylène/méthanol : 95/5. La concentration des fractions pures fournit 1,5 g de l'amide attendue.

### d) SR 46510 A

1,5 g du dérivé tritylé obtenu précédemment sont mis en solution dans 15 ml d'acide formique à 50 % dans l'eau et agités à 60°C pendant une heure. Le mélange refroidi est filtré et le filtrat est concentré sous vide. Le résidu est repris dans l'eau, lavé à l'éther, alcalinisé avec de la soude, extrait au chlorure de méthylène, décanté, séché sur MgS0₄ et concentré sous vide. Le résidu obtenu est repris dans 5 ml de chlorure de méthylène et on ajoute de l'éther chlorhydrique jusqu'à pH = 1. m = 1g F = 100 °C (décomposition).

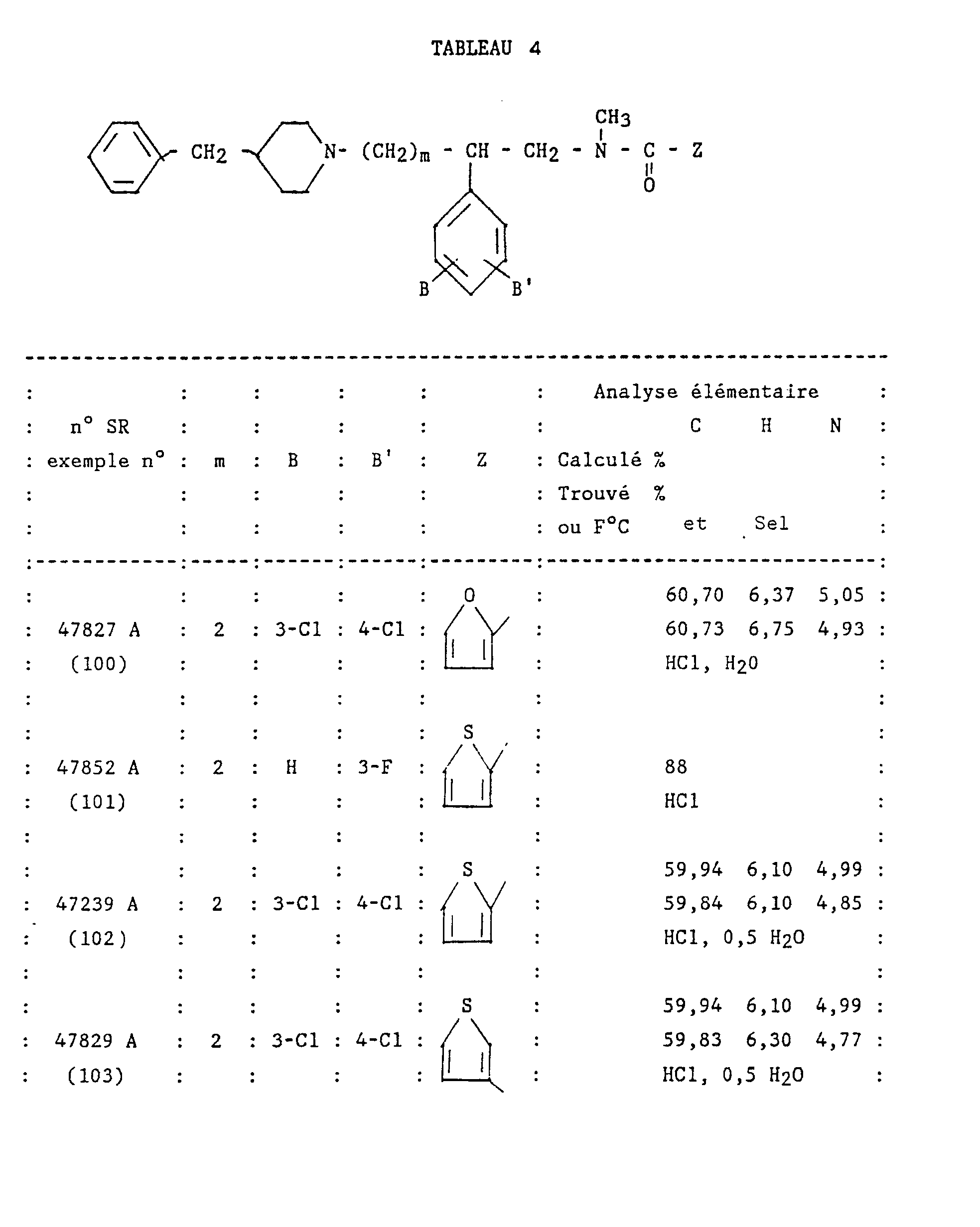

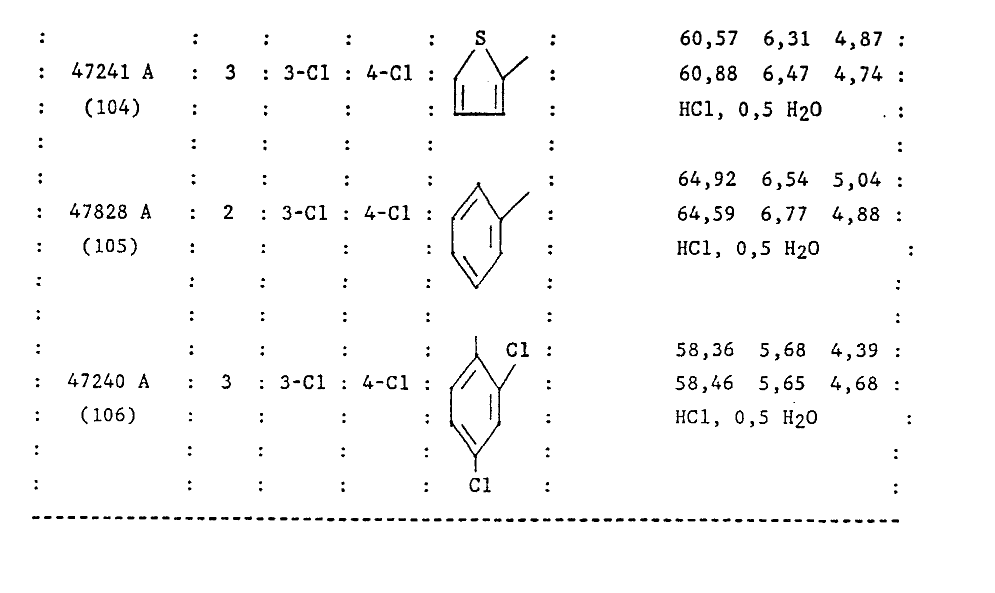

### EXEMPLE 107

Chlorhydrate de N [(benzyl-4 pipéridinyl-1)-4 (dichloro-3,4 phényl)-2 isobutyl-2 butyl]-dichloro-2,4 benzamide. SR 46753 A.

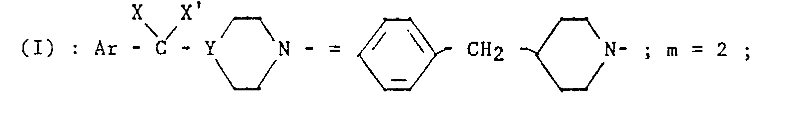

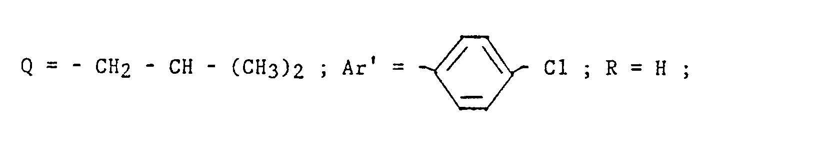

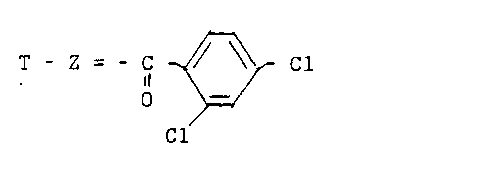

### a) (benzyl-4 pipéridinyl-1 )-4 (dichloro-3,4 phényl)-2 isobutyl-2 butyronitrile.

6 g de (benzyl-4 pipéridinyl-1)-4 (dichloro-3,4 phényl)-2 butyronitrile sont mis en solution dans 70 ml d'éther anhydre en présence de 0,62 g d'amidure de sodium. Le mélange réactionnel est chauffé à reflux pendant 2 heures, puis on le laisse revenir à température ambiante et ajoute 2,12 g de bromo-1 méthyl-2 propane. Le mélange réactionnel est chauffé à reflux pendant 24 heures et concentré sous vide. Le résidu est repris dans l'eau, extrait à l'acétate d'éthyle, décanté, séché sur MgSO₄, filtré et concentré sous vide.

Le produit attendu est obtenu après une purification par chromatographie sur gel de silice, éluant : hexane/acétate d'éthyle : 90/10.

### b) Amino-1 (benzyl-4 pipéridinyl-1)-4 (dichloro-3,4 phényl)-2 isobutyl-2 butane.

2,6 g du produit obtenu précédemment sont mis en solution dans un mélange de 30 ml d'ammoniaque et 20 ml d'eau. On ajoute une quantité catalytique de nickel de Raney et on hydrogène à pression atmosphérique à température ambiante. Le catalyseur est séparé par filtration et le filtrat est concentré sous vide. Le résidu est repris dans le méthanol et l'addition d'éther chlorhydrique permet l'obtention du chlorhydrate.

### c) SR 46753 A.

En faisant réagir le produit obtenu précédemment avec le chlorure de l'acide dichloro-2,4 benzoïque comme décrit dans l'exemple 1 on obtient le SR 46753 A. F = 126°C

Les composés décrits dans le tableau 5 ont été synthétisés selon l'exemple 107.

Dans la formule ci-dessous, le groupe Ar indiqué dans la formule 1 est un groupe dichloro-3,4 phényle et le groupe Z est un groupe dichloro-2,4 phényle.

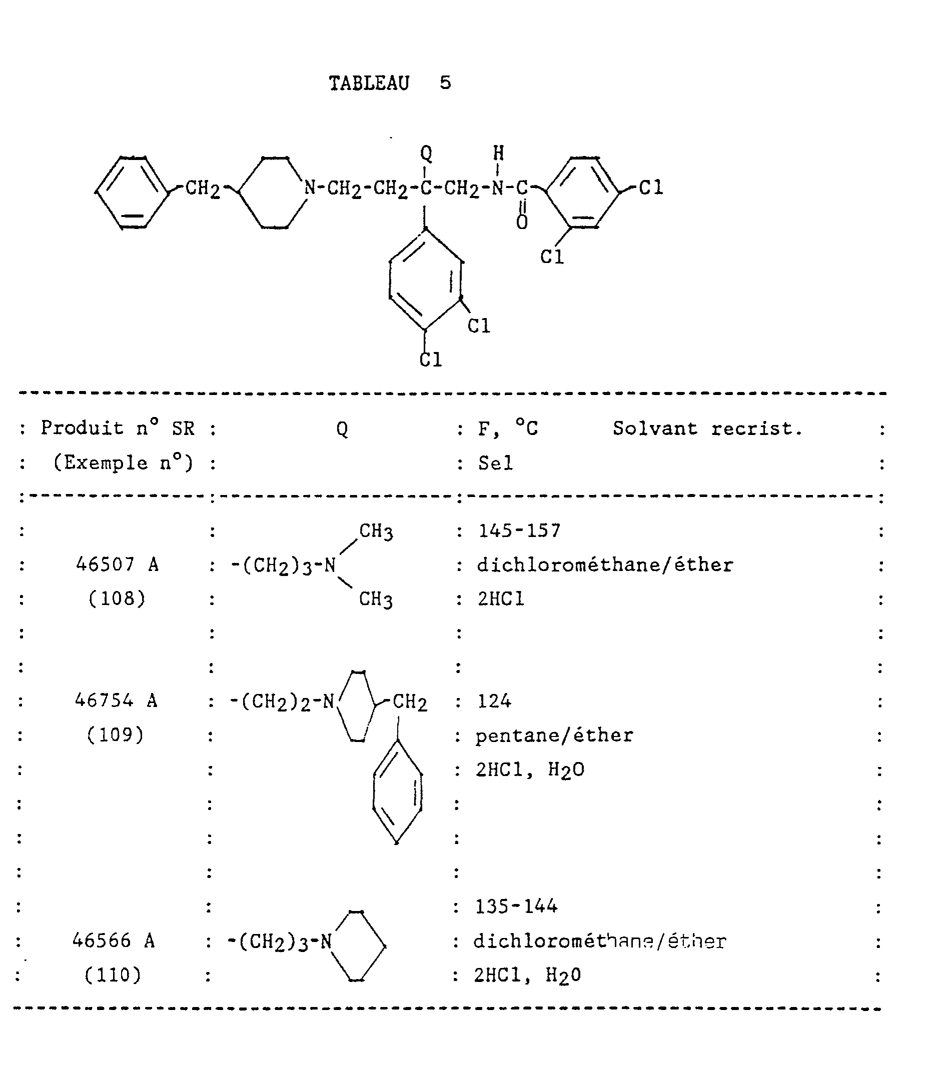

### EXEMPLE 111

Chlorhydrate de N [(benzoyl-4 pipéridinyl-1)-4 (dichloro-3,4 phényl)-2 butyl]-dichloro-2,4 benzamide. SR 46159 A. 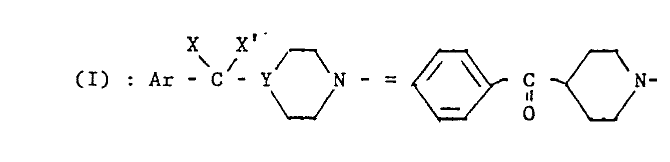 ; m = 2 ; Q = H ;

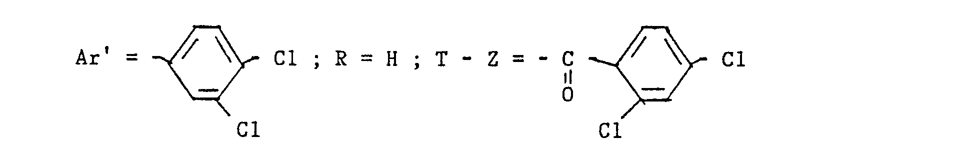

### a) (Dichloro-3,4 phényl)-3 (tétrahydropyranyl-2)-1 nitrile-3 propane.

20 g d'hydrure de sodium à 55-60 % dans l'huile sont mis en suspension dans 200 ml de tétrahydrofuranne sec. On ajoute goutte à goutte à 20 C, en 30 minutes, une solution de 85 g de dichloro-3,4 phényl acétonitrile dans 500 ml de tétrahydrofuranne puis on agite le mélange réactionnel à température ambiante pendant 2 heures. Le mélange est refroidi à -20 C et on ajoute une solution de 98 g de bromo-1 tétrahydropyranyl-2 éthane dans 100 ml de tétrahydrofuranne, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de chlorure de sodium, décante, sèche sur MgS0₄ et concentre sous vide.

Le résidu est chromatographié sur gel de silice, éluant : dichlorométhane. Les fractions de produit pur sont concentrées sous vide pour fournir 83,6 g d'une huile.

### b) Amino-1 (dichloro-3,4 phényl)-2 (tétrahydropyranyl-2)-4 butane.

83,6 g du nitrile obtenu précédemment sont mis en solution dans 100 ml d'éthanol absolu. On ajoute 350 ml d'ammoniaque concentré puis, sous balayage d'azote on ajoute du nickel de Raney (10 % de la quantité d'amine de départ. On hydrogène ensuite sous atmosphère d'hydrogène à température ambiante et pression ordinaire.

11,9 litres d'hydrogène sont absorbés en 3 heures. Le catalyseur est séparé par filtration sur célite, le filtrat est concentré sous vide, le résidu est repris dans une solution saturée de chlorure de sodium. Après extraction à l'éther et séchage sur MgSO₄ on obtient 82,5 g d'une huile.

### c) (Dichloro-2,4 benzoylamino)-1 (dichloro-3,4 phényl)-2 (tétrahydropyranyloxy-2)-4 butane.

80 g de l'amine obtenue précédemment sont mis en solution dans 800 ml de dichlorométhane. La solution est refroidie à 0 C, on ajoute 38,4 ml de triéthylamine puis 55 g de chlorure de l'acide dichloro-2,4 benzoïque. Le mélange réactionnel est alors agité à température ambiante pendant une heure puis lavé à l'eau. La phase organique est décantée, séchée sur MgS0₄ et concentrée sous vide pour fournir 120 g d'une huile.

### d) (Dichloro-2,4 benzoylamino)-1 (dichloro-3,4 phényl)-2 butanol-4.

120 g du produit obtenu précédemment sont mis en solution dans 1 litre de méthanol en présence de 12 g d'acide paratoluènesulfonique. Le mélange réactionnel est agité pendant 18 heures à température ambiante puis concentré sous vide. Le résidu est repris dans le dichlorométhane et lavé avec une solution à 10 % de carbonate de sodium. La phase organique est décantée et séchée sur MgS0₄ pour fournir 106 g d'une huile.

### e) (Dichloro-2,4 benzoylamino)-1 (dichloro-3,4 phényl)-2 mésyloxy-4 butane.

106 g de l'alcool obtenu précédemment sont mis en solution dans 1 I de dichlorométhane puis on ajoute à la solution refroidie à 0°C 44 ml de triéthylamine et 24,2 ml de chlorure de mésyle. Le mélange réactionnel est agité à 0°C pendant 45 minutes, lavé 3 fois à l'eau glacée, décanté, séché sur MgS0₄ et concentré sous vide.

Le résidu est recristallisé de l'éther isopropylique. m = 95 g

### f) SR 46159 A.

3 g du mésylate obtenu précédemment et 3,1 g de benzoyl-4 pipéridine sont mis en solution dans 7 ml de dichlorométhane et le mélange réactionnel est chauffé à reflux pendant 24 heures. Le mélange est dilué en dichlorométhane, lavé à l'eau, puis avec une solution de soude diluée, puis encore à l'eau. La phase organique est décantée, séchée sur MgSO₄ et concentrée sous vide. Le résidu est chromatographié sur gel de silice, éluant : dichlorométhane/méthanol : 70/30.

Les fractions de produit pur sont concentrées sous vide, le résidu est dilué dans le dichlorométhane et l'addition d'éther chlorhydrique permet l'obtention du chlorhydrate. m = 930 mg

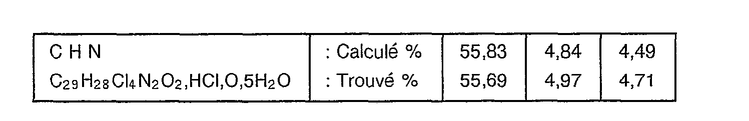

Les composés des exemples répertoriés dans le tableau 6 (I, Ar = dichloro-3,4 phényle ; Z = dichloro-2,4 phényle) ont été préparés selon l'exemple 111.

Les composés des exemples répertoriés dans le tableau 7 (I,Ar' = dichloro-3,4 phényle) et le tableau 8 (I, Ar' = dichloro-3,4 phényle ou trifluorométhyl-3 phényle) ont été préparés selon les exemples 1, 2 ou 3.

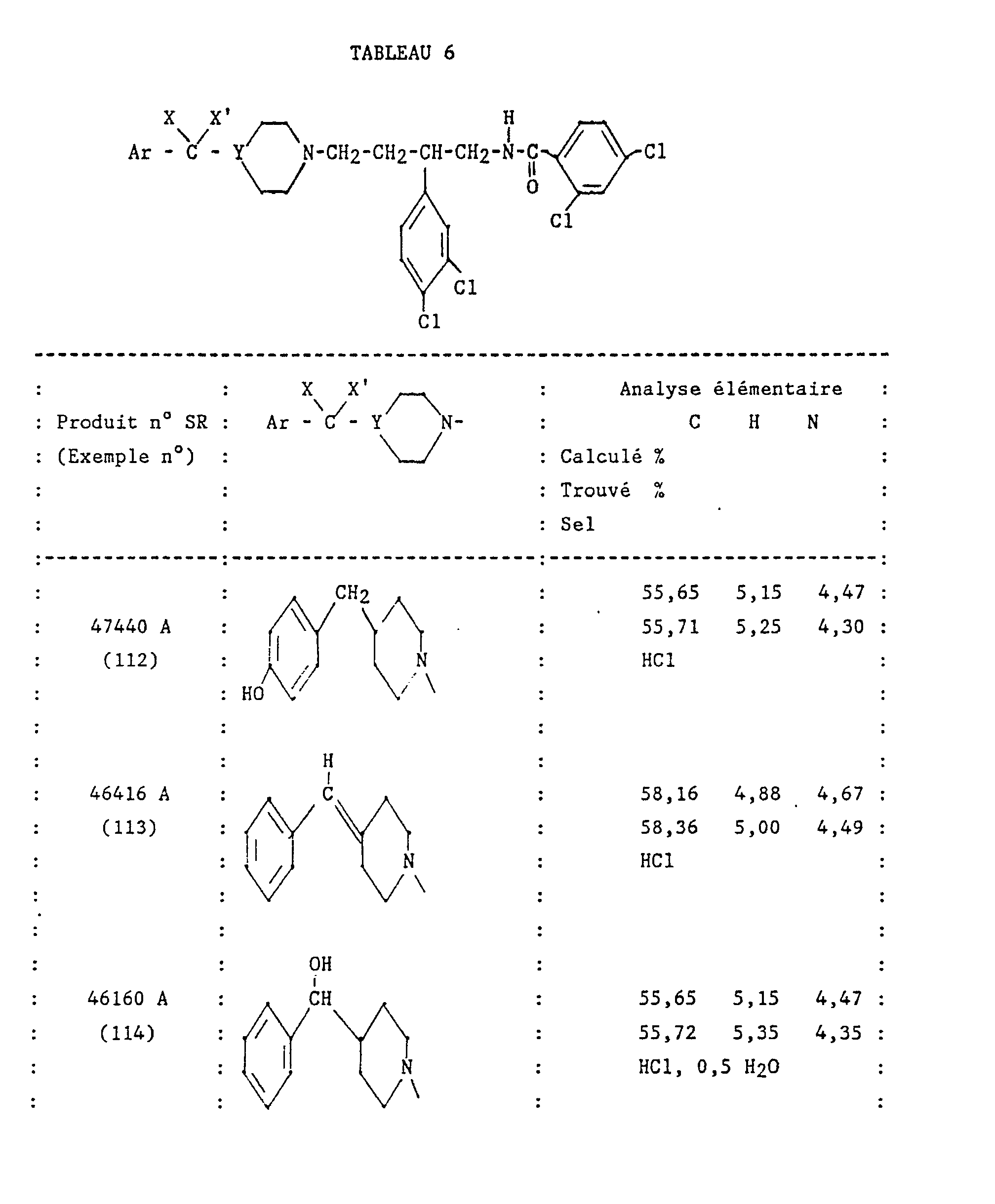

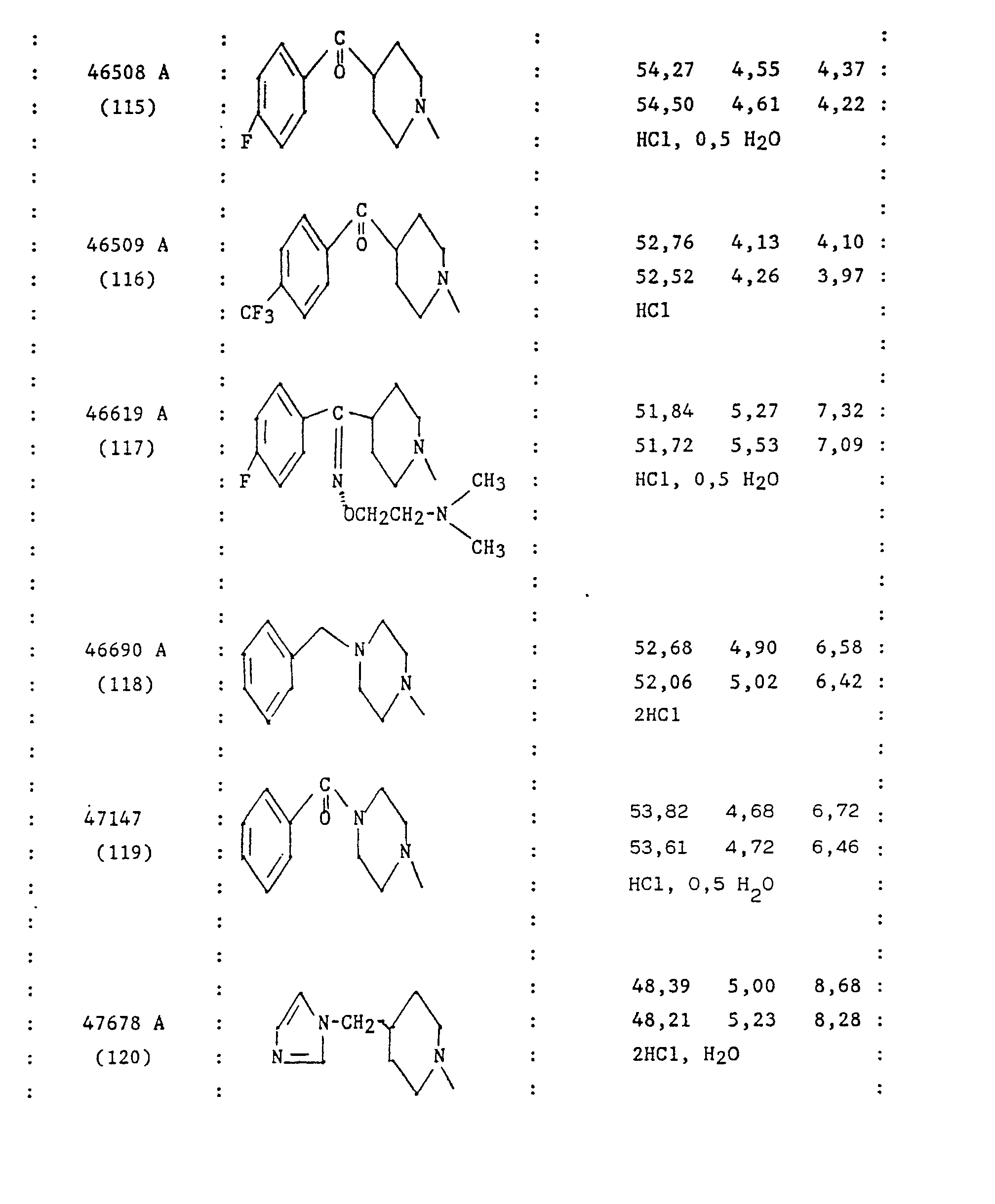

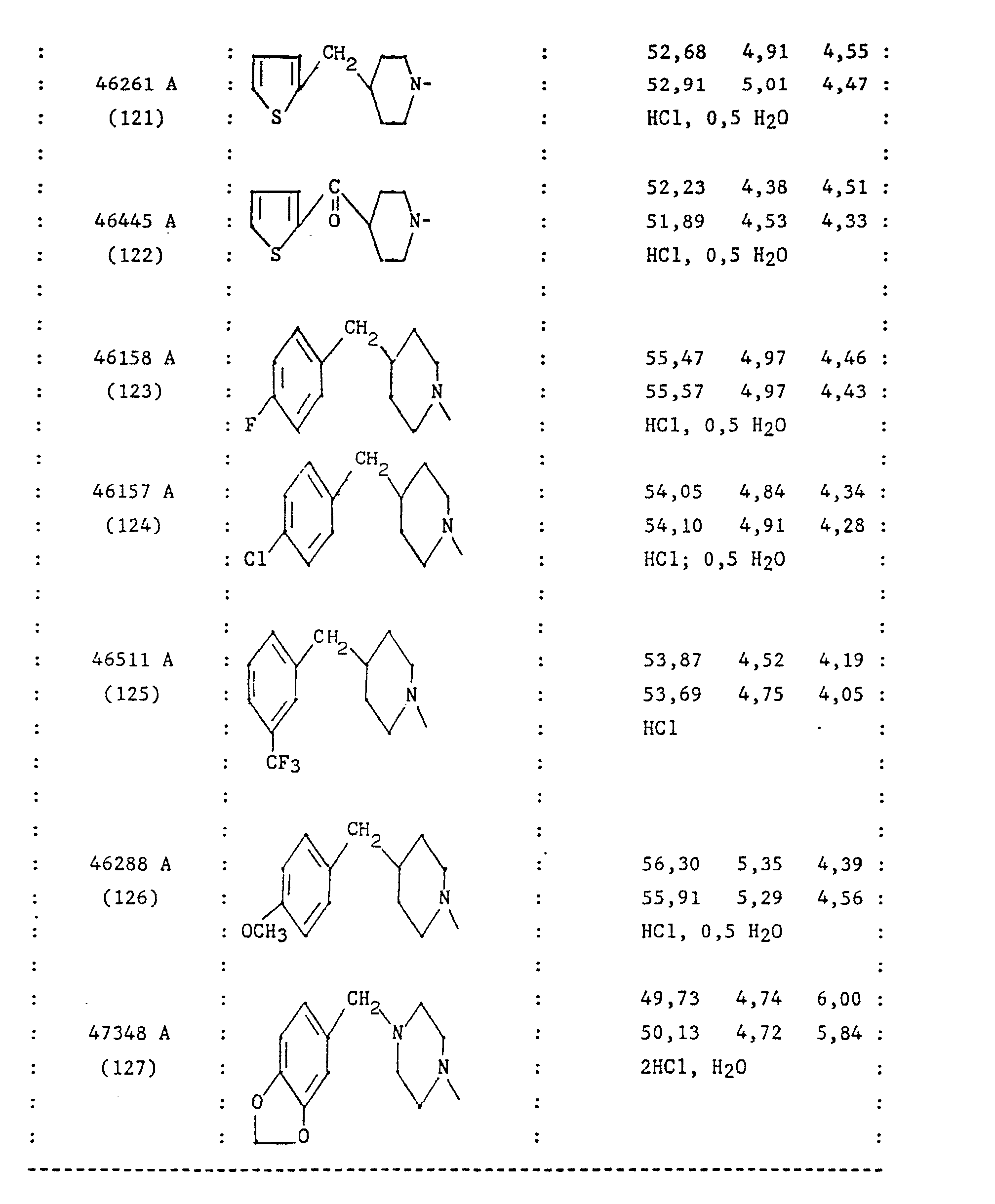

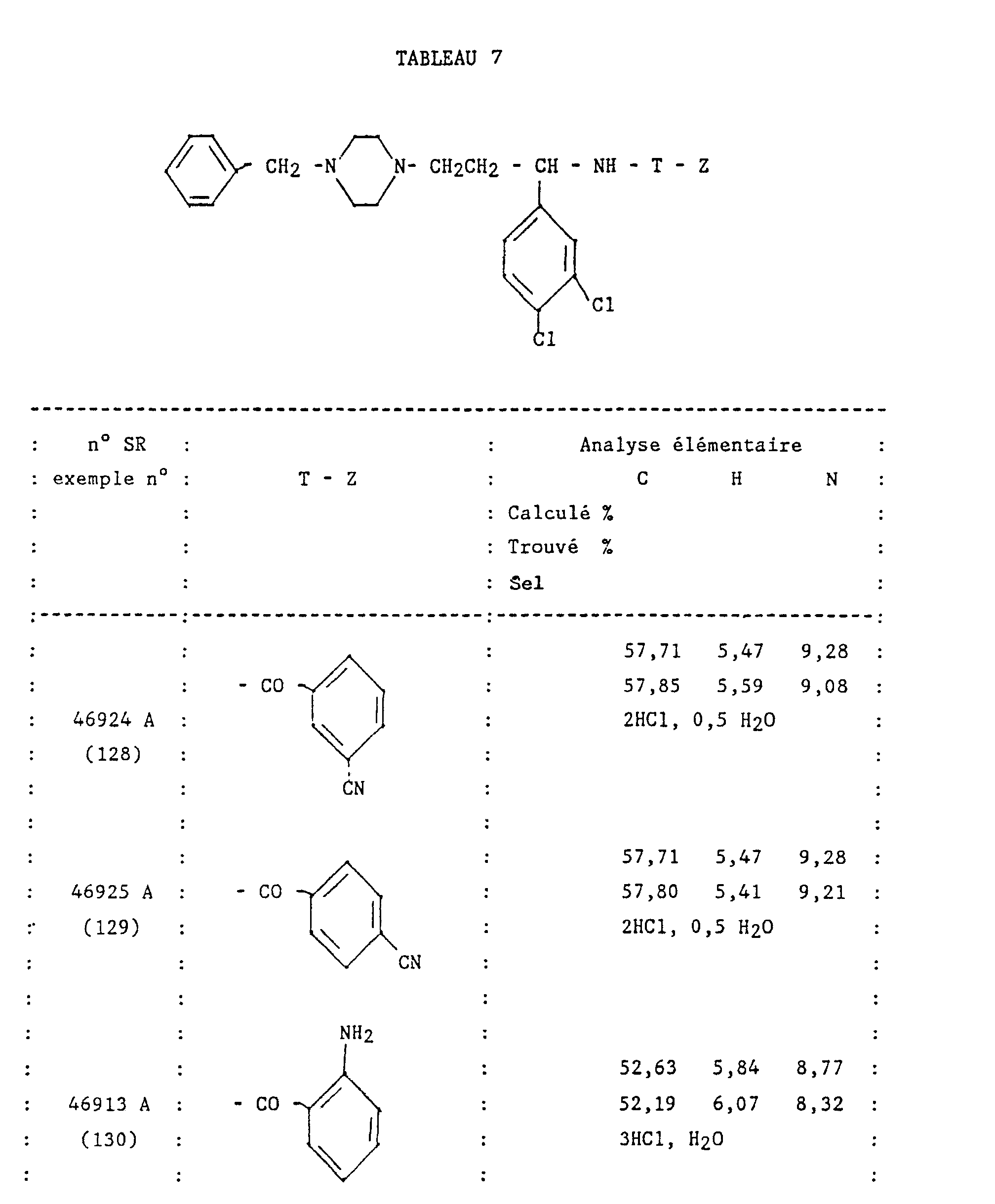

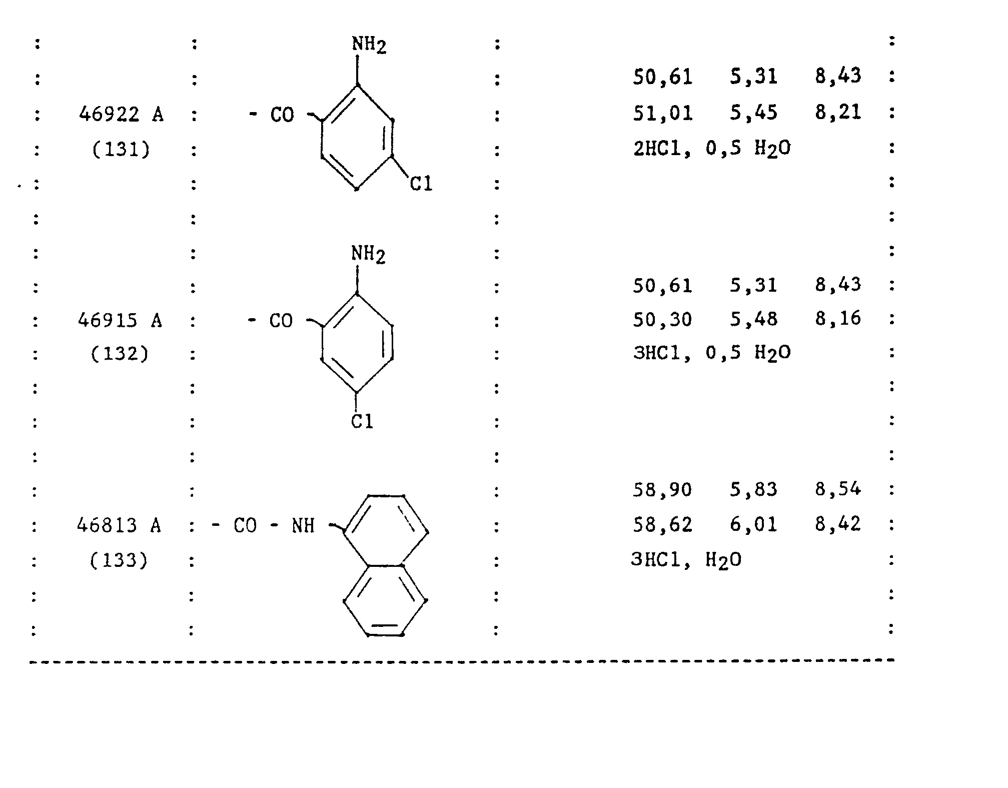

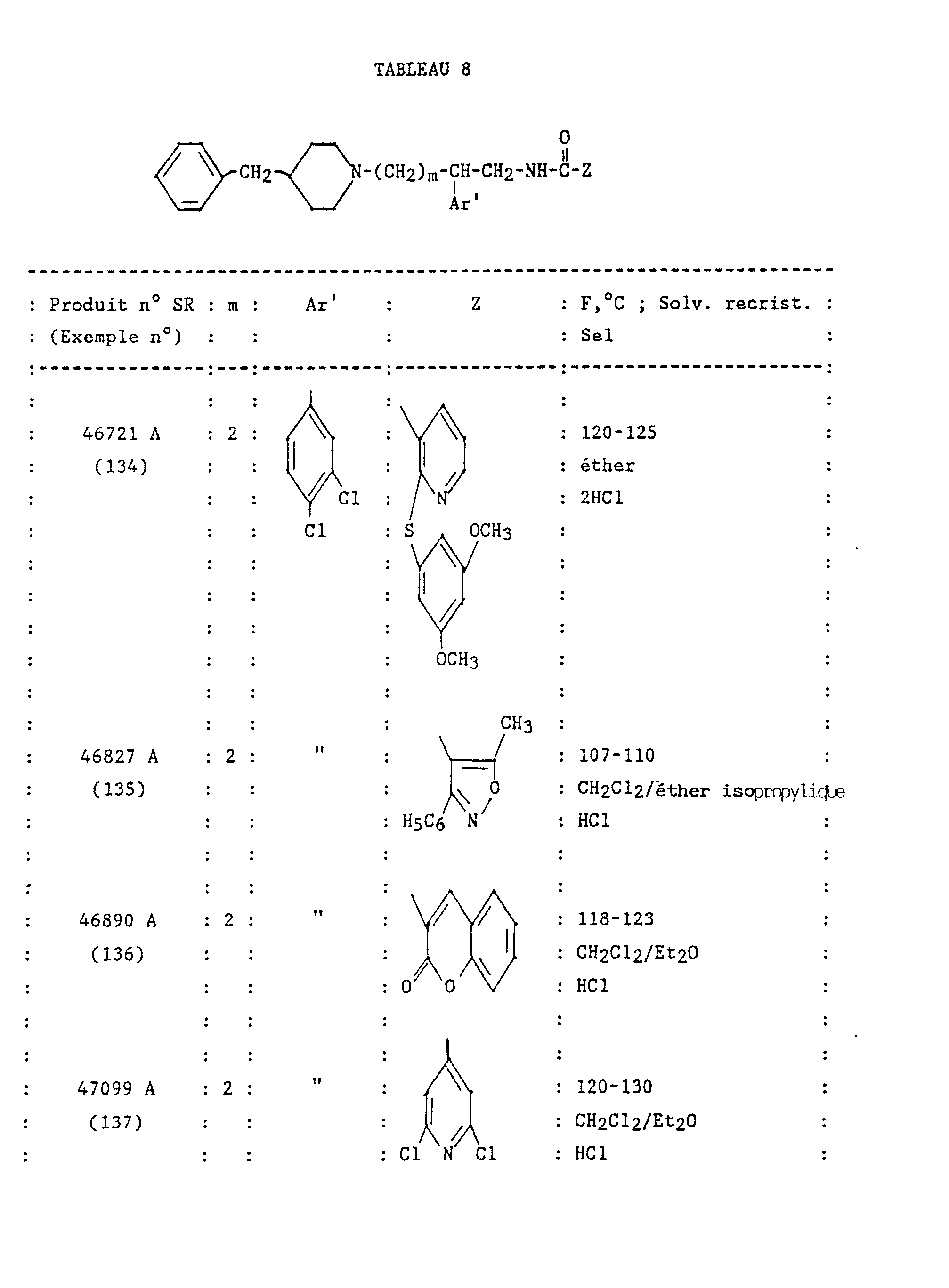

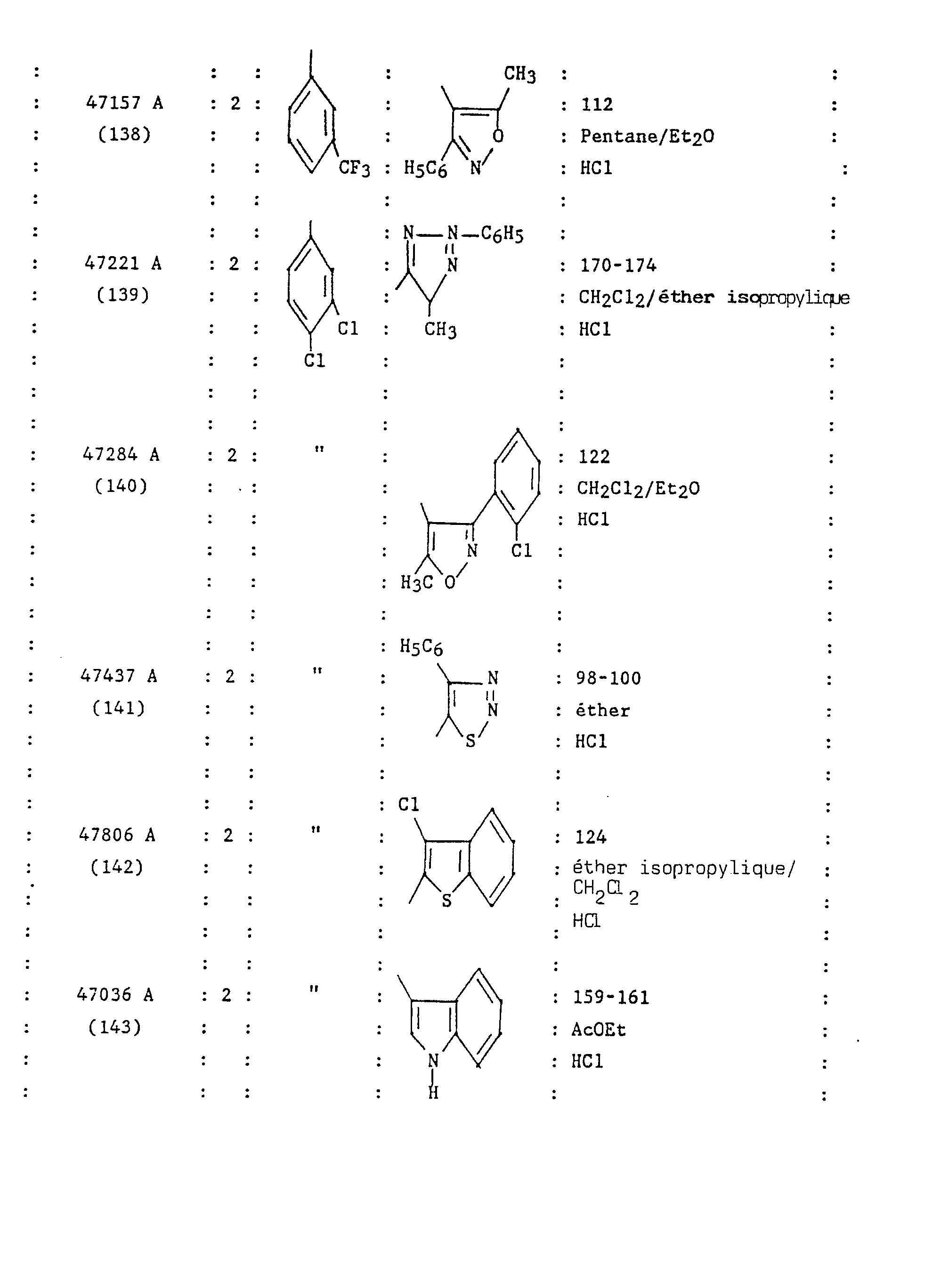

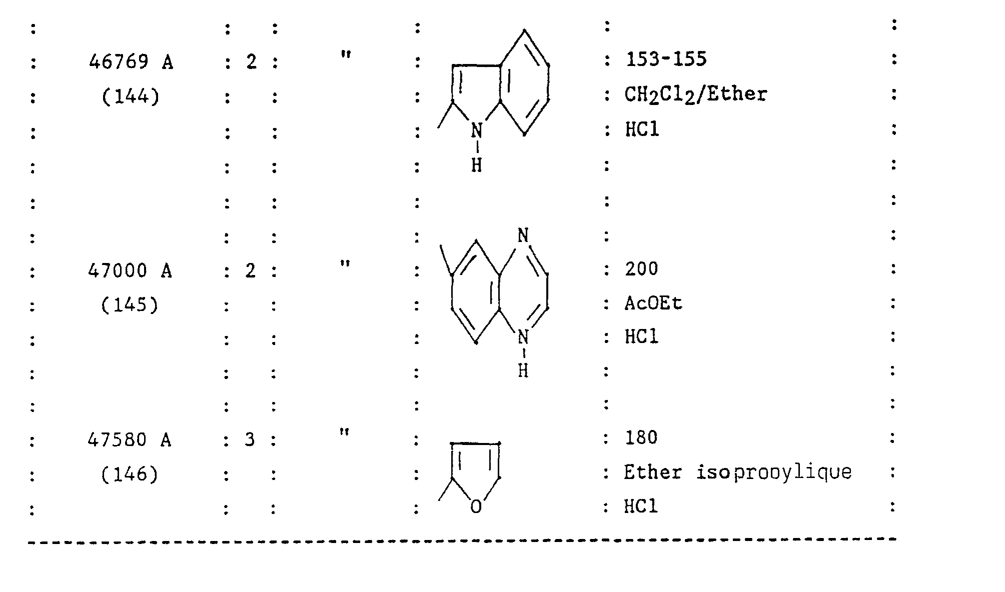

### EXEMPLE 147

Chlorhydrate de N[(benzyl-4 pipéridinyl-1)-4 (dichloro-3,4 phényl)-2 butyl]-dichloro-2,4 benzamide (+). SR 47050 A. 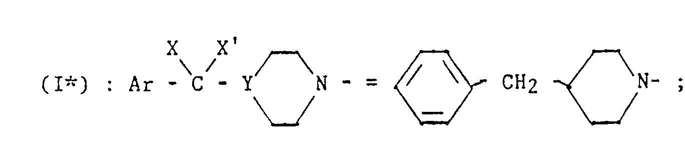 m = 2 ; Q ₌ H ;

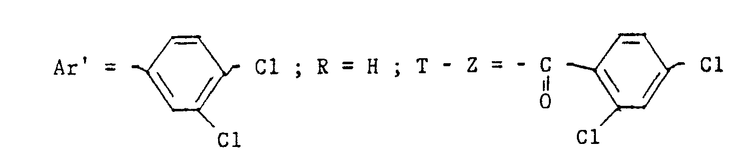

Les pouvoirs rotatoires des composés ci-dessous ont été mesurés à 25°C.

### A) N-(phényl-1 éthyl)β(tert-butoxy carbonylaminométhyl) -dichloro-3,4 benzènepropanamide.

### Etape 1

Dans un tricol de 2 litres balayé à l'azote, on introduit 39,6 ml de diisopropylamine en solution dans 200 ml de THF anhydre. On refroidit à -60°C et additionne à cette température dans l'ordre :
- 176 ml d'une solution 1,6 M de butyllithium dans l'hexane,
- 50 g de dichloro-3,4 benzène acétonotrile dans 300 ml de THF, puis,
- 39,4 ml de bromoacétate de tert-butyle dans 100 ml de THF.

On laisse revenir la température à 0°C en 2 heures 30. Le mélange est versé sur 3 1 de solution aqueuse saturée de chlorure d'ammonium. On extrait 2 fois à l'éther, sèche sur sulfate de magnésium, évapore les solvants. L'huile obtenue est chromatographiée sur 1 kg de silice H. Elution avec un mélange cyclohexane/acétate d'éthyle : 95/5. On obtient ainsi 44,3 g de β-cyanodichloro-3,4 benzènepropanoate de tert-butyle. F = 67 C.

### Etape 2

Un mélange de 40 g du produit obtenu précédemment (étape 1), 700 ml d'éthanol absolu, 200 ml d'ammoniaque concentré (20 %) et 3 spatules de nickel Raney est agité sous atmosphère d'hydrogène pendant 5 heures. Après filtration du catalyseur et évaporation des solvants on obtient 38,8 g de β-aminométhyldichloro-3,4 benzènepropanoate de tert-butyle sous forme d'huile.

### Etape 3

Une solution de 23,5 g du produit obtenu précédemment (étape 2) dans 150 ml de dichlorométhane est refroidie à -10° C. On additionne 250 ml d'acide trifluoracétique puis on laisse remonter la température à 20°C en 1 h 30.

Après élimination des solvants, on obtient 27 g de trifluoroacétate de l'acide β-aminométhyldichloro-3,4 benzènepropanoïque sous forme d'huile.

### Etape 4

A 27 g du produit obtenu précédemment (étape 3) en solution dans 150 ml d'eau, on additionne 150 ml de dioxanne puis 30 ml de triéthylamine, puis une solution de 23 g de dicarbonate de di-tert-butyle dans 50 ml de dioxanne. On chauffe à 100° C pendant 1 heure. Le dioxanne est éliminé sous vide et la solution obtenue est lavée à l'éther isopropylique. La phase aqueuse est versée sur 1,5 1 de solution tampon phosphate pH = 2. Après extraction à l'éther et séchage sur sulfate de magnésium on évapore les solvants. L'huile obtenue est cristallisée dans l'éther isopropylique, ce qui donne 20,3 g de l'acide β-(tert- butoxycarbonylaminométhyl)-dichloro-3,4 benzènepropanoïque.

### Etape 5

A 10 g du produit obtenu précédemment (étape 4) en solution dans 150 ml de dichlorométhane on additionne dans l'ordre :
- 8 ml de triéthylamine,
- 3,5 g de S(-) a-méthylbenzylamine,
- 14 g de BOP (hexafluorophosphate de benzotriazolyl N-oxytrisdiméthylaminophosphonium).

Après agitation à température ambiante pendant 1 heure, on lave à l'eau, puis avec une solution tampon phosphate pH = 2, puis avec une solution aqueuse saturée en bicarbonate de sodium. On sèche sur sulfate de magnésium, élimine les solvants sous vide, pour obtenir 12 g de [ N-(phényl-1 éthyl)]β(tert- butoxycarbonylaminométhyl)-dichloro-3,4 benzènepropanamide.

### B) Ester méthylique de l'acide β-(dichloro-2,4 benzoylaminométhyl)-dichloro-3,4 benzènepropanoïque (+).

### Etape 1

Séparation des diastéréoisomères du [N-(phényl-1 éthyl) ] β-tert-butoxycarbonylaminométhyl-dichloro-3,4 benzènepropanamide.

Le produit brut est un mélange de deux diastéréoisomères. Ils sont séparables par chromatographie sur couche mince. On les sépare de façon préparative à l'aide d'une chromatographie sur 400 g de silice H en éluant avec un mélange toluène/acétate d'éthyle : 80/20. L'isomère le moins polaire sort en premier et on en recueille 5,8 g. F = 146-147° C [α] D = -43,6° (c = 1 dans le chloroforme).

### Etape 2

Une solution de 5 g du produit obtenu précédemment dans 10 ml de dioxanne et 50 ml d'acide chlorhydrique 6 N est chauffée à reflux durant la nuit. Après refroidissement de la solution, on la lave à l'éther puis on neutralise progressivement la phase aqueuse avec du bicarbonate de sodium solide jusqu'à pH = 7. On obtient alors un précipité que l'on filtre, lave à l'eau, à l'isopropanol puis à l'éther. Après séchage, on obtient 1,88 g de l'acide β-aminométhyl-dichloro-3,4 benzènepropanoïque. F = 202-204 °C.

### Etape 3

A une suspension de 1,85 g de produit obtenu précédemment (étape 2) dans 20 ml de méthanol refroidie à -20°C sous azote, on ajoute 1,10 ml de chlorure de thionyle puis on laisse remonter la température à 20°C. 2 heures après, on ajoute 200 ml d'éther, filtre et lave à l'éther le produit qui a cristallisé. Après séchage, on obtient 2,15 g de β-aminométhyl-dichloro-3,4 benzènepropanoate de méthyle (-). F = 184-186 °C [a] D = -4,3° (c = 1 dans le méthanol).

### Etape 4

A une solution de 2,0 g de produit obtenu précédemment (étape 3) et 1,5 g de triéthylamine dans 20 ml de dichlorométhane refroidie à 0°C, on ajoute une solution de chlorure de dichloro-2,4 benzoyle (1,54 g) dans 5 ml de dichlorométhane. 5 mn après, on concentre à sec la solution, ajoute de l'eau et extrait à l'acétate d'éthyle. Le résidu obtenu est ensuite cristallisé dans l'éther isopropylique. On obtient ainsi 2,72 g de β-(dichloro-2,4 benzoylaminoéthyl)-benzènepropanoate de méthyle (+). F = 105-107° C [a] D = + 26,6° (c = 1 dans le chloroforme).

### C) Ester méthylique de l'acide β-(dichloro-2,4 benzoylaminométhyl)-dichloro-3,4 benzènepropanoïque (-).

### Etape 1

En procédant comme il est décrit dans l'exemple 1 B) étape 1, on recueille l'isomère le plus polaire en éluant avec un mélange toluène/acétate d'éthyle : 80/20 puis 60/40. La concentration des fractions fournit 5,4 g de [N-(phényl-1 éthyl)] β-(tert-butoxycarbonyl-aminométhyl)-dichloro-3,4 benzènepropanamide. F = 161-162° C [a] D = -18,4° (c = 1 dans le chloroforme).

### Etape 2

En procédant comme il est décrit dans l'exemple 1 B) étape 2, on prépare l'acide β-aminométhyl- dichloro-3,4 benzènepropanoïque. F = 202-204 °C

### Etape 3

En procédant comme il est décrit dans l'exemple 1 B) étape 3, on prépare le β-aminométhyl-dichloro-3,4 benzènepropanoate de méthyle (+). F = 184-185° C [α] D = + 3,9° (c = 1 dans le méthanol).

### Etape 4

En procédant comme il est décrit dans l'exemple 1 B) étape 4, on prépare le β-(dichloro-2,4 benzoylaminométhyl)-dichloro-3,4 benzènepropanoate de méthyle (-). F = 108-109°C [a] D = -27,7° (c = 1 dans le chloroforme).

### D) Réduction des esters méthyliques de l'acide β-(dichloro-2,4 benzoylaminométhyl)- dichloro-3,4 benzènepropanoïque (+) ou (-).

On prépare tout d'abord une solution 0,5 M de borohydrure de calcium dans le THF en laissant agiter 3 heures une suspension de borohydrure de sodium (0,1 mole) et de chlorure de calcium (0,05 mole) dans 100 ml de THF. On ajoute ensuite 13 ml de cette solution à une solution de 2,5 g d'ester méthylique de l'acide β-(dichloro-2,4 benzoylaminométhyl)- dichloro3,4 benzènepropanoïque (+) ou (-) dans 20 ml de THF. On laisse agiter durant la nuit. Le lendemain, la solution est refroidie à 0° C puis hydrolysée avec de l'eau puis de l'acide chlorhydrique dilué. Après extraction à l'éther, on recueille l'alcool (+) ou (-) pratiquement pur sous forme d'huile.

### E) Préparation des dérivés mésylates : méthane sulfonates du (dichloro-2,4 benzoylaminométhyl)-γ-dichloro-3,4 benzènepropanol (+) ou (-).

1,3 g de l'alcool obtenu précédemment sont mis en solution dans 30 ml de dichlorométhane puis on ajoute à la solution refroidie à 0°C, 0,5 ml de triéthylamine et 0,3 ml de chlorure de mésyle. Le mélange réactionnel est agité à 0°C pendant 45 minutes, lavé 3 fois à l'eau glacée, décanté, séché sur MgSO₄ et concentré sous vide.

Le résidu est chromatographié sur gel de silice, éluant : acétate d'éthyle/pentane 60/40. Les fractions pures sont concentrées sous vide.

Ainsi on obtient à partir de l'ester (+) un résidu qui est recristallisé de l'éther isopropylique pour fournir 1,1 g de méthanesulfonate du (dichloro-2,4 benzoylaminométhyl)-γ-dichloro-3,4 benzènepropanol (+). F = 74-77°C [α] D = + 21,2° (c = 1 dans le chloroforme).

Ainsi on obtient à partir de l'ester (-) en procédant comme ci-dessus, le méthanesulfonate du (dichloro-2,4 benzoylaminométhyl)-y-dichloro-3,4 benzènepropanol (-). F = 72-76 °C [α] D = -22,5° (c = 1 dans le chloroforme).

### F) Préparation du chlorhydrate de N[(benzyl-4 pipéridinyl-1)-4 (dichloro-3,4 phényl)-2 butyl]-dichloro-2,4 benzamide (+). SR 47050 A.

0,6 g du mésylate (+) obtenu précédemment et 0,54 g de benzyl-4 pipéridine sont mis en solution dans 1 ml de diméthylformamide et le mélange réactionnel est chauffée à 60° C pendant 30 minutes. On ajoute de l'eau et extrait à l'acétate d'éthyle. La phase organique est concentrée sous vide et le résidu est chromatographié sur gel de silice. Eluant : dichlorométhane/méthanol : 97/3.

Les fractions de produit pur sont concentrées sous vide, le résidu est dilué dans le dichlorométhane et l'addition d'éther chlorhydrique permet l'obtention du chlorhydrate. m = 0,5 g [a] D = + 14,0° (c = 1 dans le chloroforme).

### EXEMPLE 148

Chlorhydrate de N[(benzyl-4 pipéridinyl-1)-4 (dichloro-3,4 phényl)-2 butyl]-dichloro-2,4 benzamide (-). SR 47051 A. 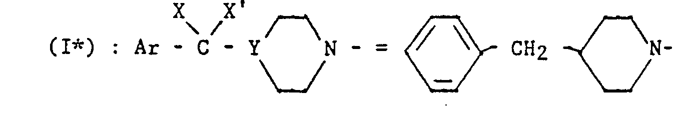 ; m = 2 ; Q ₌ H ;

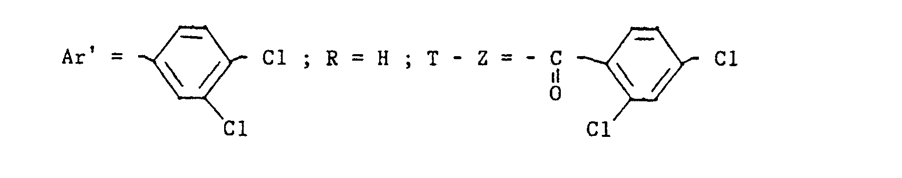

En procédant de la même façon que précédemment (selon exemple 147 F)) mais en utilisant comme produit de départ l'isomère (-) mésylate, on obtient le SR 47051 A. [a] D = -14,5°(c = 1 dans le chloroforme).

### EXEMPLE 149

En procédant comme il est décrit dans l'exemple 147 ci-dessus, on prépare : - le chlorhydrate de N[(benzyl-4 pipéridinyl-1)-4 (difluoro-3,4 phényl)-2 butyl]-dichloro-2,4 benzamide (-). SR 47243 A. 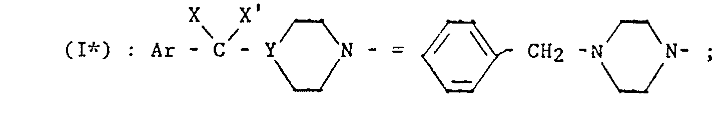 m = 2 ; Q = H ; 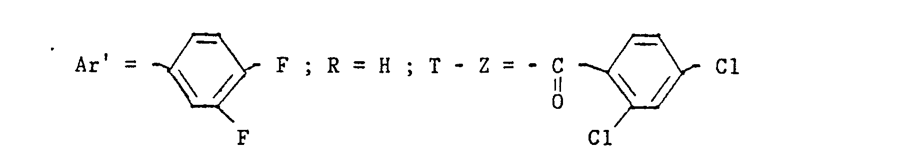 [a] D = -8,5°(c = 1 dans le chloroforme).

### EXEMPLE 150

En procédant comme il est décrit dans l'exemple 147 ci-dessus, on prépare :
- le chlorhydrate de N[(benzyl-4 pipéridinyl-1)-4 (difluoro-3,4 phényl)-2 butyl]-dichloro-2,4 benzamide (+). SR 47238 A. 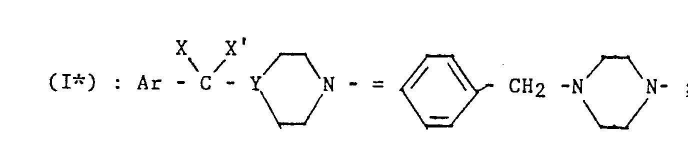 ; m ₌ 2 ; Q ₌ H ; 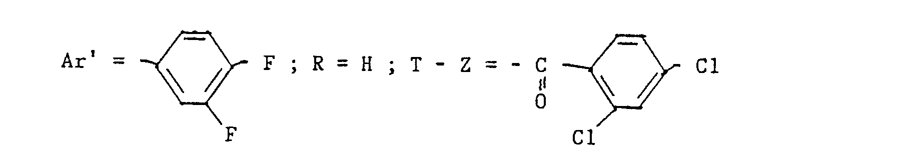 [α] D = +7,3°(c = 1 dans le chloroforme).

### EXEMPLE 151

Chlorhydrate de N [(benzyl-4 pipéridinyl-l)-4 (dichloro-3,4 phényl)-2 butyl] fluoro-4 naphtalène-1 carboxamide (+) et (-). 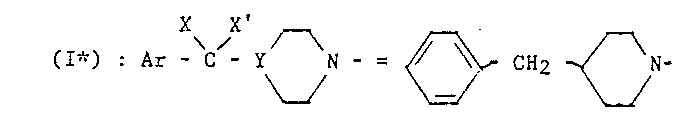 ; m ₌ 2 ; Q ₌ H ;

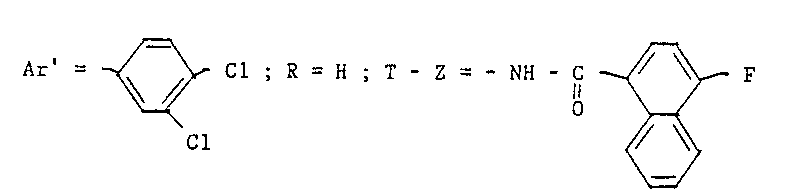

### Etape 1

### amino-1 (dichloro-3,4 phényl)-2 hydroxy-4 butane.

150 ml d'une solution saturée d'éther chlorhydrique sont ajoutés à 149 g de (tétrahydropyranyl-2 oxy)-4 (dichloro-3,4 phényl)-2 amino-1 butane en solution dans 700 ml de méthanol. Le mélange est agité pendant 1/2 heure à température ambiante, concentré sous vide et le résidu est repris dans 500 ml d'eau et lavé à l'éther. La phase aqueuse est alcalinisée avec une solution d'hydroxyde de sodium et extraite deux fois au dichlorométhane. Les phases organiques sont séchées sur MgSO₄, filtrées et concentrées sous vide. Le résidu est repris dans 400 ml d'éther isopropylique et le mélange est agité pendant une heure à température ambiante. Le précipité est filtré et lavé à l'éther. m = 98,2 g F = 90-91 °C

### Etape 2

### - Enantiomère (+) de l'amino-1 (dichloro-3,4 phényl)-2 hydroxy-4 butane.

A 59,65 g d'acide D (-) tartrique en solution dans 2 litres de méthanol chauffé au reflux, on ajoute 93 g du racémique, précédemment préparé selon l'étape 1, en solution dans 300 ml de méthanol. On laisse revenir à température ambiante, filtre les cristaux, lave au méthanol et sèche sous vide à 50° C sur P₂O₅. m = 64,8 g [a] D = -5,2° (c = 1 dans l'eau).

On recristallise ensuite dans 2,96 1 de méthanol, filtre les cristaux les lave au méthanol et sèche sous vide à 50°C sur P₂0₅ m = 45,3 g [a] D = -4,5° (c = 1 dans l'eau) F = 201° C.

Le D (-) tartrate est repris dans 250 ml d'eau, on alcalinise avec une solution concentrée d'hydroxyde de sodium et extrait avec 3 fois 200 ml de dichlorométhane, lave avec une solution saturée de chlorure de sodium, décante, sèche sur MgS0₄, filtre et concentre sous vide. Le résidu est repris dans l'éther isopropylique, le mélange est agité pendant une heure à température ambiante, les cristaux sont filtrés et lavés à l'éther isopropylique. m = 24,7 g [α] D = +9,0° (c = 1 dans le méthanol) F = 79-80°C.

### Enantiomère (-) de l'amino-1 (dichloro-3,4 phényl)-2 hydroxy-4 butane.

En procédant comme précédemment et en utilisant l'acide L (+) tartrique on obtient l'énantiomère (-). [a] D = -9,2° (c = 1 dans le méthanol) F = 79-80°C.

### Etape 3

### N [(dichloro-3,4 phényl)-2 mésyloxy-4 butyl] fluoro-4 naphtalène-1 carboxamide (énantiomère (-)).

4,45 g de chlorure de l'acide fluoro-4 naphtoïque en solution dans 50 ml de dichlorométhane sont ajoutés goutte à goutte à -60°C à une solution de 5 g du produit obtenu précédemment (énantiomère (+)) dans 100 ml de dichlorométhane en présence de 2,6 g de triéthylamine. On agite le mélange pendant 15 minutes à -60°C et laisse remonter la température à -30°C. On ajoute alors 2,5 g de triéthylamine et 2,7 g de chlorure de mésyle et laisse revenir à température ambiante. On lave à l'eau sèche la phase organique sur MgSO₄, et concentre sous vide. Le résidu est chromatographié sur gel de silice, éluant : dichlorométhane/méthanol 99,5/0,5. Les fractions pures sont concentrées sous vide. m = 8,4 g [a] D = -22,8° (c = 1 dans le méthanol).

### N- [(dichloro-3,4 phényl)-2 mésyloxy-4 butyl] fluoro-4 naphtalène-1 carboxamide (énantiomère (+)).

L'énantiomère (+) est obtenu en procédant comme décrit précédemment à l'étape 3 mais en utilisant l'énantiomère (-) de l'étape 2. [α] D = +22,7° (c = 1 dans le méthanol).

### Etape 4

Chlorhydrate de N- [(benzyl-4 pipéridinyl-1 )-4 (dichloro-3,4 phényl)-2 butyl] fluoro-4 naphtalène-1 carboxamide. Enantiomère (-) SR 48225 A.

7 g de l'énantiomère (-) obtenu à l'étape 3 et 5,02 g de benzyl-4 pipéridine sont mis en solution dans 15 ml de diméthylformamide et le mélange réactionnel est chauffé à 70°C pendant deux heures. Le mélange est versé sur de l'eau, extrait à l'acétate d'éthyle, les phases organiques sont lavées avec une solution saturée de chlorure de sodium, séchées sur MgS04 et concentrées sous vide. Le résidu est chromatographié sur gel de silice, éluant : dichlorométhane/méthanol 97/3.

Le fractions de produit pur sont concentrées sous vide, le résidu est dilué dans le dichlorométhane et l'addition d'éther chlorhydrique permet l'obtention du chlorhydrate. m = 6,2 g [a] D = -35,5° (c = 1 dans le méthanol).

### Enantiomère (+). SR 48226 A

En procédant de la même façon que pour l'énantiomère (-) précédemment préparé mais en utilisant l'énantiomère (+) obtenu à l'étape 3, on obtient l'énantiomère (+). m = 7g g [α]D = +36,0° (c = 1 dans le méthanol).

### EXEMPLE 152 :

Chlorhydrate de N-méthyl-N [(benzyl-4 pipéridinyl-1 )-4 (dichloro-3,4 phényl)-2 butyl] phénylacétamide. SR 48172 A.

A partir du chlorhydrate de (benzyl-4 pipéridinyl-1)-4 (dichloro-3,4 phényl)-2 N-méthylamino-1 butane décrit dans l'étape a) de l'Exemple 98, par réaction avec le chlorure de l'acide phénylacétique selon le mode opératoire décrit dans l'Exemple 1, on obtient le composé SR 48172 A.

## Revendications

1. Un composé de formule 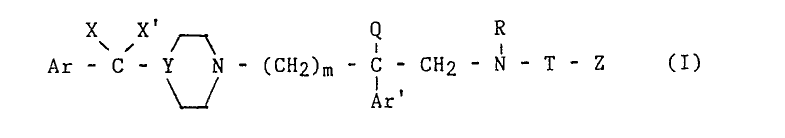 dans laquelle :
- m est un nombre entier de 1 à 3;
- Ar et Ar représentent, indépendamment, un groupe thiényle; un groupe phényle non substitué, mono ou di-substitué par un atome d'halogène, par un alkyle en C₁-C₃, par un trifluorométhyle, par un alcoxy dans lequel l'alkyle est en C₁-C₃, par un hydroxyle, par un méthylènedioxy ; un groupe imidazolyle ; Ar pouvant également être un groupe benzothiényle non substitué ou substitué par un halogène; un groupe naphtyle non substitué ou substitué par un halogène ; un groupe biphényle; un indolyle non substitué ou substitué sur l'azote par un groupe benzyle ;
- X représente l'hydrogène;
- X représente l'hydrogène, un groupe hydroxyle, ou est réuni à X ci-dessous pour former une liaison carbone-carbone ;
. ou bien X et X', ensemble, forment un groupe oxo ou dialkylaminoalkyloxyimino de formule =N-0-(CH2)p-Am, où p est 2 ou 3 et Am est un groupe dialkylamino, chaque alkyle pouvant contenir de 1 à 4 atomes de carbone;
- Y représente un atome d'azote ou un groupe C(X") où X est l'hydrogène ou, avec X', forme une liaison carbone-carbone ;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino, chaque alkyle pouvant contenir de 1 à 4 atomes de carbone;
- R représente l'hydrogène, un groupe méthyle ou un groupe (CH₂)ₙ-L, où n est un nombre entier de 2 à 6 et L est l'hydrogène ou un groupe amino,
- T représente un groupe choisi parmi 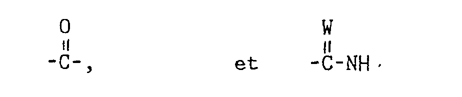 W étant un atome d'oxygène ou de soufre, et
- Z représente soit l'hydrogène, soit M ou OM lorsque T représente le groupe 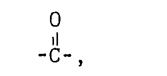 soit M lorsque T représente le groupe 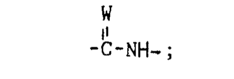
M représente l'hydrogène ou un alkyle droit ou ramifié en C₁-C₆ ; un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1) imidazolyl-2 thioalkyle dans lequel le groupe alkyle comprend de 1 à 3 atomes de carbone ; un oxo-1 phénylindan-3 yle-2 ; un groupe aromatique ou hétéroaromatique non substitué, mono ou polysubstitué;
ou un de ses sels avec des acides minéraux ou organiques.

2. Un composé optiquement pur de formule : 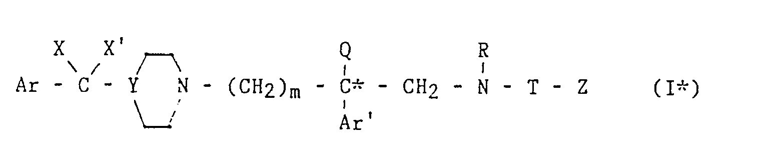 dans laquelle :
- "*" signifie que l'atome de carbone ainsi marqué a une configuration absolue (+) ou (-) déterminée
- m, Ar et Ar', X, X', Y, Q, R, T et Z sont tels que définis dans la revendication 1. ou un de ses sels avec des acides minéraux ou organiques.

3. N [(benzyl-4 pipéridinyl-1)-4 (dichloro-3,4 phényl)-2 butyl]-dichloro-2,4 benzamide ou un de ses sels pharmaceutiquement acceptables.

4. N [(benzyl-4 pipéridinyl-1)-4 (difluoro-3,4 phényl)-2 butyl]-dichloro-2,4 benzamide ou un de ses sels pharmaceutiquement acceptables.

5. N [(benzyl-4 pipéridinyl-1)-4 (dichloro-3,4 phényl)-2 butyl] fluoro-4 naphtalène-1 carboxamide ou un de ses sels pharmaceutiquement acceptables.

6. N [(benzyl-4 pipéridinyl-l)-4 (dichloro-3,4 phényl)-2 butyl]-dichloro-2,4 benzamide (+) ou un de ses sels pharmaceutiquement acceptables.

7. N [(benzyl-4 pipéridinyl-1 )-4 (dichloro-3,4 phényl)-2 butyl]-dichloro-2,4 benzamide (-) ou un de ses sels pharmaceutiquement acceptables.

8. N [(benzyl-4 pipéridinyl-1 )-4 (difluoro-3,4 phényl)-2 butyl]-dichloro-2,4 benzamide (+) ou un de ses sels pharmaceutiquement acceptables.

9. N [(benzyl-4 pipéridinyl-1)-4 (difluoro-3,4 phényl)-2 butyl]-dichloro-2,4 benzamide (-) ou un de ses sels pharmaceutiquement acceptables.

10. N [(benzyl-4 pipéridinyl-1)-4 (dichloro-3,4 phényl)-2 butyl] fluoro-4 naphtalène-1 carboxamide (+) ou un de ses sels pharmaceutiquement acceptables.

11. N [(benzyl-4 pipéridinyl-1 )-4 (dichloro-3,4 phényl)-2 butyl] fluoro-4 naphtalène-1 carboxamide (-) ou un de ses sels pharmaceutiquement acceptables.

12. Procédé pour la préparation de composés selon la revendication 1, caractérisé en ce que

a) on traite une amine libre de formule : 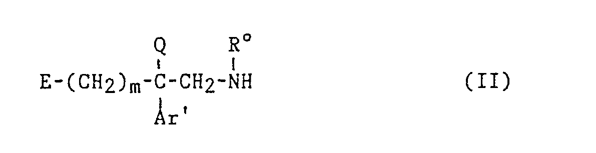 dans laquelle m, Ar' et Q sont tels que définis précédemment dans la revendication 1 ; R° représente l'hydrogène, un groupe méthyle ou un groupe (CH₂)ₙ-L°, où n est tel que défini dans la revendication 1, et L° est l'hydrogène ou un groupe amino protégé par un groupe N-protecteur ; et E représente un groupe hydroxy ou un groupe O-protégé tel que tétrahydropyran-2-yloxy, ou un groupe 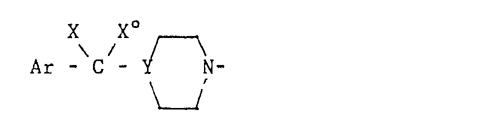 où Ar, X et Y sont tels que définis dans la revendication 1 et X° représente le groupe X , tel que défini dans la revendication 1, dans lequel le groupe hydroxy est protégé par un groupe O-protecteur ; - soit avec un dérivé fonctionnel d'un acide de formule : 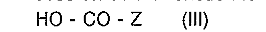 dans laquelle Z est tel que défini dans la revendication 1, lorsqu'on doit préparer un composé de formule (I) où T est -CO-, - soit avec un iso(thio)cyanate de formule : 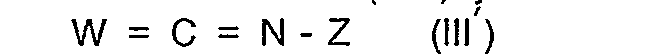 dans laquelle W et Z sont tels que définis dans la revendication 1, lorsqu'on doit préparer un composé de formule (I) où T est -C(W)-NH-, pour former le composé de formule : 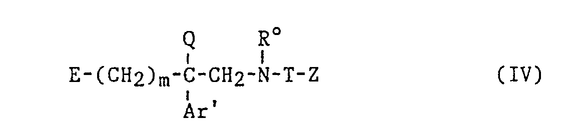
b) puis, lorsque E représente le tétrahydropyranyloxy, on élimine le groupe tétrahydropyranyle par hydrolyse acide, l'hydrolyse pouvant alternativement avoir lieu à l'étape a) sur l'amine de départ de formule (II),
c) on traite l'alcanolamine N-substituée ainsi obtenue de formule: 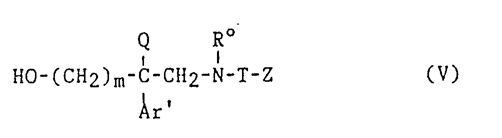 avec le chlorure de méthanesulfonyle
d) on fait réagir le mésylate ainsi obtenu de formule : 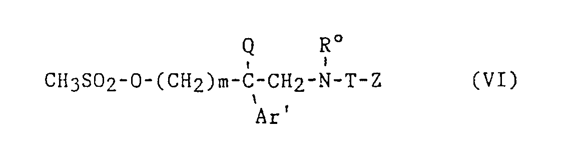 avec une amine secondaire de formule : 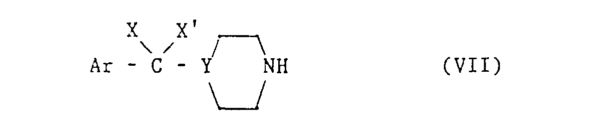 dans laquelle Ar, Y, X et X sont tels que définis dans la revendication 1,
e) on élimine les groupes O-protecteur et N-protecteur éventuels et on transforme éventuellement le produit ainsi obtenu en un de ses sels.

13. Procédé stéréosélectif pour la préparation de composés optiquement purs de formule (1^{*}), selon la revendication 2, caractérisé en ce que on traite un composé de formule : 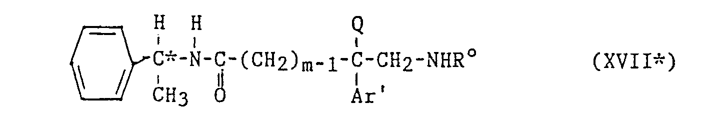 dans un solvant, en milieu acide, pour fournir l'aminoacide de formule: 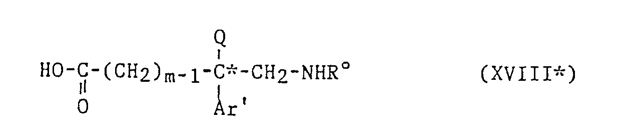 qui est estérifié dans un alcanol AIkOH, où Alk est un alkyle de 1 à 4 atomes de carbone, en milieu acide, puis on traite l'ester correspondant de formule : 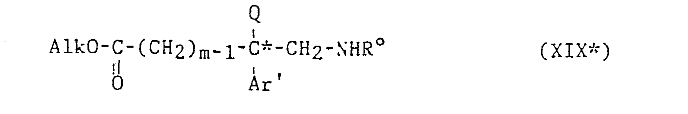 dans laquelle Alk est tel que défini ci-dessus; Q, Ar et m sont tels que définis dans la revendication 1 et R est tel que défini dans la revendication 12,
- soit avec un dérivé fonctionnel d'un acide de formule : 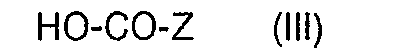
- soit avec un iso(thio)cyanate de formule : 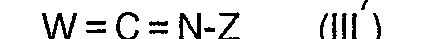 Z et W étant tel que définis ci-dessus, dans la revendication 2, on soumet l'ester ainsi obtenu de formule : 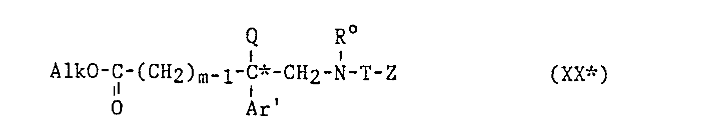 à l'action d'un agent réducteur et on transforme l'alcool correspondant de formule : 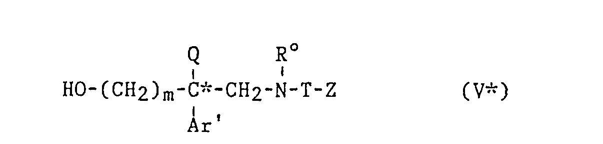 en son ester méthanesulfonate de formule: 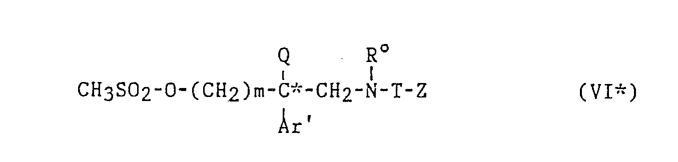 qui, par traitement avec une amine de formule : 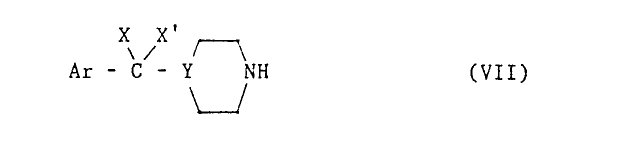 dans laquelle Ar,X,X et Y sont tels que définis dans la revendication 1 et déprotection éventuelle, donne le composé de formule (1^{*}).

14. Un composé de formule : 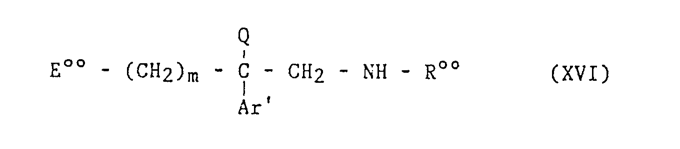 dans laquelle
- E° ° représente un groupe tétrahydropyranyloxy, un groupe hydroxy, ou un groupe 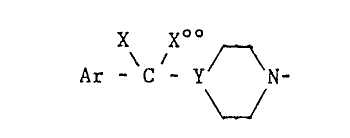
- X° ° représente l'hydrogène, un groupe hydroxyle libre ou protégé par un groupe 0-protecteur, ou est réuni à X" ci-dessous pour former une liaison carbone-carbone ;
. ou bien X et X° °, ensemble, forment un groupe oxo ou dialkylaminoalkyloxyimino de formule =N-0-(CH₂)p-Am, où p est 2 ou 3 et Am est un groupe dialkylamino, chaque alkyle pouvant contenir de 1 à 4 atomes de carbone ;
- Y représente un atome d'azote ou un groupe C(X ) où X est l'hydrogène, ou, avec X° °, forme une liaison carbone-carbone ;
- R° ° représente l'hydrogène, un groupe méthyle ou un groupe (CH₂)ₙ-L° °, où n est un nombre de 2 à 6 et L° ° est l'hydrogène ou un groupe amino libre ou protégé par un groupe N-protecteur ;
- m, Ar et Ar, X et Q sont tels que définis dans la revendication 1 ou un de ses sels.

15. Composé selon la revendication 14, de formule (XVI), dans laquelle m, Q, Ar sont tels que définis dans la revendication 1, E° ° est un groupe hydroxy, R° ° est R°, ce dernier ayant la signification définie dans la revendication 12, ledit composé étant sous forme optiquement pure.

16. Composé de formule : 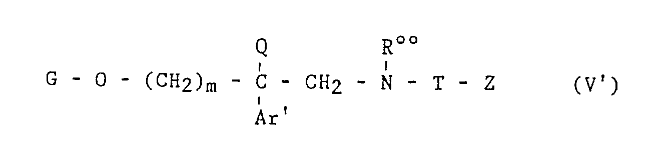 dans laquelle :
- G est l'hydrogène, un groupe tétrahydropyranyle ou un groupe méthanesulfonyle ;
- R ° ° représente l'hydrogène, un groupe méthyle ou un groupe (CH₂)ₙ-L° °, où n est un nombre de 2 à 6 et L° ° est l'hydrogène ou un groupe amino libre ou protégé par un groupe N-protecteur ;
- m,Ar', Q, T et Z sont tels que définis dans la revendication 1. ou un de ses sels.

17. Composé selon la revendication 16, de formule (V ) dans laquelle m, Q, T, Ar et Z sont tels que définis dans la revendication 1, G est l'hydrogène et R° ° est R° , ce dernier ayant la signification définie dans la revendication 12, ledit composé étant sous forme optiquement pure.

18. Composé selon la revendication 15 de formule (V') dans laquelle m, Q, T, Ar et Z sont tels que définis dans la revendication 1, G est un groupe méthanesulfonyle et R ° ° est R °, ce dernier ayant la signification définie dans la revendication 12, ledit composé étant sous forme optiquement pure.

19. Composition pharmaceutique contenant, en tant que principe actif, un composé de formule (I) ou (1^{*}) selon l'une des revendications 1 ou 2.

20. Composition pharmaceutique selon la revendication 19, sous forme d'unité de dosage, dans laquelle le principe actif est mélangé à au moins un excipient pharmaceutique.

21. Composition selon la revendication 20 contenant de 2,5 à 1000 mg de principe actif.

22. Composé selon la revendication 14, caractérisé en ce que E ° représente un groupe hydroxy et R ° représente l'hydrogène.

23. Chlorhydrate de N-méthyl-N[ (benzyl-4 pipéridinyl-1)-4 (dichloro-3,4 phényl)-2 butyl] phénylacétamide.

## Revendications (titre)

1. Procédé pour la préparation de composés de formule : 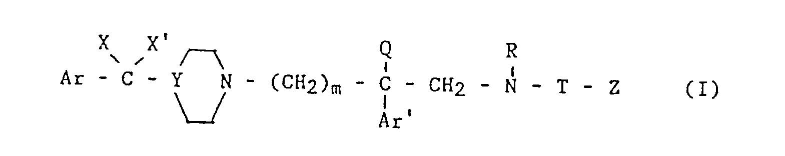 dans laquelle :
- m est un nombre entier de 1 à 3;
- Ar et Ar représentent, indépendamment, un groupe thiényle ; un groupe phényle non substitué, mono ou di-substitué par un atome d'halogène, par un alkyle en C₁-C₃, par un trifluorométhyle, par un alcoxy dans lequel l'alkyle est en C₁-C₃, par un hydroxyle, par un méthylènedioxy ; un groupe imidazolyle ; Ar pouvant également être un groupe benzothiényle non substitué ou substitué par un halogène ; un groupe naphtyle non substitué ou substitué par un halogène ; un groupe biphényle ; un indolyle non substitué ou substitué sur l'azote par un groupe benzyle ;
- X représente l'hydrogène;
- X représente l'hydrogène, un groupe hydroxyle, ou est réuni à X" ci-dessous pour former une liaison carbone-carbone ;
. ou bien X et X , ensemble, forment un groupe oxo ou dialkylaminoalkyloxyimino de formule =N-0-(CH2)p-Am, où p est 2 ou 3 et Am est un groupe dialkylamino, chaque alkyle pouvant contenir de 1 à 4 atomes de carbone;
- Y représente un atome d'azote ou un groupe C(X") où X" est l'hydrogène ou, avec X , forme une liaison carbone-carbone ;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino, chaque alkyle pouvant contenir de 1 à 4 atomes de carbone;
- R représente l'hydrogène, un groupe méthyle ou un groupe (CH₂)ₙ-L, où n est un nombre entier de 2 à 6 et L est l'hydrogène ou un groupe amino,
- T représente un groupe choisi parmi 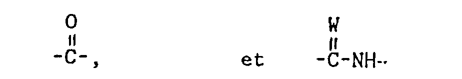 W étant un atome d'oxygène ou de soufre, et
- Z représente soit l'hydrogène, soit M ou OM lorsque T représente le groupe 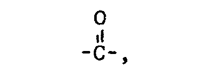 soit M lorsque T représente le groupe 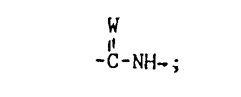
M représente l'hydrogène ou un alkyle droit ou ramifié en Ci-Ce ; un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1 ) imidazolyl-2 thioalkyle dans lequel le groupe alkyle comprend de 1 à 3 atomes de carbone; un oxo-1 phénylindan-3 yle-2 ; un groupe aromatique ou hétéroaromatique non substitué, mono ou polysubstitué;
ou un de ses sels avec des acides minéraux ou organiques,

caractérisé en ce que
a) on traite une amine libre de formule : 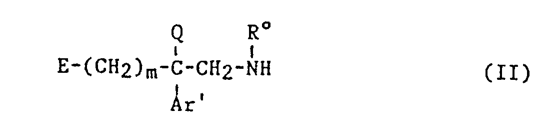 dans laquelle m, Ar' et Q sont tels que définis précédemment R représente l'hydrogène, un groupe méthyle ou un groupe (CH₂)ₙ-L°, où n est tel que défini précédemment, et L est l'hydrogène ou un groupe amino protégé par un groupe N-protecteur ; et E représente un groupe hydroxy, un groupe 0- protégé tel que tétrahydropyran-2-yloxy, ou un groupe 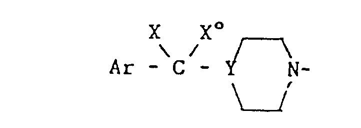 où Ar, X et Y sont tels que définis prédécemment et X° représente le groupe X', tel que défini précédemment, dans lequel le groupe hydroxy est protégé par un groupe 0-protecteur : - soit avec un dérivé fonctionnel d'un acide de formule ; 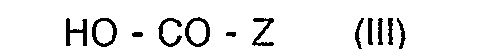 dans laquelle Z est tel que défini ci-dessus, lorsqu'on doit préparer un composé de formule (I) où T est -CO-, - soit avec un iso(thio)cyanate de formule : 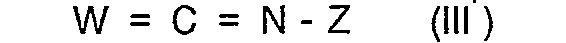 dans laquelle W et Z sont tels que définis ci-dessus, lorsqu'on doit préparer un composé de formule (I) où T est -C(W)-NH-, pour former le composé de formule : 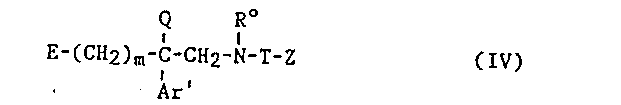
b) puis, lorsque E représente le tétrahydropyranyloxy, on élimine le groupe tétrahydropyranyle par hydrolyse acide douce, l'hydrolyse pouvant alternativement avoir lieu à l'étape a) sur l'amine de départ de formule (II),
c) on traite l'alcanolamine N-substituée ainsi obtenue de formule : 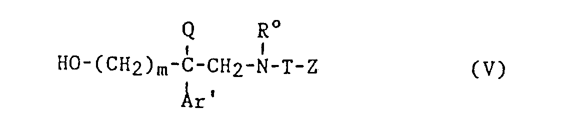 avec le chlorure de méthanesulfonyle
d) on fait réagir le mésylate ainsi obtenu de formule : 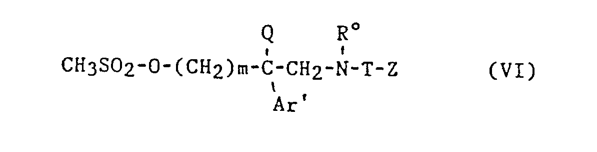 avec une amine secondaire de formule : 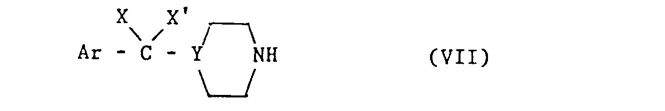 dans laquelle Ar,Y,X et X sont tels que définis précédemment,
e) on élimine les groupes 0-protecteur et N-protecteur éventuels et on transforme éventuellement le produit ainsi obtenu en un de ses sels.

2. Procédé pour la préparation de composés optiquement purs de formule : 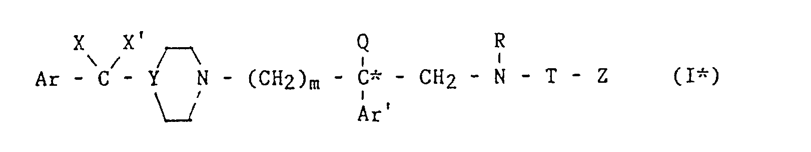 dans laquelle :
- "*" signifie que l'atome de carbone ainsi marqué a une configuration absolue (+) ou (-) déterminée ;
- m, Ar et Ar', X, X , Y, Q, R, T et Z sont tels que définis dans la revendication 1, ou d'un de ses sels avec des acides minéraux ou organiques, caractérisé en ce qu'on traite un composé de formule : 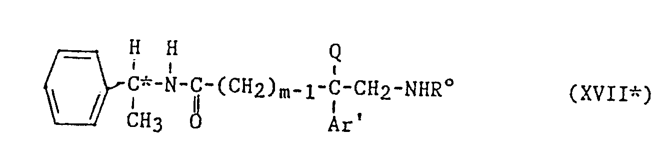 dans un solvant, en milieu acide, pour fournir l'aminoacide de formule: 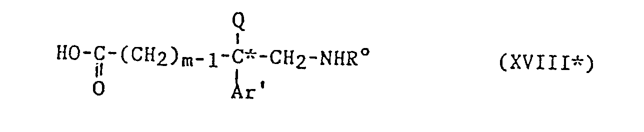 qui est estérifié dans un alcanol AlkOH, où Alk est un alkyle de 1 à 4 atomes de carbone, en milieu acide, puis on traite l'ester correspondant de formule : 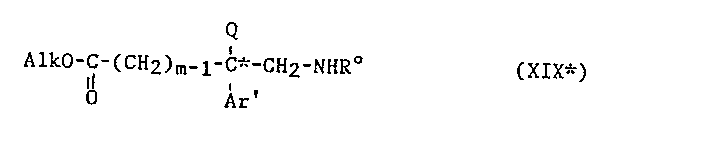 dans laquelle Alk est tel que défini ci-dessus et Q, Ar, R° et m sont tels que définis dans la revendication 1,
- soit avec un dérivé fonctionnel d'un acide de formule : 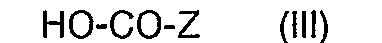
- soit avec un iso(thio)cyanate de formule : 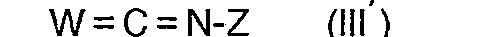 Z et W étant tels que définis dans la revendication 1, on soumet l'ester ainsi obtenu de formule : 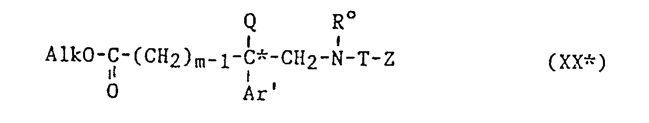 à l'action d'un agent réducteur et on transforme l'alcool correspondant de formule : 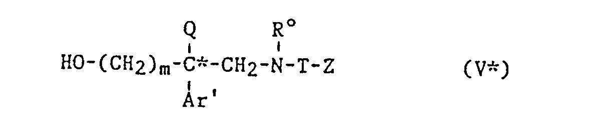 en son ester méthanesulfonate de formule: 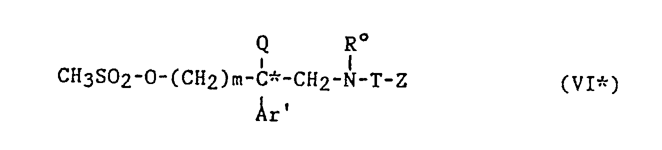 qui, par traitement avec une amine de formule : 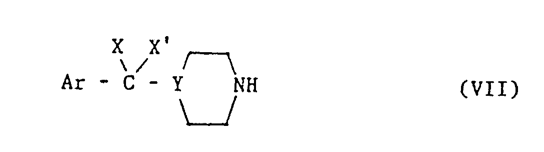 dans laquelle Ar,X,X et Y sont tels que définis dans la revendication 1 et déprotection éventuelle, donne le composé de formule (1^{*}), que l'on transforme éventuellement en un de ses sels.

3. Procédé pour la préparation de composés de formule : 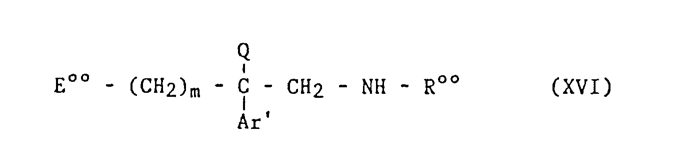 dans laquelle
- E° ° représente un groupe tétrahydropyranyloxy, un groupe hydroxy, ou un groupe 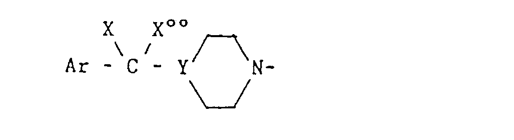
- X° ° représente l'hydrogène, un groupe hydroxyle libre ou protégé par un groupe O-protecteur, ou est réuni à X" ci-dessous pour former une liaison carbone-carbone ;
. ou bien X et X° °, ensemble, forment un groupe oxo ou dialkylaminoalkyloxyimino de formule = N-O-(CH₂)p-Am, où p est 2 ou 3 et Am est un groupe dialkylamino, chaque alkyle pouvant contenir de 1 à 4 atomes de carbone ;
- Y représente un atome d'azote ou un groupe C(X") où X est l'hydrogène, ou, avec X° °, forme une liaison carbone-carbone ;
- R° ° représente l'hydrogène, un groupe méthyle ou un groupe (CH₂)ₙ-L° °, où n est un nombre de 2 à 6 et L° ° est l'hydrogène ou un groupe amino libre ou protégé par un groupe N-protecteur ;
- m, Ar et Ar', X et Q sont tels que définis dans la revendication 1 ou de l'un de ses sels, caractérisé en ce que l'on soumet un nitrile de formule 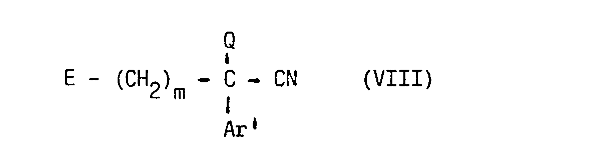 dans laquelle E est tel que défini dans la revendication 1, à une hydrogénation dans un alcanol en présence d'un catalyseur, pour obtenir l'amine libre primaire de formule : 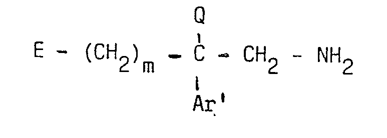 ladite amine libre primaire étant ensuite
- soit isolée si l'on doit préparer des composés de formule (XVI) dans laquelle R° ° est l'hydrogène, et les groupes O- et N-protecteurs étant ensuite éventuellement éliminés pour obtenir des composés de formule : 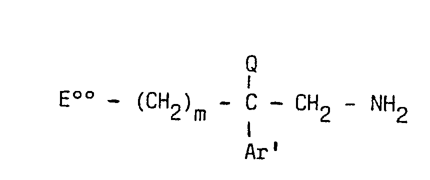
- soit, pour préparer des composés de formule (XVI) où R° ° est méthyle, traitée avec un chloroformiate, par exemple un chloroformiate de formule CI-CO-0-Alk où Alk est un alkyle en Ci-C₄, pour obtenir les carbamates de formule : 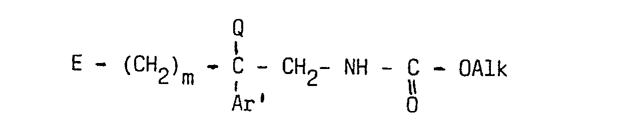 qui sont ensuite réduits par un agent réducteur tel qu'un hydrure métallique, ou un hydrure de bore, dans un solvant tel que l'éther ou le toluène à une température comprise entre la température ambiante et 60°C, pour obtenir une méthylamine de formule : 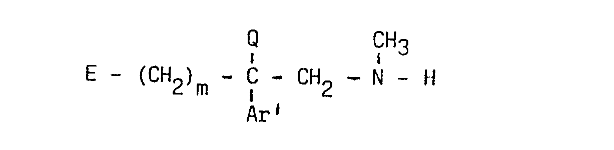 et les groupes 0- et N-protecteurs étant ensuite éventuellement éliminés pour obtenir un composé de formule : 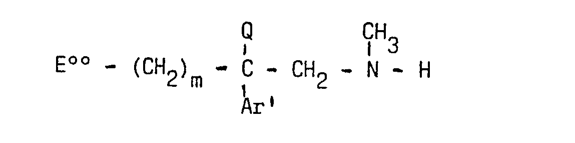
- soit pour préparer les composés de formule (XVI) où R° ° est un groupe -(CH₂)ₙ-L° ° , où n et L° ° sont tels que définis ci-dessus, traitée avec un dérivé fonctionnel réactif de l'acide de formule : 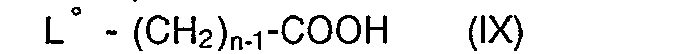 pour obtenir un amide de formule : 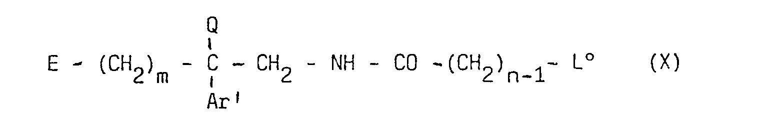 dans laquelle m, n, E, Ar', Q et L° sont tels que définis dans la revendication 1, l'amide (X) étant ensuite réduite pour donner le composé de formule : 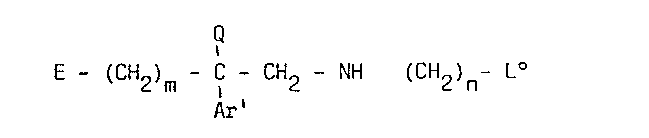 et les groupes O et N-protecteurs étant ensuite éventuellement éliminés pour obtenir un composé de formule : 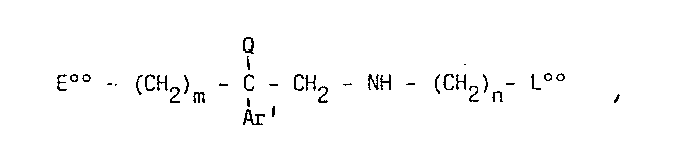 ces composés étant susceptibles de donner des sels avec des acides optiquement actifs afin d'obtenir des composés optiquement purs.

4. Procédé pour la préparation de composés de formule : 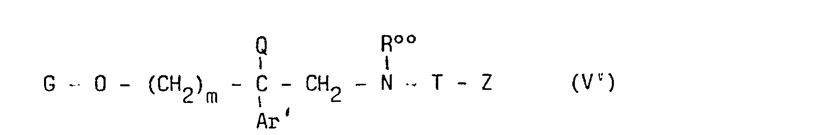 dans laquelle :
- G est l'hydrogène, un groupe tétrahydropyranyle ou un groupe méthanesulfonyle ;
- R représente l'hydrogène, un groupe méthyle ou un groupe (CH₂)ₙ-L° ° , où n est un nombre de 2 à 6 et L° ° est l'hydrogène ou un groupe amino libre ou protégé par un groupe N-protecteur ;
- m, Ar', Q, T et Z sont tels que définis dans la revendication 1, ou d'un de leurs sels, caractérisé en ce que :
a) on traite une amine libre de formule : 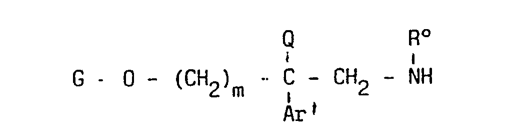 dans laquelle G, m, Ar et Q sont tels que définis ci-dessus, et R° représente l' hydrogène, un groupe méthyle ou un groupe (CH₂)ₙ-L°, où n est un nombre de 2 à 6 et L° est l'hydrogène ou un groupe amino protégé par un groupe N-protecteur, - soit avec un dérivé fonctionnel d'un acide de formule : 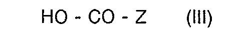 dans laquelle Z est tel que défini dans la revendication 1, lorsqu'on doit préparer un composé de formule (V') où T est -CO-, - soit avec un iso(thio)cyanate de formule : 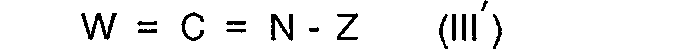 dans laquelle W et Z sont tels que définis dans la revendication 1, lorsqu'on doit préparer un composé de formule 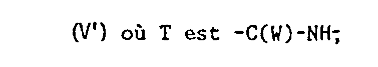 pour former le composé de formule : 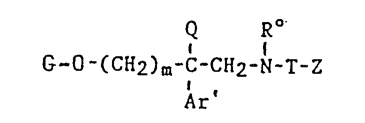
b) puis lorsque G représente un groupe tétrahydropyranyle, on élimine ce groupe par hydrolyse acide pour obtenir l'alcanolamine N-substituée de formule : 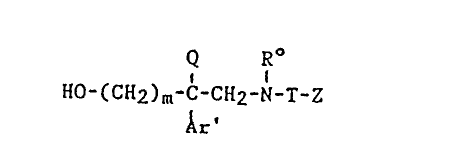
c) puis éventuellement, on traite cette alcanolamine avec le chlorure de méthanesulfonyle pour obtenir le mésylate de formule : 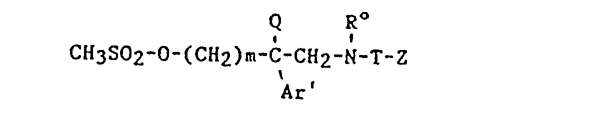
d) on élimine éventuellement les groupes N-protecteurs après chaque étape, et on transforme éventuellement le produit ainsi obtenu en un de ses sels.

5. Procédé selon la revendication 1 pour la préparation du N [ (benzyl-4 pipéridinyl-1)-4 (dichloro-3,4 phényl)-2 butyl ]-dichloro-2,4 benzamide ou d'un de ses sels , caractérisé en ce qu'on utilise :
- une amine libre de formule (II) dans laquelle m = 2 ; Ar' est un groupe dichloro-3,4-phényl ; Q et R° représentent l'hydrogène ;
- un dérivé fonctionnel d'un acide de formule (III) dans laquelle Z est un groupe 2,4-dichlorophényl ;
- et une amine secondaire de formule (VII) dans laquelle Ar est un groupe phényl ; X et X représentent l'hydrogène ; et Y représente -CH.

6. Procédé selon la revendication 1 pour la préparation du N [ (benzyl-4 pipéridinyl-1)-4 (difluoro-3,4 phényl)-2 butyl]-dichloro-2,4 benzamide ou d'un de ses sels , caractérisé en ce qu'on utilise :
- une amine libre de formule (II) dans laquelle m = 2 ; Ar est un groupe difluoro-3,4 phényl ; Q et R° représentent l'hydrogène ;
- un dérivé fonctionnel d'un acide de formule (III) dans laquelle Z est un groupe 2,4-dichlorophényl ;
- et une amine secondaire de formule (VII) dans laquelle Ar est un groupe phényl ; X et X' représentent l'hydrogène ; et Y représente -CH.

7. Procédé selon la revendication 1 pour la préparation du N [ (benzyl-4 pipéridinyl-1)-4 (dichloro-3,4 phényl)-2 butyl ] fluoro-4-naphtalène-1 carboxamide ou d'un de ses sels, caractérisé en ce qu'on utilise
- une amine libre de formule (II) dans laquelle m = 2 ; Ar est un groupe dichloro-3,4 phényl ; Q et R représentent l'hydrogène ;
- un dérivé fonctionnel d'un acide de formule (III) dans laquelle Z est un groupe fluoro-4-naphtyl-1 ;
- et une amine secondaire de formule (VII) dans laquelle Ar est un groupe phényl ; X et X représentent l'hydrogène ; et Y représente -CH.

8. Procédé selon la revendication 2 pour la préparation du N [ (benzyl-4 pipéridinyl-1)-4 (dichloro-3,4 phényl)-2 butyl ]-dichloro-2,4 benzamide sous forme optiquement pure (+) ou (-) ou d'un de ses sels , caractérisé en ce qu'on utilise :
- un composé de formule (XVII*) dans laquelle m = 2 ; Ar est un groupe dichloro-3,4 phényl ; et R° est l'hydrogène,
- un dérivé fonctionnel d'un acide de formule (III) dans laquelle Z est un groupe 2,4-dichlorophényl ;
- et une amine de formule (VII) dans laquelle Ar est un groupe phényl ; X et X représentent l'hydrogène ; et Y représente -CH.

9. Procédé selon la revendication 2 pour la préparation du N [ (benzyl-4 pipéridinyl-1)-4 (difluoro-3,4 phényl)-2 butyl ]-dichloro-2,4 benzamide sous forme optiquement pure (+) ou (-) ou d'un de ses sels , caractérisé en ce qu'on utilise :
- un composé de formule (XVII*) dans laquelle m = 2 ; Ar est un groupe difluoro-3,4 phényl ; et R° est l'hydrogène,
- un dérivé fonctionnel d'un acide de formule (III) dans laquelle Z est un groupe 2,4-dichlorophényl ;
- et une amine secondaire de formule (VII) dans laquelle Ar est un groupe phényl ; X et X représentent l'hydrogène ; et Y représente -CH.

10. Procédé selon la revendication 2 pour la préparation du N [ (benzyl-4 pipéridinyl-1)-4 (dichloro-3,4 phényl)-2 butyl] fluoro-4 naphtalène-1 carboxamide sous forme optiquement pure (+) ou (-), caractérisé en ce qu'on utilise :
- un composé de formule (XII^{*}) dans laquelle m = 2 ; Ar est un groupe dichloro-3,4 phényl ; et R est l'hydrogène,
- un dérivé fonctionnel d'un acide de formule (III) dans laquelle Z est un groupe fluoro-4-naphtyl-1 ;
- et une amine secondaire de formule (VII) dans laquelle Ar est un groupe phényl ; X et X représentent l'hydrogène ; et Y représente -CH.

11. Procédé pour la préparation d'un composé optiquement pur de formule : 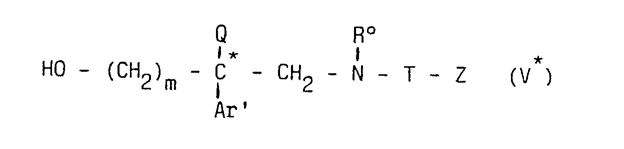 dans laquelle
- "*" signifie que l'atome de carbone ainsi marqué a une configuration (+) ou (-) déterminée ;
- m, Ar', Q, R°, T et Z sont tels que définis dans la revendication 1 ou d'un de ses sels avec des acides minéraux ou organiques, caractérisé en ce qu'on traite un composé de formule : 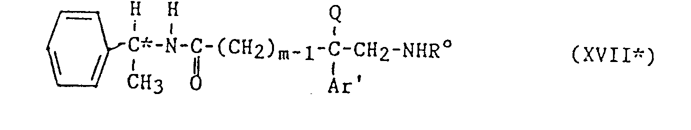 dans un solvant, en milieu acide, pour fournir l'aminoacide de formule: 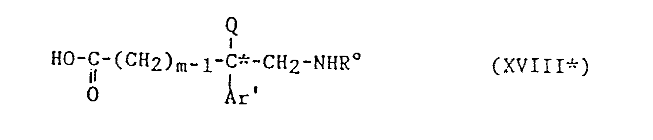 qui est estérifié dans un alcanol AlkOH, où Alk est un alkyle de 1 à 4 atomes de carbone, en milieu acide, puis on traite l'ester correspondant de formule : 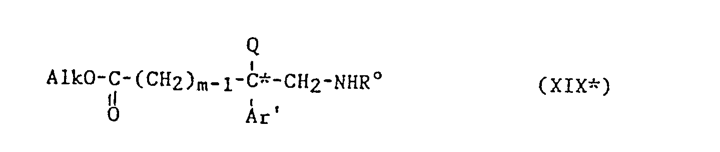 dans laquelle Alk est tel que défini ci-dessus, et Q, Ar', R et m sont tels que définis dans la revendication 1,
- soit avec un dérivé fonctionnel d'un acide de formule : 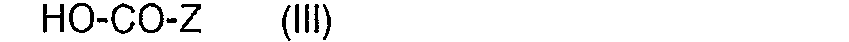
- soit avec un iso(thio)cyanate de formule : 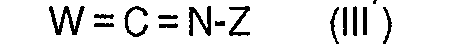 Z et W étant tels que définis dans la revendication 1, on soumet l'ester ainsi obtenu de formule : 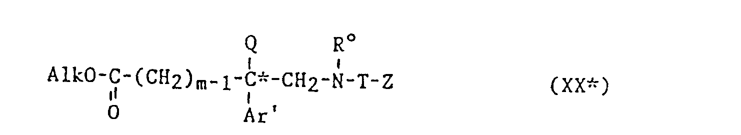 à l'action d'un agent réducteur, le produit obtenu étant éventuellement soumis à une déprotection et transformé en un de ses sels.

12. Procédé pour la préparation d'un composé optiquement pur de formule : 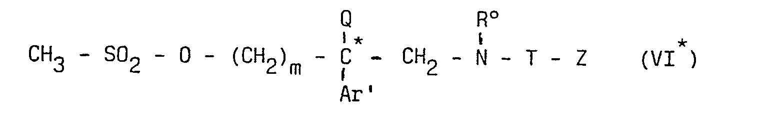 dans laquelle
- "^{*}" signifie que l'atome de carbone ainsi marqué a une configuration (+) ou (-) déterminée ;
- m, Ar, Q, R°, T et Z sont tels que définis dans la revendication 1 ou d'un de ses sels avec des acides minéraux ou organiques, caractérisé en ce qu'on traite un composé de formule : 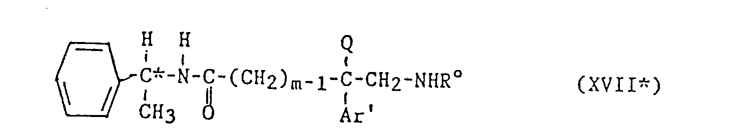 dans un solvant, en milieu acide, pour fournir l'aminoacide de formule: 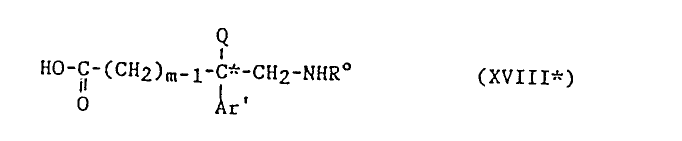 qui est estérifié dans un alcanol AlkOH, où Alk est un alkyle de 1 à 4 atomes de carbone, en milieu acide, puis on traite l'ester correspondant de formule : 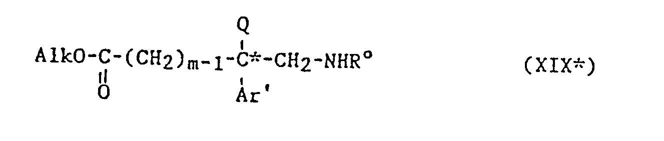 dans laquelle Alk est tel que défini ci-dessus et Q, Ar', R° et m sont tels que définis dans la revendication 1,
- soit avec un dérivé fonctionnel d'un acide de formule : 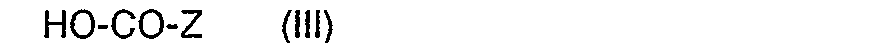
- soit avec un iso(thio)cyanate de formule : 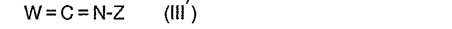 Z et W étant tels que définis dans la revendication 1, on soumet l'ester ainsi obtenu de formule : 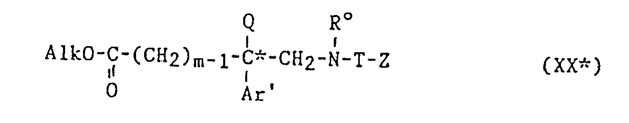 à l'action d'un agent réducteur et on transforme l'alcool correspondant de formule : 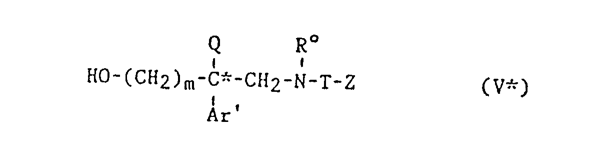 en son ester méthanesulfonate, le produit obtenu étant éventuellement soumis à une déprotection et transformé en un de ses sels.

13. Procédé selon la revendication 3 pour la préparation de composés de formule : 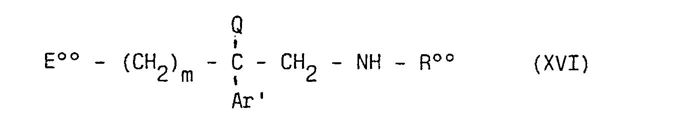 dans laquelle
- E° ° représente un groupe hydroxy,
- R° ° représente l'hydrogène,
- m, Q et Ar sont tels que définis dans la revendication 3, ou de l'un de ses sels, caractérisé en ce que l'on soumet un nitrile de formule 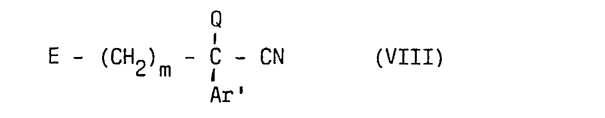 dans laquelle E est un groupe hydroxy ou un groupe O-protecteur tel que tétrahydropyranyloxy à une hydrogénation dans un alcanol en présence d'un catalyseur, suivie de l'élimination du groupe O-protecteur éventuel, et en ce que l'on transforme éventuellement le composé obtenu en l'un de ses sels.

14. Procédé selon la revendication 1 pour la préparation du chlorhydrate de N-méthyl-N [ (benzyl-4 pipéridinyl-1)-4 (dichloro-3,4 phényl)-2 butyl ] phénylacétamide, caractérisé en ce qu'on utilise :
- une amine libre de formule (I) dans laquelle m = 2 ; Ar est un groupe (dichloro-3,4 phényl) ; Q est l'hydrogène et R est méthyle;
- un dérivé fonctionnel d'un acide de formule (III) dans laquelle Z est un groupe benzyle,
- et une amine secondaire de formule (VII) dans laquelle Ar est un groupe phényl; X et X représentent l'hydrogène; et Y représente -CH.

15. Procédé pour la préparation d'une composition pharmaceutique caractérisé en ce que l'on mélange,en tant que principe actif, un composé préparé selon le procédé de l'une quelconque des revendications 1 à 14 avec un véhicule pharmaceutiquement acceptable.

## Revendications (titre)

1. Un composé de formule 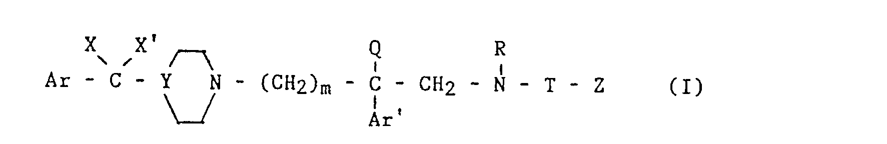 dans laquelle :
- m est un nombre entier de 1 à 3;
- Ar et Ar' représentent, indépendamment, un groupe thiényle; un groupe phényle non substitué, mono ou di-substitué par un atome d'halogène, par un alkyle en C₁-C₃, par un trifluorométhyle, par un alcoxy dans lequel l'alkyle est en Ci-C₃, par un hydroxyle, par un méthylènedioxy ; un groupe imidazolyle ; Ar pouvant également être un groupe benzothiényle non substitué ou substitué par un halogène; un groupe naphtyle non substitué ou substitué par un halogène ; un groupe biphényle; un indolyle non substitué ou substitué sur l'azote par un groupe benzyle ;
- X représente l'hydrogène;
- X représente l'hydrogène, un groupe hydroxyle, ou est réuni à X ci-dessous pour former une liaison carbone-carbone ;
. ou bien X et X , ensemble, forment un groupe oxo ou dialkylaminoalkyloxyimino de formule = N-O-(CH₂)ₚ-Am, où p est 2 ou 3 et Am est un groupe dialkylamino, chaque alkyle pouvant contenir de 1 à 4 atomes de carbone;
- Y représente un atome d'azote ou un groupe C(X") où X est l'hydrogène ou, avec X , forme une liaison carbone-carbone ;
- Q représente l'hydrogène, un groupe alkyle en C₁-C₄ ou un groupe aminoalkyle de formule -(CH₂)_{q}-Am', où q est 2 ou 3 et Am est un groupe pipéridino, benzyl-4 pipéridino ou dialkylamino, chaque alkyle pouvant contenir de 1 à 4 atomes de carbone;
- R représente l'hydrogène, un groupe méthyle ou un groupe (CH₂)ₙ-L, où n est un nombre entier de 2 à 6 et L est l'hydrogène ou un groupe amino,
- T représente un groupe choisi parmi 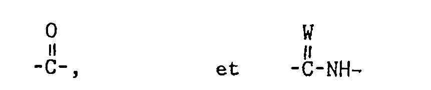 W étant un atome d'oxygène ou de soufre, et
- Z représente soit l'hydrogène, soit M ou OM lorsque T représente le groupe 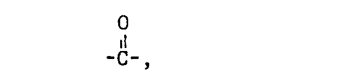 soit M lorsque T représente le groupe 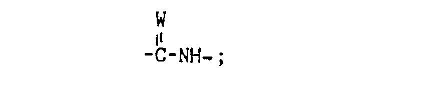
M représente l'hydrogène ou un alkyle droit ou ramifié en Ci-Ce ; un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (méthyl-1 ) imidazolyl-2 thioalkyle dans lequel le groupe alkyle comprend de 1 à 3 atomes de carbone ; un oxo-1 phénylindan-3 yle-2 ; un groupe aromatique ou hétéroaromatique non substitué, mono ou polysubstitué;
ou un de ses sels avec des acides minéraux ou organiques.

2. Un composé optiquement pur de formule : 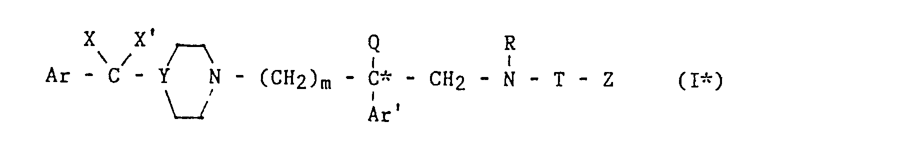 dans laquelle :
- "^{*}" signifie que l'atome de carbone ainsi marqué a une configuration absolue (+) ou (-) déterminée ;
- m, Ar et Ar', X, X', Y, Q, R, T et Z sont tels que définis dans la revendication 1. ou un de ses sels avec des acides minéraux ou organiques.

3. N [ (benzyl-4 pipéridinyl-1)-4 (dichloro-3,4 phényl)-2 butyl ]-dichloro-2,4 benzamide ou un de ses sels pharmaceutiquement acceptables.

4. N [ (benzyl-4 pipéridinyl-1)-4 (difluoro-3,4 phényl)-2 butyl ]-dichloro-2,4 benzamide ou un de ses sels pharmaceutiquement acceptables.

5. N [ (benzyl-4 pipéridinyl-1 )-4 (dichloro-3,4 phényl)-2 butyl ] fluoro-4 naphtalène-1 carboxamide ou un de ses sels pharmaceutiquement acceptables.

6. N [ (benzyl-4 pipéridinyl-l)-4 (dichloro-3,4 phényl)-2 butyl ]-dichloro-2,4 benzamide (+) ou un de ses sels pharmaceutiquement acceptables.

7. N [ (benzyl-4 pipéridinyl-1)-4 (dichloro-3,4 phényl)-2 butyl ]-dichloro-2,4 benzamide (-) ou un de ses sels pharmaceutiquement acceptables.

8. N [ (benzyl-4 pipéridinyl-1)-4 (difluoro-3,4 phényl)-2 butyl ]-dichloro-2,4 benzamide (+) ou un de ses sels pharmaceutiquement acceptables.

9. N [ (benzyl-4 pipéridinyl-1)-4 (difluoro-3,4 phényl)-2 butyl ]-dichloro-2,4 benzamide (-) ou un de ses sels pharmaceutiquement acceptables.

10. N [ (benzyl-4 pipéridinyl-1)-4 (dichloro-3,4 phényl)-2 butyl ] fluoro-4 naphtalène-1 carboxamide (+) ou un de ses sels pharmaceutiquement acceptables.

11. N [ (benzyl-4 pipéridinyl-1)-4 (dichloro-3,4 phényl)-2 butyl ] fluoro-4 naphtalène-1 carboxamide (-) ou un de ses sels pharmaceutiquement acceptables.

12. Procédé pour la préparation de composés selon la revendication 1, caractérisé en ce que a) on traite une amine libre de formule : 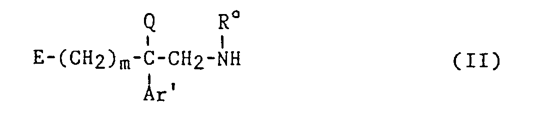 dans laquelle m, Ar et Q sont tels que définis précédemment dans la revendication 1 ; R représente l'hydrogène, un groupe méthyle ou un groupe (CH₂)ₙ-L°, où n est tel que défini dans la revendication 1, et L est l'hydrogène ou un groupe amino protégé par un groupe N-protecteur ; et E représente un groupe hydroxy ou un groupe O-protégé tel que tétrahydropyran-2-yloxy, ou un groupe 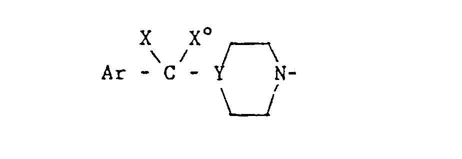 où Ar, X et Y sont tels que définis dans la revendication 1 et X° représente le groupe X', tel que défini dans la revendication 1, dans lequel le groupe hydroxy est protégé par un groupe O-protecteur ; - soit avec un dérivé fonctionnel d'un acide de formule : 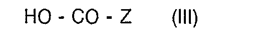 dans laquelle Z est tel que défini dans la revendication 1, lorsqu'on doit préparer un composé de formule (I) où T est -CO-, - soit avec un iso(thio)cyanate de formule : 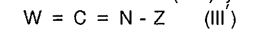 dans laquelle W et Z sont tels que définis dans la revendication 1, lorsqu'on doit préparer un composé de formule (I) où T est 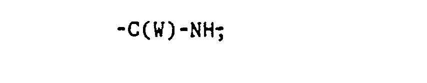 pour former le composé de formule : 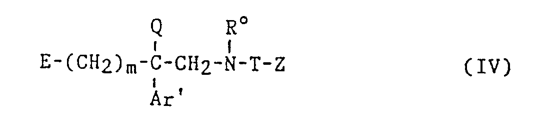
b) puis, lorsque E représente le tétrahydropyranyloxy, on élimine le groupe tétrahydropyranyle par hydrolyse acide, l'hydrolyse pouvant alternativement avoir lieu à l'étape a) sur l'amine de départ de formule (II),
c) on traite l'alcanolamine N-substituée ainsi obtenue de formule: 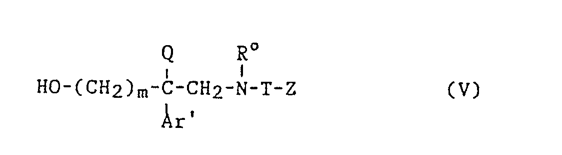 avec le chlorure de méthanesulfonyle
d) on fait réagir le mésylate ainsi obtenu de formule : 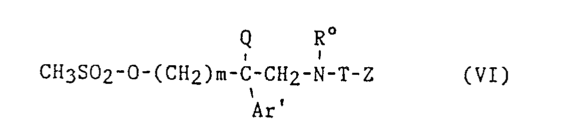 avec une amine secondaire de formule : 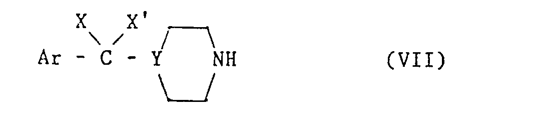 dans laquelle Ar, Y, X et X sont tels que définis dans la revendication 1,
e) on élimine les groupes 0-protecteur et N-protecteur éventuels et on transforme éventuellement le produit ainsi obtenu en un de ses sels.

13. Procédé stéréosélectif pour la préparation de composés optiquement purs de formule (1^{*}), selon la revendication 2, caractérisé en ce que on traite un composé de formule : 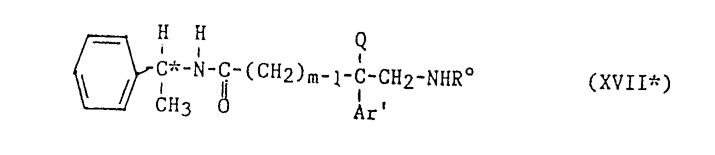 dans un solvant, en milieu acide, pour fournir l'aminoacide de formule: 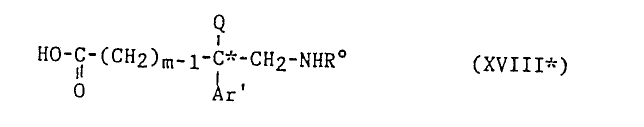 qui est estérifié dans un alcanol AIkOH, où Alk est un alkyle de 1 à 4 atomes de carbone, en milieu acide, puis on traite l'ester correspondant de formule : 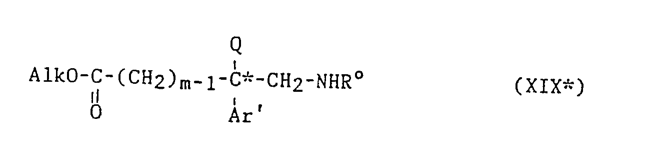 dans laquelle Alk est tel que défini ci-dessus; Q, Ar et m sont tels que définis dans la revendication 1 et R est tel que défini dans la revendication 12,
- soit avec un dérivé fonctionnel d'un acide de formule : 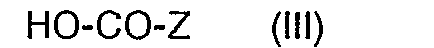
- soit avec un iso(thio)cyanate de formule : 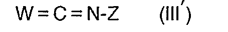 Z et W étant tel que définis ci-dessus, dans la revendication 2, on soumet l'ester ainsi obtenu de formule : 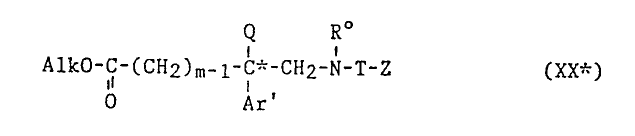 à l'action d'un agent réducteur et on transforme l'alcool correspondant de formule : 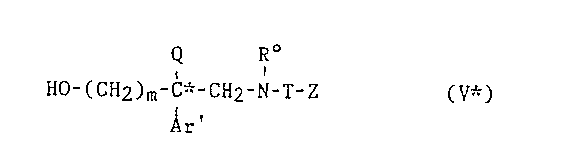 en son ester méthanesulfonate de formule: 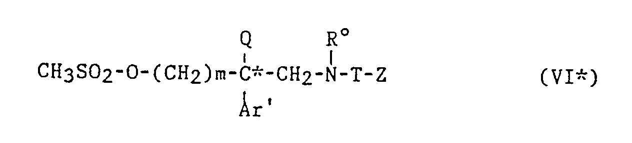 qui, par traitement avec une amine de formule : 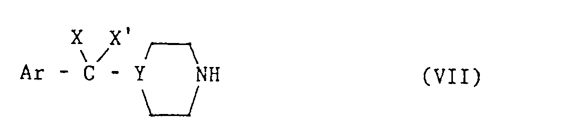 dans laquelle Ar,X,X et Y sont tels que définis dans la revendication 1 et déprotection éventuelle, donne le composé de formule (1^{*}).

14. Un composé de formule : 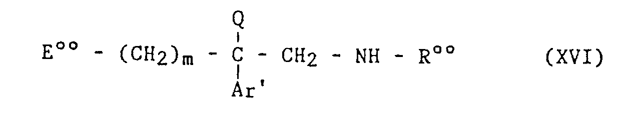 dans laquelle
- E° ° représente un groupe tétrahydropyranyloxy, un groupe hydroxy, ou un groupe 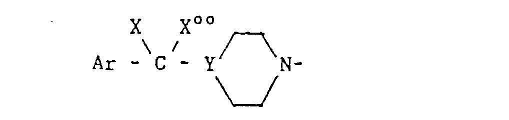
- X° ° représente l'hydrogène, un groupe hydroxyle libre ou protégé par un groupe 0-protecteur, ou est réuni à X" ci-dessous pour former une liaison carbone-carbone ; . ou bien X et X° °, ensemble, forment un groupe oxo ou dialkylaminoalkyloxyimino de formule = N-O-(CH₂)p-Am, où p est 2 ou 3 et Am est un groupe dialkylamino, chaque alkyle pouvant contenir de 1 à 4 atomes de carbone ;
- Y représente un atome d'azote ou un groupe C(X") où X est l'hydrogène, ou, avec X° °, forme une liaison carbone-carbone;
- R° représente l'hydrogène, un groupe méthyle ou un groupe (CH₂)ₙ₋L° °, où n est un nombre de 2 à 6 et L° est l'hydrogène ou un groupe amino libre ou protégé par un groupe N-protecteur ; - m, Ar et Ar', X et Q sont tels que définis dans la revendication 1 ou un de ses sels.

15. Composé selon la revendication 14, de formule (XVI), dans laquelle m, Q, Ar' sont tels que définis dans la revendication 1, E° ° est un groupe hydroxy, R ° ° est R°, ce dernier ayant la signification définie dans la revendication 12, ledit composé étant sous forme optiquement pure.

16. Composé de formule : 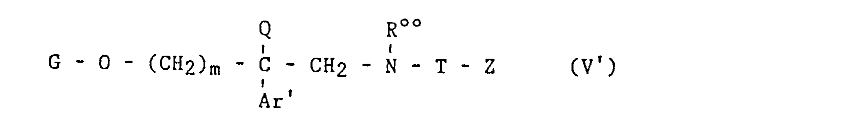 dans laquelle :
- G est l'hydrogène, un groupe tétrahydropyranyle ou un groupe méthanesulfonyle ;
- R° ° représente l'hydrogène, un groupe méthyle ou un groupe (CH₂)ₙ-L° °, où n est un nombre de 2 à 6 et L° est l'hydrogène ou un groupe amino libre ou protégé par un groupe N-protecteur ;
- m,Ar', Q, T et Z sont tels que définis dans la revendication 1. ou un de ses sels.

17. Composé selon la revendication 16, de formule (V') dans laquelle m, Q, T, Ar' et Z sont tels que définis dans la revendication 1, G est l'hydrogène et R ° est R°, ce dernier ayant la signification définie dans la revendication 12, ledit composé étant sous forme optiquement pure.

18. Composé selon la revendication 15 de formule (V') dans laquelle m, Q, T, Ar' et Z sont tels que définis dans la revendication 1, G est un groupe méthanesulfonyle et R° ° est R°, ce dernier ayant la signification définie dans la revendication 12, ledit composé étant sous forme optiquement pure.

19. Composé selon la revendication 14, caractérisé en ce que E° ° représente un groupe hydroxy et R représente l'hydrogène.

20. Chlorhydrate de N-méthyl-N[ (benzyl-4 pipéridinyl-1 )-4 (dichloro-3,4 phényl)-2 butyl] phénylacétamide.

21. Procédé pour le préparation d'une composition pharmaceutique, caractérisé en ce que l'on mélange, en tant que principe actif, un composé selon l'une quelconque des revendications 1 à 11 et 14 à 20 avec un véhicule pharmaceutiquement acceptable.
